(19) Europäisches Patentamt
European Patent Office
Office européen des brevets

(11) EP 3 733 710 A1

(12) EUROPEAN PATENT APPLICATION

(43) Date of publication:
**04.11.2020 Bulletin 2020/45**

(51) Int Cl.:
***C07K 16/28*** $^{(2006.01)}$ ***C07K 16/32*** $^{(2006.01)}$

(21) Application number: **20165091.8**

(22) Date of filing: **25.09.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.09.2013 US 201361882428 P**

(62) Document number(s) of the earlier application(s) in accordance with Art. 76 EPC:
**14782039.3 / 3 049 440**

(71) Applicant: **Amgen, Inc**
**Thousand Oaks, CA 91320-1799 (US)**

(72) Inventors:
- **MICHAELS, Mark L.**
  **Thousand Oaks, CA California 91320-1799 (US)**
- **BORGES, Luis G.**
  **Thousand Oaks, CA California 91320-1799 (US)**
- **YAN, Wei**
  **Thousand Oaks, CA California 91320-1799 (US)**

(74) Representative: **Schiweck Weinzierl Koch Patentanwälte Partnerschaft mbB**
**Ganghoferstraße 68 B**
**80339 München (DE)**

Remarks:
•This application was filed on 24.03.2020 as a divisional application to the application mentioned under INID code 62.
•Claims filed after the date of receipt of the divisional application (Rule 68(4) EPC).

# (54) HETRODIMERIC V-C-FC-V-C ANTIBODY

(57) Described herein is a dimeric, bispecific antibody format. Each polypeptide chain of the dimer comprises an immunoglobulin variable region, an immunoglobulin constant region, an Fc polypeptide chain, another immunoglobulin variable region, and another immunoglobulin constant region. Also described are methods of making and using such antibodies.

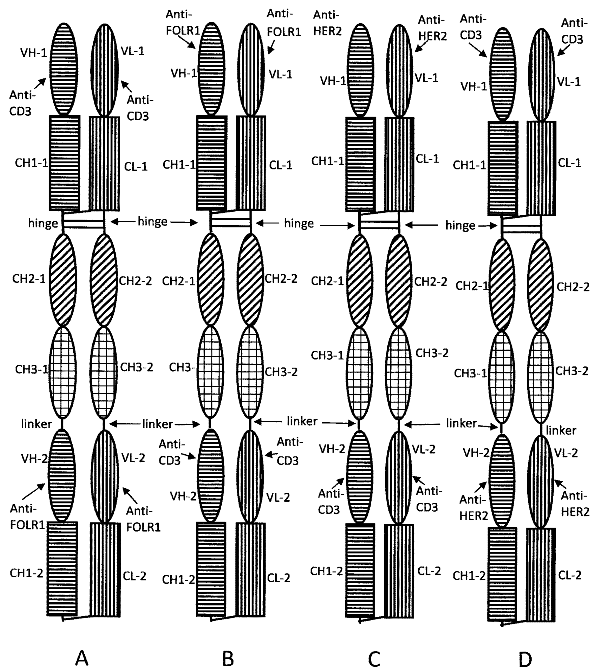


Figure 2

EP 3 733 710 A1

Printed by Jouve, 75001 PARIS (FR)

SEQUENCE LISTING

<110> AMGEN INC.

<120> V-C-FC-V-C ANTIBODY

<130> AMG15546PCTEP

<140> EP 14782039.3
<141> 2014-09-25

<150> 61/882,428
<151> 2013-09-25

<160> 101

<170> PatentIn version 3.5

<210> 1
<211> 708
<212> PRT
<213> Artificial Sequence

<220>
<221> source
<223> /note="Description of Artificial Sequence: Synthetic polypeptide"

<400> 1

```
Met Gly Ser Thr Ala Ile Leu Gly Leu Leu Leu Ala Val Leu Gln Gly
1               5                   10                  15

Gly Arg Ala Gln Val Gln Leu Gln Glu Ser Gly Pro Gly Leu Val Lys
            20                  25                  30

Pro Ser Gln Thr Leu Ser Leu Thr Cys Thr Val Ser Gly Gly Ser Ile
        35                  40                  45

Ser Ser Gly Ala Tyr Tyr Trp Thr Trp Ile Arg Gln His Pro Gly Lys
    50                  55                  60

Gly Leu Glu Trp Ile Gly Tyr Ile Tyr Tyr Ser Gly Ser Thr Tyr Tyr
65                  70                  75                  80

Asn Pro Ser Leu Lys Ser Arg Val Ser Ile Ser Ile Asp Thr Ser Lys
                85                  90                  95

Asn Gln Phe Ser Leu Lys Leu Ser Ser Val Thr Ala Ala Asp Thr Ala
            100                 105                 110

Val Tyr Tyr Cys Ala Arg Gly Ser Ser Ser Trp Phe Asp Tyr Trp Gly
        115                 120                 125

Gln Gly Thr Leu Val Thr Val Ser Ser Ala Ser Thr Lys Gly Pro Ser
    130                 135                 140
```

```
Val Phe Pro Leu Ala Pro Ser Ser Lys Ser Thr Ser Gly Gly Thr Ala
145                 150                 155                 160

Ala Leu Gly Cys Leu Val Lys Asp Tyr Phe Pro Glu Pro Val Thr Val
                165                 170                 175

Ser Trp Asn Ser Gly Ala Leu Thr Ser Gly Val His Thr Phe Pro Ala
            180                 185                 190

Val Leu Gln Ser Ser Gly Leu Tyr Ser Leu Ser Ser Val Val Thr Val
        195                 200                 205

Pro Ser Ser Ser Leu Gly Thr Gln Thr Tyr Ile Cys Asn Val Asn His
    210                 215                 220

Lys Pro Ser Asn Thr Lys Val Asp Lys Arg Val Glu Pro Lys Ser Cys
225                 230                 235                 240

Asp Lys Thr His Thr Cys Pro Pro Cys Pro Ala Pro Glu Ala Ala Gly
                245                 250                 255

Gly Pro Ser Val Phe Leu Phe Pro Pro Lys Pro Lys Asp Thr Leu Met
            260                 265                 270

Ile Ser Arg Thr Pro Glu Val Thr Cys Val Val Val Asp Val Ser His
        275                 280                 285

Glu Asp Pro Glu Val Lys Phe Asn Trp Tyr Val Asp Gly Val Glu Val
    290                 295                 300

His Asn Ala Lys Thr Lys Pro Arg Glu Glu Gln Tyr Asn Ser Thr Tyr
305                 310                 315                 320

Arg Val Val Ser Val Leu Thr Val Leu His Gln Asp Trp Leu Asn Gly
                325                 330                 335

Lys Glu Tyr Lys Cys Lys Val Ser Asn Lys Ala Leu Pro Ala Pro Ile
            340                 345                 350

Glu Lys Thr Ile Ser Lys Ala Lys Gly Gln Pro Arg Glu Pro Gln Val
        355                 360                 365

Tyr Thr Leu Pro Pro Ser Arg Lys Glu Met Thr Lys Asn Gln Val Ser
    370                 375                 380

Leu Thr Cys Leu Val Lys Gly Phe Tyr Pro Ser Asp Ile Ala Val Glu
```

385 390 395 400

Trp Glu Ser Asn Gly Gln Pro Glu Asn Asn Tyr Lys Thr Thr Pro Pro
405 410 415

Val Leu Lys Ser Asp Gly Ser Phe Phe Leu Tyr Ser Lys Leu Thr Val
420 425 430

Asp Lys Ser Arg Trp Gln Gln Gly Asn Val Phe Ser Cys Ser Val Met
435 440 445

His Glu Ala Leu His Asn His Tyr Thr Gln Lys Ser Leu Ser Leu Ser
450 455 460

Pro Gly Lys Ala Ala Ala Gly Gly Gly Gly Ser Gly Gly Gly Gly Ser
465 470 475 480

Glu Val Gln Leu Val Glu Ser Gly Gly Gly Leu Val Gln Pro Gly Gly
485 490 495

Ser Leu Lys Leu Ser Cys Ala Ala Ser Gly Phe Thr Phe Asn Ser Tyr
500 505 510

Ala Met Asn Trp Val Arg Gln Ala Pro Gly Lys Gly Leu Glu Trp Val
515 520 525

Ala Arg Ile Arg Ser Lys Tyr Asn Asn Tyr Ala Thr Tyr Tyr Ala Asp
530 535 540

Ser Val Lys Gly Arg Phe Thr Ile Ser Arg Asp Asp Ser Lys Asn Thr
545 550 555 560

Ala Tyr Leu Gln Met Asn Asn Leu Lys Thr Glu Asp Thr Ala Val Tyr
565 570 575

Tyr Cys Val Arg His Gly Asn Phe Gly Asn Ser Tyr Val Ser Trp Trp
580 585 590

Ala Tyr Trp Gly Gln Gly Thr Leu Val Thr Val Ser Ser Ala Ser Thr
595 600 605

Lys Gly Pro Ser Val Phe Pro Leu Ala Pro Ser Ser Lys Ser Thr Ser
610 615 620

Gly Gly Thr Ala Ala Leu Gly Cys Leu Val Lys Asp Tyr Phe Pro Glu
625 630 635 640

Pro Val Thr Val Ser Trp Asn Ser Gly Ala Leu Thr Ser Gly Val His
645 650 655

Thr Phe Pro Ala Val Leu Gln Ser Ser Gly Leu Tyr Ser Leu Ser Ser
660 665 670

Val Val Thr Val Pro Ser Ser Ser Leu Gly Thr Gln Thr Tyr Ile Cys
675 680 685

Asn Val Asn His Lys Pro Ser Asn Thr Lys Val Asp Lys Arg Val Glu
690 695 700

Pro Lys Ser Cys
705

<210> 2
<211> 2124
<212> DNA
<213> Artificial Sequence

<220>
<221> source
<223> /note="Description of Artificial Sequence: Synthetic polynucleotide"

<400> 2
atggggtcaa ccgccatcct tggcctcctc ctggctgtcc tgcagggagg gcgcgcccag 60

gtgcagctgc aggagtcggg cccaggactg gtgaagcctt cacagaccct gtccctcacc 120

tgcactgtct ctggtggctc catcagcagt ggtgcttact actggacctg gatccgccag 180

cacccaggga agggcctgga gtggattggg tacatctatt acagtgggag cacctactac 240

aacccgtccc tcaagagtcg agttagcata tcaatagaca cgtctaagaa ccagttctcc 300

ctgaagctga gctctgtgac tgccgcggac acggccgtgt attactgtgc gcgaggcagc 360

agcagctggt tcgactactg gggccaggga accctggtca ccgtctcctc agctagcacc 420

aagggcccat ccgtcttccc cctggcaccc tcctccaaga gcacctctgg gggcacagcg 480

gccctgggct gcctggtcaa ggactacttc cccgaaccgg tgacggtgtc gtggaactca 540

ggcgccctga ccagcggcgt gcacaccttc ccggctgtcc tacagtcctc aggactctac 600

tccctcagca gcgtggtgac cgtgccctcc agcagcttgg gcacccagac ctacatctgc 660

aacgtgaatc acaagcccag caacaccaag gtggacaaga gagttgagcc caaatcttgt 720

gacaaaactc acacatgccc accgtgccca gcacctgaag cagctggggg accgtcagtc 780

ttcctcttcc ccccaaaacc caaggacacc ctcatgatct cccggacccc tgaggtcaca 840

tgcgtggtgg tggacgtgag ccacgaagac cctgaggtca agttcaactg gtacgtggac 900

ggcgtggagg tgcataatgc caagacaaag ccgcgggagg agcagtacaa cagcacgtac 960

cgtgtggtca gcgtcctcac cgtcctgcac caggactggc tgaatggcaa ggagtacaag 1020

tgcaaggtct ccaacaaagc cctcccagcc cccatcgaga aaaccatctc caaagccaaa 1080

gggcagcccc gagaaccaca ggtgtacacc ctgcccccat cccggaagga gatgaccaag 1140

aaccaggtca gcctgacctg cctggtcaaa ggcttctatc ccagcgacat cgccgtggag 1200

tgggagagca atgggcagcc ggagaacaac tacaagacca cgcctcccgt gctgaagtcc 1260

gacggctcct tcttcctcta tagcaagctc accgtggaca agagcaggtg gcagcagggg 1320

aacgtcttct catgctccgt gatgcatgag gctctgcaca accactacac gcagaagagc 1380

ctctccctgt ctccgggtaa agcggccgct ggaggcggcg gttcaggcgg aggtggctct 1440

gaggtgcagc tggtcgagtc tggaggagga ttggtgcagc ctggagggtc attgaaactc 1500

tcatgtgcag cctctggatt caccttcaat agctacgcca tgaactgggt ccgccaggct 1560

ccaggaaagg gtttggaatg ggttgctcgc ataagaagta aatataataa ttatgcaaca 1620

tattatgccg attcagtgaa aggcaggttc accatctcca gagatgattc aaaaaacact 1680

gcctatctac aaatgaacaa cttgaaaact gaggacactg ccgtgtacta ctgtgtgaga 1740

catgggaact tcggtaatag ctacgtttcc tggtgggctt actggggcca agggactctg 1800

gtcaccgtct cctcagctag caccaagggc ccatccgtct tccccctggc accctcctcc 1860

aagagcacct ctgggggcac agcggccctg ggctgcctgg tcaaggacta cttccccgaa 1920

ccggtgacgg tgtcgtggaa ctcaggcgcc ctgaccagcg gcgtgcacac cttcccggct 1980

gtcctacagt cctcaggact ctactccctc agcagcgtgg tgaccgtgcc ctccagcagc 2040

ttgggcaccc agacctacat ctgcaacgtg aatcacaagc ccagcaacac caaggtggac 2100

aagagagttg agcccaaatc ttgt 2124

<210> 3
<211> 690
<212> PRT
<213> Artificial Sequence

<220>
<221> source
<223> /note="Description of Artificial Sequence: Synthetic
polypeptide"

<400> 3
Met Gly Ser Thr Ala Ile Leu Gly Leu Leu Leu Ala Val Leu Gln Gly
1 5 10 15

Gly Arg Ala Gln Ser Val Leu Thr Gln Pro Pro Ser Val Ser Glu Ala
20 25 30

Pro Arg Gln Arg Val Thr Ile Ser Cys Ser Gly Ser Ser Ser Asn Ile
35 40 45

Gly Asn Asn Ala Val Asn Trp Tyr Gln Gln Leu Pro Gly Lys Ala Pro
50 55 60

Lys Leu Leu Ile Tyr Tyr Asp Asp Met Leu Ser Ser Gly Val Ser Asp
65 70 75 80

Arg Phe Ser Gly Ser Lys Ser Gly Thr Ser Ala Ser Leu Ala Ile Ser
85 90 95

Gly Leu Gln Ser Glu Asp Glu Ala Asp Tyr Tyr Cys Ala Ala Trp Asp
100 105 110

Asp Ser Leu Asn Gly Val Val Phe Gly Gly Gly Thr Lys Leu Thr Val
115 120 125

Leu Gly Gln Pro Lys Ala Ala Pro Ser Val Thr Leu Phe Pro Pro Ser
130 135 140

Ser Glu Glu Leu Gln Ala Asn Lys Ala Thr Leu Val Cys Leu Ile Ser
145 150 155 160

Asp Phe Tyr Pro Gly Ala Val Thr Val Ala Trp Lys Ala Asp Ser Ser
165 170 175

Pro Val Lys Ala Gly Val Glu Thr Thr Thr Pro Ser Lys Gln Ser Asn
180 185 190

Asn Lys Tyr Ala Ala Ser Ser Tyr Leu Ser Leu Thr Pro Glu Gln Trp
195 200 205

Lys Ser His Arg Ser Tyr Ser Cys Gln Val Thr His Glu Gly Ser Thr
210 215 220

Val Glu Lys Thr Val Ala Pro Thr Glu Cys Ser Asp Lys Thr His Thr
225 230 235 240

Cys Pro Pro Cys Pro Ala Pro Glu Ala Ala Gly Gly Pro Ser Val Phe
245 250 255

Leu Phe Pro Pro Lys Pro Lys Asp Thr Leu Met Ile Ser Arg Thr Pro
260 265 270

Glu Val Thr Cys Val Val Val Asp Val Ser His Glu Asp Pro Glu Val
275 280 285

Lys Phe Asn Trp Tyr Val Asp Gly Val Glu Val His Asn Ala Lys Thr
290 295 300

Lys Pro Arg Glu Glu Gln Tyr Asn Ser Thr Tyr Arg Val Val Ser Val
305                 310                 315                 320

Leu Thr Val Leu His Gln Asp Trp Leu Asn Gly Lys Glu Tyr Lys Cys
                325                 330                 335

Lys Val Ser Asn Lys Ala Leu Pro Ala Pro Ile Glu Lys Thr Ile Ser
            340                 345                 350

Lys Ala Lys Gly Gln Pro Arg Glu Pro Gln Val Tyr Thr Leu Pro Pro
        355                 360                 365

Ser Arg Glu Glu Met Thr Lys Asn Gln Val Ser Leu Thr Cys Leu Val
    370                 375                 380

Lys Gly Phe Tyr Pro Ser Asp Ile Ala Val Glu Trp Glu Ser Asn Gly
385                 390                 395                 400

Gln Pro Glu Asn Asn Tyr Asp Thr Thr Pro Pro Val Leu Asp Ser Asp
                405                 410                 415

Gly Ser Phe Phe Leu Tyr Ser Glu Leu Thr Val Asp Lys Ser Arg Trp
            420                 425                 430

Gln Gln Gly Asn Val Phe Ser Cys Ser Val Met His Glu Ala Leu His
        435                 440                 445

Asn His Tyr Thr Gln Lys Ser Leu Ser Leu Ser Pro Gly Lys Ala Ala
    450                 455                 460

Ala Gly Gly Gly Gly Ser Gly Gly Gly Gly Ser Gln Thr Val Val Thr
465                 470                 475                 480

Gln Glu Pro Ser Leu Thr Val Ser Pro Gly Gly Thr Val Thr Leu Thr
                485                 490                 495

Cys Gly Ser Ser Thr Gly Ala Val Thr Ser Gly Asn Tyr Pro Asn Trp
            500                 505                 510

Val Gln Gln Lys Pro Gly Gln Ala Pro Arg Gly Leu Ile Gly Gly Thr
        515                 520                 525

Lys Phe Leu Ala Pro Gly Thr Pro Ala Arg Phe Ser Gly Ser Leu Leu
    530                 535                 540

Gly Gly Lys Ala Ala Leu Thr Leu Ser Gly Val Gln Pro Glu Asp Glu

```
545                 550                 555                 560

Ala Glu Tyr Tyr Cys Val Leu Trp Tyr Ser Asn Arg Trp Val Phe Gly
                565                 570                 575

Gly Gly Thr Lys Leu Thr Val Leu Gly Gln Pro Lys Ala Ala Pro Ser
            580                 585                 590

Val Thr Leu Phe Pro Pro Ser Ser Glu Glu Leu Gln Ala Asn Lys Ala
        595                 600                 605

Thr Leu Val Cys Leu Ile Ser Asp Phe Tyr Pro Gly Ala Val Thr Val
    610                 615                 620

Ala Trp Lys Ala Asp Ser Ser Pro Val Lys Ala Gly Val Glu Thr Thr
625                 630                 635                 640

Thr Pro Ser Lys Gln Ser Asn Asn Lys Tyr Ala Ala Ser Ser Tyr Leu
                645                 650                 655

Ser Leu Thr Pro Glu Gln Trp Lys Ser His Arg Ser Tyr Ser Cys Gln
            660                 665                 670

Val Thr His Glu Gly Ser Thr Val Glu Lys Thr Val Ala Pro Thr Glu
        675                 680                 685

Cys Ser
    690


<210> 4
<211> 2070
<212> DNA
<213> Artificial Sequence

<220>
<221> source
<223> /note="Description of Artificial Sequence: Synthetic
      polynucleotide"

<400> 4
atggggtcaa ccgccatcct tggcctcctc ctggctgtcc tgcagggagg gcgcgcccag      60

tctgtgctga ctcagccacc ctcggtgtct gaagccccca ggcagagggt caccatctcc     120

tgttctggaa gcagctccaa catcggaaat aatgctgtaa actggtacca gcagctccca     180

ggaaaggctc ccaaactcct catctattat gatgatatgt tgtcttcagg ggtctcggac     240

cgattttctg gctccaagtc tggcacctca gcctccctgg ccatcagtgg gctccagtct     300

gaggatgagg ctgattatta ctgtgcagca tgggatgaca gcctgaatgg tgtggtattc     360

ggcggaggga ccaagctgac cgtcctaggt cagcccaagg ctgcccccctc ggtcactctg     420
```

```
ttcccgccct cctctgagga gcttcaagcc aacaaggcca cactggtgtg tctcataagt      480
gacttctacc cgggagccgt gacagtggcc tggaaggcag atagcagccc cgtcaaggcg      540
ggagtggaga ccaccacacc ctccaaacaa agcaacaaca agtacgcggc cagcagctat      600
ctgagcctga cgcctgagca gtggaagtcc cacagaagct acagctgcca ggtcacgcat      660
gaagggagca ccgtggagaa gacagtggcc cctacagaat gttcagacaa aactcacaca      720
tgcccaccgt gcccagcacc tgaagcagct gggggaccgt cagtcttcct cttcccccca      780
aaacccaagg acaccctcat gatctcccgg acccctgagg tcacatgcgt ggtggtggac      840
gtgagccacg aagaccctga ggtcaagttc aactggtacg tggacggcgt ggaggtgcat      900
aatgccaaga caaagccgcg ggaggagcag tacaacagca cgtaccgtgt ggtcagcgtc      960
ctcaccgtcc tgcaccagga ctggctgaat ggcaaggagt acaagtgcaa ggtctccaac     1020
aaagccctcc cagcccccat cgagaaaacc atctccaaag ccaaagggca gccccgagaa     1080
ccacaggtgt acaccctgcc cccatcccgg gaggagatga ccaagaacca ggtcagcctg     1140
acctgcctgg tcaaaggctt ctatcccagc gacatcgccg tggagtggga gagcaatggg     1200
cagccggaga acaactacga caccacgcct cccgtgctgg actccgacgg ctccttcttc     1260
ctctatagcg agctcaccgt ggacaagagc aggtggcagc aggggaacgt cttctcatgc     1320
tccgtgatgc atgaggctct gcacaaccac tacacgcaga agagcctctc cctgtctccg     1380
ggtaaagcgg ccgctggagg cggcggttca ggcggaggtg gctctcagac tgttgtgact     1440
caggaacctt cactcaccgt atcacctggt ggaacagtca cactcacttg tggctcctcg     1500
actggggctg ttacatctgg caactaccca aactgggtcc aacaaaaacc aggtcaggca     1560
ccccgtggtc taataggtgg gactaagttc ctcgcccccg gtactcctgc cagattctca     1620
ggctccctgc ttggaggcaa ggctgccctc accctctcag ggtacagcc agaggatgag       1680
gcagaatatt actgtgttct atggtacagc aaccgctggg tgttcggtgg aggaaccaaa     1740
ctgactgtcc taggtcagcc caaggctgcc ccctcggtca ctctgttccc gccctcctct     1800
gaggagcttc aagccaacaa ggccacactg gtgtgtctca taagtgactt ctacccggga     1860
gccgtgacag tggcctggaa ggcagatagc agccccgtca aggcgggagt ggagaccacc     1920
acaccctcca aacaaagcaa caacaagtac gcggccagca gctatctgag cctgacgcct     1980
gagcagtgga agtcccacag aagctacagc tgccaggtca cgcatgaagg gagcaccgtg     2040
gagaagacag tggcccctac agaatgttca                                      2070
```

<210> 5
<211> 708
<212> PRT
<213> Artificial Sequence

<220>
<221> source
<223> /note="Description of Artificial Sequence: Synthetic
polypeptide"

<400> 5
Met Gly Ser Thr Ala Ile Leu Gly Leu Leu Leu Ala Val Leu Gln Gly
1               5                   10                  15

Gly Arg Ala Glu Val Gln Leu Val Glu Ser Gly Gly Gly Leu Val Gln
            20                  25                  30

Pro Gly Gly Ser Leu Lys Leu Ser Cys Ala Ala Ser Gly Phe Thr Phe
        35                  40                  45

Asn Ser Tyr Ala Met Asn Trp Val Arg Gln Ala Pro Gly Lys Gly Leu
    50                  55                  60

Glu Trp Val Ala Arg Ile Arg Ser Lys Tyr Asn Asn Tyr Ala Thr Tyr
65                  70                  75                  80

Tyr Ala Asp Ser Val Lys Gly Arg Phe Thr Ile Ser Arg Asp Asp Ser
                85                  90                  95

Lys Asn Thr Ala Tyr Leu Gln Met Asn Asn Leu Lys Thr Glu Asp Thr
            100                 105                 110

Ala Val Tyr Tyr Cys Val Arg His Gly Asn Phe Gly Asn Ser Tyr Val
        115                 120                 125

Ser Trp Trp Ala Tyr Trp Gly Gln Gly Thr Leu Val Thr Val Ser Ser
    130                 135                 140

Ala Ser Thr Lys Gly Pro Ser Val Phe Pro Leu Ala Pro Ser Ser Lys
145                 150                 155                 160

Ser Thr Ser Gly Gly Thr Ala Ala Leu Gly Cys Leu Val Lys Asp Tyr
                165                 170                 175

Phe Pro Glu Pro Val Thr Val Ser Trp Asn Ser Gly Ala Leu Thr Ser
            180                 185                 190

Gly Val His Thr Phe Pro Ala Val Leu Gln Ser Ser Gly Leu Tyr Ser
        195                 200                 205

Leu Ser Ser Val Val Thr Val Pro Ser Ser Ser Leu Gly Thr Gln Thr
    210                 215                 220

Tyr Ile Cys Asn Val Asn His Lys Pro Ser Asn Thr Lys Val Asp Lys

```
225                 230                 235                 240

Arg Val Glu Pro Lys Ser Cys Asp Lys Thr His Thr Cys Pro Pro Cys
                245                 250                 255

Pro Ala Pro Glu Ala Ala Gly Gly Pro Ser Val Phe Leu Phe Pro Pro
            260                 265                 270

Lys Pro Lys Asp Thr Leu Met Ile Ser Arg Thr Pro Glu Val Thr Cys
        275                 280                 285

Val Val Val Asp Val Ser His Glu Asp Pro Glu Val Lys Phe Asn Trp
    290                 295                 300

Tyr Val Asp Gly Val Glu Val His Asn Ala Lys Thr Lys Pro Arg Glu
305                 310                 315                 320

Glu Gln Tyr Asn Ser Thr Tyr Arg Val Val Ser Val Leu Thr Val Leu
                325                 330                 335

His Gln Asp Trp Leu Asn Gly Lys Glu Tyr Lys Cys Lys Val Ser Asn
            340                 345                 350

Lys Ala Leu Pro Ala Pro Ile Glu Lys Thr Ile Ser Lys Ala Lys Gly
        355                 360                 365

Gln Pro Arg Glu Pro Gln Val Tyr Thr Leu Pro Pro Ser Arg Lys Glu
    370                 375                 380

Met Thr Lys Asn Gln Val Ser Leu Thr Cys Leu Val Lys Gly Phe Tyr
385                 390                 395                 400

Pro Ser Asp Ile Ala Val Glu Trp Glu Ser Asn Gly Gln Pro Glu Asn
                405                 410                 415

Asn Tyr Lys Thr Thr Pro Pro Val Leu Lys Ser Asp Gly Ser Phe Phe
            420                 425                 430

Leu Tyr Ser Lys Leu Thr Val Asp Lys Ser Arg Trp Gln Gln Gly Asn
        435                 440                 445

Val Phe Ser Cys Ser Val Met His Glu Ala Leu His Asn His Tyr Thr
    450                 455                 460

Gln Lys Ser Leu Ser Leu Ser Pro Gly Lys Ala Ala Ala Gly Gly Gly
465                 470                 475                 480
```

Gly Ser Gly Gly Gly Gly Ser Gln Val Gln Leu Gln Glu Ser Gly Pro
485 490 495

Gly Leu Val Lys Pro Ser Gln Thr Leu Ser Leu Thr Cys Thr Val Ser
500 505 510

Gly Gly Ser Ile Ser Ser Gly Ala Tyr Tyr Trp Thr Trp Ile Arg Gln
515 520 525

His Pro Gly Lys Gly Leu Glu Trp Ile Gly Tyr Ile Tyr Tyr Ser Gly
530 535 540

Ser Thr Tyr Tyr Asn Pro Ser Leu Lys Ser Arg Val Ser Ile Ser Ile
545 550 555 560

Asp Thr Ser Lys Asn Gln Phe Ser Leu Lys Leu Ser Ser Val Thr Ala
565 570 575

Ala Asp Thr Ala Val Tyr Tyr Cys Ala Arg Gly Ser Ser Ser Trp Phe
580 585 590

Asp Tyr Trp Gly Gln Gly Thr Leu Val Thr Val Ser Ser Ala Ser Thr
595 600 605

Lys Gly Pro Ser Val Phe Pro Leu Ala Pro Ser Ser Lys Ser Thr Ser
610 615 620

Gly Gly Thr Ala Ala Leu Gly Cys Leu Val Lys Asp Tyr Phe Pro Glu
625 630 635 640

Pro Val Thr Val Ser Trp Asn Ser Gly Ala Leu Thr Ser Gly Val His
645 650 655

Thr Phe Pro Ala Val Leu Gln Ser Ser Gly Leu Tyr Ser Leu Ser Ser
660 665 670

Val Val Thr Val Pro Ser Ser Ser Leu Gly Thr Gln Thr Tyr Ile Cys
675 680 685

Asn Val Asn His Lys Pro Ser Asn Thr Lys Val Asp Lys Arg Val Glu
690 695 700

Pro Lys Ser Cys
705

<210> 6
<211> 2124
<212> DNA

<213> Artificial Sequence

<220>
<221> source
<223> /note="Description of Artificial Sequence: Synthetic
polynucleotide"

<400> 6
atggggtcaa ccgccatcct tggcctcctc ctggctgtcc tgcagggagg gcgcgccgag 60

gtgcagctgg tcgagtctgg aggaggattg gtgcagcctg gagggtcatt gaaactctca 120

tgtgcagcct ctggattcac cttcaatagc tacgccatga actgggtccg ccaggctcca 180

ggaaagggtt tggaatgggt tgctcgcata agaagtaaat ataataatta tgcaacatat 240

tatgccgatt cagtgaaagg caggttcacc atctccagag atgattcaaa aaacactgcc 300

tatctacaaa tgaacaactt gaaaactgag gacactgccg tgtactactg tgtgagacat 360

gggaacttcg gtaatagcta cgtttcctgg tgggcttact ggggccaagg gactctggtc 420

accgtctcct cagctagcac caagggccca tccgtcttcc ccctggcacc ctcctccaag 480

agcacctctg ggggcacagc ggccctgggc tgcctggtca aggactactt ccccgaaccg 540

gtgacggtgt cgtggaactc aggcgccctg accagcggcg tgcacacctt cccggctgtc 600

ctacagtcct caggactcta ctccctcagc agcgtggtga ccgtgccctc cagcagcttg 660

ggcacccaga cctacatctg caacgtgaat cacaagccca gcaacaccaa ggtggacaag 720

agagttgagc ccaaatcttg tgacaaaact cacacatgcc caccgtgccc agcacctgaa 780

gcagctgggg gaccgtcagt cttcctcttc cccccaaaac ccaaggacac cctcatgatc 840

tcccggaccc ctgaggtcac atgcgtggtg gtggacgtga gccacgaaga ccctgaggtc 900

aagttcaact ggtacgtgga cggcgtggag gtgcataatg ccaagacaaa gccgcgggag 960

gagcagtaca acagcacgta ccgtgtggtc agcgtcctca ccgtcctgca ccaggactgg 1020

ctgaatggca aggagtacaa gtgcaaggtc tccaacaaag ccctcccagc ccccatcgag 1080

aaaaccatct ccaaagccaa agggcagccc cgagaaccac aggtgtacac cctgccccca 1140

tcccggaagg agatgaccaa gaaccaggtc agcctgacct gcctggtcaa aggcttctat 1200

cccagcgaca tcgccgtgga gtgggagagc aatgggcagc cggagaacaa ctacaagacc 1260

acgcctcccg tgctgaagtc cgacggctcc ttcttcctct atagcaagct caccgtggac 1320

aagagcaggt ggcagcaggg gaacgtcttc tcatgctccg tgatgcatga ggctctgcac 1380

aaccactaca cgcagaagag cctctccctg tctccgggta aagcggccgc tggaggcggc 1440

ggttcaggcg gaggtggctc tcaggtgcag ctgcaggagt cgggcccagg actggtgaag 1500

ccttcacaga ccctgtccct cacctgcact gtctctggtg gctccatcag cagtggtgct 1560

tactactgga cctggatccg ccagcaccca gggaagggcc tggagtggat tgggtacatc 1620

tattacagtg ggagcaccta ctacaacccg tccctcaaga gtcgagttag catatcaata 1680

```
gacacgtcta agaaccagtt ctccctgaag ctgagctctg tgactgccgc ggacacggcc      1740

gtgtattact gtgcgcgagg cagcagcagc tggttcgact actggggcca gggaaccctg      1800

gtcaccgtct cctcagctag caccaagggc ccatccgtct tccccctggc accctcctcc      1860

aagagcacct ctgggggcac agcggccctg ggctgcctgg tcaaggacta cttccccgaa      1920

ccggtgacgg tgtcgtggaa ctcaggcgcc ctgaccagcg gcgtgcacac cttcccggct      1980

gtcctacagt cctcaggact ctactccctc agcagcgtgg tgaccgtgcc ctccagcagc      2040

ttgggcaccc agacctacat ctgcaacgtg aatcacaagc ccagcaacac caaggtggac      2100

aagagagttg agcccaaatc ttgt                                              2124


<210> 7
<211> 690
<212> PRT
<213> Artificial Sequence

<220>
<221> source
<223> /note="Description of Artificial Sequence: Synthetic
      polypeptide"

<400> 7
Met Gly Ser Thr Ala Ile Leu Gly Leu Leu Leu Ala Val Leu Gln Gly
1               5                   10                  15


Gly Arg Ala Gln Thr Val Val Thr Gln Glu Pro Ser Leu Thr Val Ser
            20                  25                  30


Pro Gly Gly Thr Val Thr Leu Thr Cys Gly Ser Ser Thr Gly Ala Val
        35                  40                  45


Thr Ser Gly Asn Tyr Pro Asn Trp Val Gln Gln Lys Pro Gly Gln Ala
    50                  55                  60


Pro Arg Gly Leu Ile Gly Gly Thr Lys Phe Leu Ala Pro Gly Thr Pro
65                  70                  75                  80


Ala Arg Phe Ser Gly Ser Leu Leu Gly Gly Lys Ala Ala Leu Thr Leu
                85                  90                  95


Ser Gly Val Gln Pro Glu Asp Glu Ala Glu Tyr Tyr Cys Val Leu Trp
            100                 105                 110


Tyr Ser Asn Arg Trp Val Phe Gly Gly Gly Thr Lys Leu Thr Val Leu
        115                 120                 125


Gly Gln Pro Lys Ala Ala Pro Ser Val Thr Leu Phe Pro Pro Ser Ser
    130                 135                 140
```

Glu Glu Leu Gln Ala Asn Lys Ala Thr Leu Val Cys Leu Ile Ser Asp
145 150 155 160

Phe Tyr Pro Gly Ala Val Thr Val Ala Trp Lys Ala Asp Ser Ser Pro
165 170 175

Val Lys Ala Gly Val Glu Thr Thr Thr Pro Ser Lys Gln Ser Asn Asn
180 185 190

Lys Tyr Ala Ala Ser Ser Tyr Leu Ser Leu Thr Pro Glu Gln Trp Lys
195 200 205

Ser His Arg Ser Tyr Ser Cys Gln Val Thr His Glu Gly Ser Thr Val
210 215 220

Glu Lys Thr Val Ala Pro Thr Glu Cys Ser Asp Lys Thr His Thr Cys
225 230 235 240

Pro Pro Cys Pro Ala Pro Glu Ala Ala Gly Gly Pro Ser Val Phe Leu
245 250 255

Phe Pro Pro Lys Pro Lys Asp Thr Leu Met Ile Ser Arg Thr Pro Glu
260 265 270

Val Thr Cys Val Val Val Asp Val Ser His Glu Asp Pro Glu Val Lys
275 280 285

Phe Asn Trp Tyr Val Asp Gly Val Glu Val His Asn Ala Lys Thr Lys
290 295 300

Pro Arg Glu Glu Gln Tyr Asn Ser Thr Tyr Arg Val Val Ser Val Leu
305 310 315 320

Thr Val Leu His Gln Asp Trp Leu Asn Gly Lys Glu Tyr Lys Cys Lys
325 330 335

Val Ser Asn Lys Ala Leu Pro Ala Pro Ile Glu Lys Thr Ile Ser Lys
340 345 350

Ala Lys Gly Gln Pro Arg Glu Pro Gln Val Tyr Thr Leu Pro Pro Ser
355 360 365

Arg Glu Glu Met Thr Lys Asn Gln Val Ser Leu Thr Cys Leu Val Lys
370 375 380

Gly Phe Tyr Pro Ser Asp Ile Ala Val Glu Trp Glu Ser Asn Gly Gln

385 390 395 400

Pro Glu Asn Asn Tyr Asp Thr Thr Pro Pro Val Leu Asp Ser Asp Gly
405 410 415

Ser Phe Phe Leu Tyr Ser Glu Leu Thr Val Asp Lys Ser Arg Trp Gln
420 425 430

Gln Gly Asn Val Phe Ser Cys Ser Val Met His Glu Ala Leu His Asn
435 440 445

His Tyr Thr Gln Lys Ser Leu Ser Leu Ser Pro Gly Lys Ala Ala Ala
450 455 460

Gly Gly Gly Gly Ser Gly Gly Gly Gly Ser Gln Ser Val Leu Thr Gln
465 470 475 480

Pro Pro Ser Val Ser Glu Ala Pro Arg Gln Arg Val Thr Ile Ser Cys
485 490 495

Ser Gly Ser Ser Ser Asn Ile Gly Asn Asn Ala Val Asn Trp Tyr Gln
500 505 510

Gln Leu Pro Gly Lys Ala Pro Lys Leu Leu Ile Tyr Tyr Asp Asp Met
515 520 525

Leu Ser Ser Gly Val Ser Asp Arg Phe Ser Gly Ser Lys Ser Gly Thr
530 535 540

Ser Ala Ser Leu Ala Ile Ser Gly Leu Gln Ser Glu Asp Glu Ala Asp
545 550 555 560

Tyr Tyr Cys Ala Ala Trp Asp Asp Ser Leu Asn Gly Val Val Phe Gly
565 570 575

Gly Gly Thr Lys Leu Thr Val Leu Gly Gln Pro Lys Ala Ala Pro Ser
580 585 590

Val Thr Leu Phe Pro Pro Ser Ser Glu Glu Leu Gln Ala Asn Lys Ala
595 600 605

Thr Leu Val Cys Leu Ile Ser Asp Phe Tyr Pro Gly Ala Val Thr Val
610 615 620

Ala Trp Lys Ala Asp Ser Ser Pro Val Lys Ala Gly Val Glu Thr Thr
625 630 635 640

```
Thr Pro Ser Lys Gln Ser Asn Asn Lys Tyr Ala Ala Ser Ser Tyr Leu
                645                 650                 655


Ser Leu Thr Pro Glu Gln Trp Lys Ser His Arg Ser Tyr Ser Cys Gln
            660                 665                 670


Val Thr His Glu Gly Ser Thr Val Glu Lys Thr Val Ala Pro Thr Glu
        675                 680                 685


Cys Ser
    690
```

<210> 8
<211> 2070
<212> DNA
<213> Artificial Sequence

<220>
<221> source
<223> /note="Description of Artificial Sequence: Synthetic polynucleotide"

<400> 8

```
atggggtcaa ccgccatcct tggcctcctc ctggctgtcc tgcagggagg gcgcgcccag      60
actgttgtga ctcaggaacc ttcactcacc gtatcacctg gtggaacagt cacactcact     120
tgtggctcct cgactggggc tgttacatct ggcaactacc caaactgggt ccaacaaaaa     180
ccaggtcagg caccccgtgg tctaataggt gggactaagt tcctcgcccc cggtactcct     240
gccagattct caggctccct gcttggaggc aaggctgccc tcaccctctc aggggtacag     300
ccagaggatg aggcagaata ttactgtgtt ctatggtaca gcaaccgctg ggtgttcggt     360
ggaggaacca aactgactgt cctaggtcag cccaaggctg ccccctcggt cactctgttc     420
ccgccctcct ctgaggagct tcaagccaac aaggccacac tggtgtgtct cataagtgac     480
ttctacccgg gagccgtgac agtggcctgg aaggcagata gcagccccgt caaggcggga     540
gtggagacca ccacaccctc caaacaaagc aacaacaagt acgcggccag cagctatctg     600
agcctgacgc ctgagcagtg gaagtcccac agaagctaca gctgccaggt cacgcatgaa     660
gggagcaccg tggagaagac agtggcccct acagaatgtt cagacaaaac tcacacatgc     720
ccaccgtgcc cagcacctga agcagctggg ggaccgtcag tcttcctctt ccccccaaaa     780
cccaaggaca ccctcatgat ctcccggacc cctgaggtca catgcgtggt ggtggacgtg     840
agccacgaag accctgaggt caagttcaac tggtacgtgg acggcgtgga ggtgcataat     900
gccaagacaa agccgcggga ggagcagtac aacagcacgt accgtgtggt cagcgtcctc     960
accgtcctgc accaggactg gctgaatggc aaggagtaca agtgcaaggt ctccaacaaa    1020
gccctcccag cccccatcga gaaaaccatc tccaaagcca aagggcagcc ccgagaacca    1080
```

caggtgtaca ccctgccccc atcccgggag gagatgacca agaaccaggt cagcctgacc 1140

tgcctggtca aaggcttcta tcccagcgac atcgccgtgg agtgggagag caatgggcag 1200

ccggagaaca actacgacac cacgcctccc gtgctggact ccgacggctc cttcttcctc 1260

tatagcgagc tcaccgtgga caagagcagg tggcagcagg ggaacgtctt ctcatgctcc 1320

gtgatgcatg aggctctgca caaccactac acgcagaaga gcctctccct gtctccgggt 1380

aaagcggccg ctggaggcgg cggttcaggc ggaggtggct ctcagtctgt gctgactcag 1440

ccaccctcgg tgtctgaagc ccccaggcag agggtcacca tctcctgttc tggaagcagc 1500

tccaacatcg gaaataatgc tgtaaactgg taccagcagc tcccaggaaa ggctcccaaa 1560

ctcctcatct attatgatga tatgttgtct tcaggggtct cggaccgatt ttctggctcc 1620

aagtctggca cctcagcctc cctggccatc agtgggctcc agtctgagga tgaggctgat 1680

tattactgtg cagcatggga tgacagcctg aatggtgtgg tattcggcgg agggaccaag 1740

ctgaccgtcc taggtcagcc caaggctgcc ccctcggtca ctctgttccc gccctcctct 1800

gaggagcttc aagccaacaa ggccacactg gtgtgtctca taagtgactt ctacccggga 1860

gccgtgacag tggcctggaa ggcagatagc agccccgtca aggcgggagt ggagaccacc 1920

acaccctcca aacaaagcaa caacaagtac gcggccagca gctatctgag cctgacgcct 1980

gagcagtgga agtcccacag aagctacagc tgccaggtca cgcatgaagg gagcaccgtg 2040

gagaagacag tggcccctac agaatgttca 2070

<210> 9
<211> 711
<212> PRT
<213> Artificial Sequence

<220>
<221> source
<223> /note="Description of Artificial Sequence: Synthetic polypeptide"

<400> 9
Met Gly Ser Thr Ala Ile Leu Gly Leu Leu Leu Ala Val Leu Gln Gly
1 5 10 15

Gly Arg Ala Glu Val Gln Leu Leu Glu Gln Ser Gly Ala Glu Leu Val
20 25 30

Arg Pro Gly Ala Leu Val Lys Leu Ser Cys Lys Ala Ser Gly Phe Lys
35 40 45

Ile Lys Asp Tyr Phe Val Asn Trp Val Lys Gln Arg Pro Glu Gln Gly
50 55 60

Leu Glu Trp Ile Gly Trp Ile Asp Pro Glu Asn Asp Asn Ser Leu Tyr

65 70 75 80

Gly Pro Asn Phe Gln Asp Lys Ala Ser Ile Thr Ala Asp Thr Ser Ser
85 90 95

Asn Thr Gly Tyr Leu Gln Leu Ser Gly Leu Thr Ser Glu Asp Thr Ala
100 105 110

Val Tyr Tyr Cys Ala Leu Tyr Tyr Gly Ser Arg Gly Asp Ala Met Asp
115 120 125

Tyr Trp Gly Gln Gly Thr Thr Val Thr Val Ser Ser Ala Ser Thr Lys
130 135 140

Gly Pro Ser Val Phe Pro Leu Ala Pro Ser Ser Lys Ser Thr Ser Gly
145 150 155 160

Gly Thr Ala Ala Leu Gly Cys Leu Val Lys Asp Tyr Phe Pro Glu Pro
165 170 175

Val Thr Val Ser Trp Asn Ser Gly Ala Leu Thr Ser Gly Val His Thr
180 185 190

Phe Pro Ala Val Leu Gln Ser Ser Gly Leu Tyr Ser Leu Ser Ser Val
195 200 205

Val Thr Val Pro Ser Ser Ser Leu Gly Thr Gln Thr Tyr Ile Cys Asn
210 215 220

Val Asn His Lys Pro Ser Asn Thr Lys Val Asp Lys Arg Val Glu Pro
225 230 235 240

Lys Ser Cys Asp Lys Thr His Thr Cys Pro Pro Cys Pro Ala Pro Glu
245 250 255

Ala Ala Gly Gly Pro Ser Val Phe Leu Phe Pro Pro Lys Pro Lys Asp
260 265 270

Thr Leu Met Ile Ser Arg Thr Pro Glu Val Thr Cys Val Val Val Asp
275 280 285

Val Ser His Glu Asp Pro Glu Val Lys Phe Asn Trp Tyr Val Asp Gly
290 295 300

Val Glu Val His Asn Ala Lys Thr Lys Pro Arg Glu Glu Gln Tyr Asn
305 310 315 320

Ser Thr Tyr Arg Val Val Ser Val Leu Thr Val Leu His Gln Asp Trp
325 330 335

Leu Asn Gly Lys Glu Tyr Lys Cys Lys Val Ser Asn Lys Ala Leu Pro
340 345 350

Ala Pro Ile Glu Lys Thr Ile Ser Lys Ala Lys Gly Gln Pro Arg Glu
355 360 365

Pro Gln Val Tyr Thr Leu Pro Pro Ser Arg Lys Glu Met Thr Lys Asn
370 375 380

Gln Val Ser Leu Thr Cys Leu Val Lys Gly Phe Tyr Pro Ser Asp Ile
385 390 395 400

Ala Val Glu Trp Glu Ser Asn Gly Gln Pro Glu Asn Asn Tyr Lys Thr
405 410 415

Thr Pro Pro Val Leu Lys Ser Asp Gly Ser Phe Phe Leu Tyr Ser Lys
420 425 430

Leu Thr Val Asp Lys Ser Arg Trp Gln Gln Gly Asn Val Phe Ser Cys
435 440 445

Ser Val Met His Glu Ala Leu His Asn His Tyr Thr Gln Lys Ser Leu
450 455 460

Ser Leu Ser Pro Gly Lys Ala Ala Ala Gly Gly Gly Gly Ser Gly Gly
465 470 475 480

Gly Gly Ser Glu Val Gln Leu Val Glu Ser Gly Gly Gly Leu Val Gln
485 490 495

Pro Gly Gly Ser Leu Lys Leu Ser Cys Ala Ala Ser Gly Phe Thr Phe
500 505 510

Asn Ser Tyr Ala Met Asn Trp Val Arg Gln Ala Pro Gly Lys Gly Leu
515 520 525

Glu Trp Val Ala Arg Ile Arg Ser Lys Tyr Asn Asn Tyr Ala Thr Tyr
530 535 540

Tyr Ala Asp Ser Val Lys Gly Arg Phe Thr Ile Ser Arg Asp Asp Ser
545 550 555 560

Lys Asn Thr Ala Tyr Leu Gln Met Asn Asn Leu Lys Thr Glu Asp Thr
565 570 575

Ala Val Tyr Tyr Cys Val Arg His Gly Asn Phe Gly Asn Ser Tyr Val
580 585 590

Ser Trp Trp Ala Tyr Trp Gly Gln Gly Thr Leu Val Thr Val Ser Ser
595 600 605

Ala Ser Thr Lys Gly Pro Ser Val Phe Pro Leu Ala Pro Ser Ser Lys
610 615 620

Ser Thr Ser Gly Gly Thr Ala Ala Leu Gly Cys Leu Val Lys Asp Tyr
625 630 635 640

Phe Pro Glu Pro Val Thr Val Ser Trp Asn Ser Gly Ala Leu Thr Ser
645 650 655

Gly Val His Thr Phe Pro Ala Val Leu Gln Ser Ser Gly Leu Tyr Ser
660 665 670

Leu Ser Ser Val Val Thr Val Pro Ser Ser Ser Leu Gly Thr Gln Thr
675 680 685

Tyr Ile Cys Asn Val Asn His Lys Pro Ser Asn Thr Lys Val Asp Lys
690 695 700

Arg Val Glu Pro Lys Ser Cys
705 710

<210> 10
<211> 2133
<212> DNA
<213> Artificial Sequence

<220>
<221> source
<223> /note="Description of Artificial Sequence: Synthetic polynucleotide"

<400> 10
atggggtcaa ccgccatcct tggcctcctc ctggctgtcc tgcagggagg gcgcgccgag 60

gtgcagctgc tcgagcagtc tggagctgag cttgtgaggc caggggcctt agtcaagttg 120

tcctgcaaag cttctggctt caaaattaaa gactactttg tgaactgggt gaagcagagg 180

cctgaacagg gcctggagtg gattggatgg attgatcctg agaatgataa tagtttatat 240

ggcccgaact tccaggacaa ggccagtatc acagcagaca catcctccaa cacaggctac 300

ctgcagctca gcggcctgac atctgaggac actgccgtct attactgtgc tctttattac 360

ggaagtaggg gggatgctat ggactactgg ggccaaggga ccacggtcac cgtctcctca 420

gctagcacca agggcccatc cgtcttcccc ctggcaccct cctccaagag cacctctggg 480

ggcacagcgg ccctgggctg cctggtcaag gactacttcc ccgaaccggt gacggtgtcg 540

tggaactcag gcgccctgac cagcggcgtg cacaccttcc cggctgtcct acagtcctca 600

ggactctact ccctcagcag cgtggtgacc gtgccctcca gcagcttggg cacccagacc 660

tacatctgca acgtgaatca caagcccagc aacaccaagg tggacaagag agttgagccc 720

aaatcttgtg acaaaactca cacatgccca ccgtgcccag cacctgaagc agctggggga 780

ccgtcagtct tcctcttccc cccaaaaccc aaggacaccc tcatgatctc ccggacccct 840

gaggtcacat gcgtggtggt ggacgtgagc cacgaagacc ctgaggtcaa gttcaactgg 900

tacgtggacg gcgtggaggt gcataatgcc aagacaaagc cgcgggagga gcagtacaac 960

agcacgtacc gtgtggtcag cgtcctcacc gtcctgcacc aggactggct gaatggcaag 1020

gagtacaagt gcaaggtctc caacaaagcc ctcccagccc ccatcgagaa aaccatctcc 1080

aaagccaaag ggcagccccg agaaccacag gtgtacaccc tgcccccatc ccggaaggag 1140

atgaccaaga accaggtcag cctgacctgc ctggtcaaag gcttctatcc cagcgacatc 1200

gccgtggagt gggagagcaa tgggcagccg gagaacaact acaagaccac gcctcccgtg 1260

ctgaagtccg acggctcctt cttcctctat agcaagctca ccgtggacaa gagcaggtgg 1320

cagcagggga acgtcttctc atgctccgtg atgcatgagg ctctgcacaa ccactacacg 1380

cagaagagcc tctccctgtc tccgggtaaa gcggccgctg gaggcggcgg ttcaggcgga 1440

ggtggctctg aggtgcagct ggtcgagtct ggaggaggat tggtgcagcc tggagggtca 1500

ttgaaactct catgtgcagc ctctggattc accttcaata gctacgccat gaactgggtc 1560

cgccaggctc caggaaaggg tttggaatgg gttgctcgca taagaagtaa atataataat 1620

tatgcaacat attatgccga ttcagtgaaa ggcaggttca ccatctccag agatgattca 1680

aaaaacactg cctatctaca aatgaacaac ttgaaaactg aggacactgc cgtgtactac 1740

tgtgtgagac atgggaactt cggtaatagc tacgtttcct ggtgggctta ctggggccaa 1800

gggactctgg tcaccgtctc ctcagctagc accaagggcc catccgtctt ccccctggca 1860

ccctcctcca agagcacctc tgggggcaca gcggccctgg gctgcctggt caaggactac 1920

ttccccgaac cggtgacggt gtcgtggaac tcaggcgccc tgaccagcgg cgtgcacacc 1980

ttcccggctg tcctacagtc ctcaggactc tactccctca gcagcgtggt gaccgtgccc 2040

tccagcagct tgggcaccca gacctacatc tgcaacgtga atcacaagcc cagcaacacc 2100

aaggtggaca agagagttga gcccaaatct tgt 2133

<210> 11
<211> 688
<212> PRT
<213> Artificial Sequence

<220>
<221> source
<223> /note="Description of Artificial Sequence: Synthetic
polypeptide"

<400> 11
Met Gly Ser Thr Ala Ile Leu Gly Leu Leu Leu Ala Val Leu Gln Gly
1               5                   10                  15

Gly Arg Ala Glu Leu Val Met Thr Gln Thr Pro Ser Ser Leu Ser Ala
            20                  25                  30

Ser Leu Gly Asp Arg Val Thr Ile Ser Cys Arg Ala Ser Gln Asp Ile
        35                  40                  45

Ser Asn Tyr Leu Asn Trp Tyr Gln Gln Lys Pro Asp Gly Thr Val Lys
    50                  55                  60

Leu Leu Ile Tyr Tyr Thr Ser Arg Leu His Ser Gly Val Pro Ser Arg
65                  70                  75                  80

Phe Ser Gly Ser Gly Ser Gly Thr Asp Tyr Ser Leu Thr Ile Ser Asn
                85                  90                  95

Leu Glu Gln Glu Asp Ile Ala Thr Tyr Phe Cys Gln Gln Gly Asn Thr
            100                 105                 110

Leu Pro Leu Thr Phe Gly Ala Gly Thr Lys Leu Glu Ile Lys Arg Thr
        115                 120                 125

Val Ala Ala Pro Ser Val Phe Ile Phe Pro Pro Ser Asp Glu Gln Leu
    130                 135                 140

Lys Ser Gly Thr Ala Ser Val Val Cys Leu Leu Asn Asn Phe Tyr Pro
145                 150                 155                 160

Arg Glu Ala Lys Val Gln Trp Lys Val Asp Asn Ala Leu Gln Ser Gly
                165                 170                 175

Asn Ser Gln Glu Ser Val Thr Glu Gln Asp Ser Lys Asp Ser Thr Tyr
            180                 185                 190

Ser Leu Ser Ser Thr Leu Thr Leu Ser Lys Ala Asp Tyr Glu Lys His
        195                 200                 205

Lys Val Tyr Ala Cys Glu Val Thr His Gln Gly Leu Ser Ser Pro Val
    210                 215                 220

Thr Lys Ser Phe Asn Arg Gly Glu Cys Asp Lys Thr His Thr Cys Pro

225 230 235 240

Pro Cys Pro Ala Pro Glu Ala Ala Gly Gly Pro Ser Val Phe Leu Phe
245 250 255

Pro Pro Lys Pro Lys Asp Thr Leu Met Ile Ser Arg Thr Pro Glu Val
260 265 270

Thr Cys Val Val Val Asp Val Ser His Glu Asp Pro Glu Val Lys Phe
275 280 285

Asn Trp Tyr Val Asp Gly Val Glu Val His Asn Ala Lys Thr Lys Pro
290 295 300

Arg Glu Glu Gln Tyr Asn Ser Thr Tyr Arg Val Val Ser Val Leu Thr
305 310 315 320

Val Leu His Gln Asp Trp Leu Asn Gly Lys Glu Tyr Lys Cys Lys Val
325 330 335

Ser Asn Lys Ala Leu Pro Ala Pro Ile Glu Lys Thr Ile Ser Lys Ala
340 345 350

Lys Gly Gln Pro Arg Glu Pro Gln Val Tyr Thr Leu Pro Pro Ser Arg
355 360 365

Glu Glu Met Thr Lys Asn Gln Val Ser Leu Thr Cys Leu Val Lys Gly
370 375 380

Phe Tyr Pro Ser Asp Ile Ala Val Glu Trp Glu Ser Asn Gly Gln Pro
385 390 395 400

Glu Asn Asn Tyr Asp Thr Thr Pro Pro Val Leu Asp Ser Asp Gly Ser
405 410 415

Phe Phe Leu Tyr Ser Glu Leu Thr Val Asp Lys Ser Arg Trp Gln Gln
420 425 430

Gly Asn Val Phe Ser Cys Ser Val Met His Glu Ala Leu His Asn His
435 440 445

Tyr Thr Gln Lys Ser Leu Ser Leu Ser Pro Gly Lys Ala Ala Ala Gly
450 455 460

Gly Gly Gly Ser Gly Gly Gly Gly Ser Gln Thr Val Val Thr Gln Glu
465 470 475 480

Pro Ser Leu Thr Val Ser Pro Gly Gly Thr Val Thr Leu Thr Cys Gly
485 490 495

Ser Ser Thr Gly Ala Val Thr Ser Gly Asn Tyr Pro Asn Trp Val Gln
500 505 510

Gln Lys Pro Gly Gln Ala Pro Arg Gly Leu Ile Gly Gly Thr Lys Phe
515 520 525

Leu Ala Pro Gly Thr Pro Ala Arg Phe Ser Gly Ser Leu Leu Gly Gly
530 535 540

Lys Ala Ala Leu Thr Leu Ser Gly Val Gln Pro Glu Asp Glu Ala Glu
545 550 555 560

Tyr Tyr Cys Val Leu Trp Tyr Ser Asn Arg Trp Val Phe Gly Gly Gly
565 570 575

Thr Lys Leu Thr Val Leu Gly Gln Pro Lys Ala Ala Pro Ser Val Thr
580 585 590

Leu Phe Pro Pro Ser Ser Glu Glu Leu Gln Ala Asn Lys Ala Thr Leu
595 600 605

Val Cys Leu Ile Ser Asp Phe Tyr Pro Gly Ala Val Thr Val Ala Trp
610 615 620

Lys Ala Asp Ser Ser Pro Val Lys Ala Gly Val Glu Thr Thr Thr Pro
625 630 635 640

Ser Lys Gln Ser Asn Asn Lys Tyr Ala Ala Ser Ser Tyr Leu Ser Leu
645 650 655

Thr Pro Glu Gln Trp Lys Ser His Arg Ser Tyr Ser Cys Gln Val Thr
660 665 670

His Glu Gly Ser Thr Val Glu Lys Thr Val Ala Pro Thr Glu Cys Ser
675 680 685

<210> 12
<211> 2064
<212> DNA
<213> Artificial Sequence

<220>
<221> source
<223> /note="Description of Artificial Sequence: Synthetic polynucleotide"

<400> 12

```
atggggtcaa ccgccatcct tggcctcctc ctggctgtcc tgcagggagg gcgcgccgag      60
ctcgtgatga cccagactcc atcctccctg tctgcctctc tgggagacag agtcaccatc     120
agttgcaggg caagtcagga cattagcaat tatttaaact ggtatcagca gaaaccagat     180
ggaactgtta aactcctgat ctactacaca tcaagattac actcaggagt cccatcaagg     240
ttcagtggca gtgggtctgg aacagattat tctctcacca ttagcaacct ggagcaagaa     300
gatattgcca cttacttttg ccaacagggt aatacgcttc cgctcacgtt cggtgctggg     360
accaagcttg agatcaaacg tacggtggct gcaccatctg tcttcatctt cccgccatct     420
gatgagcagt tgaaatctgg aactgcctct gttgtgtgcc tgctgaataa cttctatccc     480
agagaggcca aagtacagtg gaaggtggat aacgccctcc aatcgggtaa ctcccaggag     540
agtgtcacag agcaggacag caaggacagc acctacagcc tcagcagcac cctgacgctg     600
agcaaagcag actacgagaa acacaaagtc tacgcctgcg aagtcaccca tcagggcctg     660
agctcgcccg tcacaaagag cttcaacagg ggagagtgtg acaaaactca cacatgccca     720
ccgtgcccag cacctgaagc agctggggga ccgtcagtct tcctcttccc cccaaaaccc     780
aaggacaccc tcatgatctc ccggacccct gaggtcacat gcgtggtggt ggacgtgagc     840
cacgaagacc ctgaggtcaa gttcaactgg tacgtggacg gcgtggaggt gcataatgcc     900
aagacaaagc cgcgggagga gcagtacaac agcacgtacc gtgtggtcag cgtcctcacc     960
gtcctgcacc aggactggct gaatggcaag gagtacaagt gcaaggtctc caacaaagcc    1020
ctcccagccc ccatcgagaa aaccatctcc aaagccaaag ggcagccccg agaaccacag    1080
gtgtacaccc tgcccccatc ccgggaggag atgaccaaga accaggtcag cctgacctgc    1140
ctggtcaaag gcttctatcc cagcgacatc gccgtggagt gggagagcaa tgggcagccg    1200
gagaacaact acgacaccac gcctcccgtg ctggactccg acggctcctt cttcctctat    1260
agcgagctca ccgtggacaa gagcaggtgg cagcagggga acgtcttctc atgctccgtg    1320
atgcatgagg ctctgcacaa ccactacacg cagaagagcc tctccctgtc tccgggtaaa    1380
gcggccgctg gaggcggcgg ttcaggcgga ggtggctctc agactgttgt gactcaggaa    1440
ccttcactca ccgtatcacc tggtggaaca gtcacactca cttgtggctc ctcgactggg    1500
gctgttacat ctggcaacta cccaaactgg gtccaacaaa aaccaggtca ggcaccccgt    1560
ggtctaatag gtgggactaa gttcctcgcc cccggtactc ctgccagatt ctcaggctcc    1620
ctgcttggag gcaaggctgc cctcaccctc tcaggggtac agccagagga tgaggcagaa    1680
tattactgtg ttctatggta cagcaaccgc tgggtgttcg gtggaggaac caaactgact    1740
gtcctaggtc agcccaaggc tgccccctcg gtcactctgt tcccgccctc ctctgaggag    1800
cttcaagcca acaaggccac actggtgtgt ctcataagtg acttctaccc gggagccgtg    1860
acagtggcct ggaaggcaga tagcagcccc gtcaaggcgg gagtggagac caccacaccc    1920
```

tccaaacaaa gcaacaacaa gtacgcggcc agcagctatc tgagcctgac gcctgagcag 1980

tggaagtccc acagaagcta cagctgccag gtcacgcatg aagggagcac cgtggagaag 2040

acagtggccc ctacagaatg ttca 2064

<210> 13
<211> 12
<212> PRT
<213> Artificial Sequence

<220>
<221> source
<223> /note="Description of Artificial Sequence: Synthetic peptide"

<400> 13
Gly Gly Cys Val Phe Asn Met Phe Asn Cys Gly Gly
1 5 10

<210> 14
<211> 12
<212> PRT
<213> Artificial Sequence

<220>
<221> source
<223> /note="Description of Artificial Sequence: Synthetic peptide"

<400> 14
Gly Gly Cys His Leu Pro Phe Ala Val Cys Gly Gly
1 5 10

<210> 15
<211> 12
<212> PRT
<213> Artificial Sequence

<220>
<221> source
<223> /note="Description of Artificial Sequence: Synthetic peptide"

<400> 15
Gly Gly Cys Gly His Glu Tyr Met Trp Cys Gly Gly
1 5 10

<210> 16
<211> 12
<212> PRT
<213> Artificial Sequence

<220>
<221> source
<223> /note="Description of Artificial Sequence: Synthetic peptide"

<400> 16
Gly Gly Cys Trp Pro Leu Gln Asp Tyr Cys Gly Gly
1 5 10

<210> 17
<211> 12
<212> PRT
<213> Artificial Sequence

<220>
<221> source
<223> /note="Description of Artificial Sequence: Synthetic peptide"

<400> 17
Gly Gly Cys Met Gln Met Asn Lys Trp Cys Gly Gly
1 5 10

<210> 18
<211> 12
<212> PRT
<213> Artificial Sequence

<220>
<221> source
<223> /note="Description of Artificial Sequence: Synthetic peptide"

<400> 18
Gly Gly Cys Asp Gly Arg Thr Lys Tyr Cys Gly Gly
1 5 10

<210> 19
<211> 12
<212> PRT
<213> Artificial Sequence

<220>
<221> source
<223> /note="Description of Artificial Sequence: Synthetic peptide"

<400> 19
Gly Gly Cys Ala Leu Tyr Pro Thr Asn Cys Gly Gly
1 5 10

<210> 20
<211> 12
<212> PRT
<213> Artificial Sequence

<220>
<221> source
<223> /note="Description of Artificial Sequence: Synthetic peptide"

<400> 20
Gly Gly Cys Gly Lys His Trp His Gln Cys Gly Gly
1 5 10

<210> 21
<211> 12
<212> PRT
<213> Artificial Sequence

<220>
<221> source
<223> /note="Description of Artificial Sequence: Synthetic peptide"

<400> 21
Gly Gly Cys His Ser Phe Lys His Phe Cys Gly Gly
1 5 10

<210> 22
<211> 12
<212> PRT
<213> Artificial Sequence

<220>
<221> source
<223> /note="Description of Artificial Sequence: Synthetic peptide"

<400> 22
Gly Gly Cys Gln Gly Met Trp Thr Trp Cys Gly Gly
1 5 10

<210> 23
<211> 14
<212> PRT
<213> Artificial Sequence

<220>
<221> source
<223> /note="Description of Artificial Sequence: Synthetic peptide"

<400> 23
Gly Gly Cys Ala Gln Gln Trp His His Glu Tyr Cys Gly Gly
1 5 10

<210> 24
<211> 12
<212> PRT
<213> Artificial Sequence

<220>
<221> source
<223> /note="Description of Artificial Sequence: Synthetic peptide"

<400> 24
Gly Gly Cys Glu Arg Phe His His Ala Cys Gly Gly
1 5 10

<210> 25

<211> 91
<212> PRT
<213> Artificial Sequence

<220>
<221> source
<223> /note="Description of Artificial Sequence: Synthetic polypeptide"

<400> 25
Val Pro Arg Asp Leu Glu Val Val Ala Ala Thr Pro Thr Ser Leu Leu
1 5 10 15

Ile Ser Trp Asp Ala Pro His His Gly Val Ala Tyr Tyr Arg Ile Thr
20 25 30

Tyr Gly Glu Thr Gly Gly Asn Ser Pro Val Gln Glu Phe Thr Val Pro
35 40 45

Gly Ser Lys Ser Thr Ala Thr Ile Ser Gly Leu Lys Pro Gly Val Asp
50 55 60

Tyr Thr Ile Asn Val Tyr Ala Val Leu Ala Tyr Pro Arg Gly Tyr Pro
65 70 75 80

Leu Ser Lys Pro Ile Ser Ile Asn Tyr Arg Thr
85 90

<210> 26
<211> 232
<212> PRT
<213> Homo sapiens

<400> 26
Glu Pro Lys Ser Cys Asp Lys Thr His Thr Cys Pro Pro Cys Pro Ala
1 5 10 15

Pro Glu Leu Leu Gly Gly Pro Ser Val Phe Leu Phe Pro Pro Lys Pro
20 25 30

Lys Asp Thr Leu Met Ile Ser Arg Thr Pro Glu Val Thr Cys Val Val
35 40 45

Val Asp Val Ser His Glu Asp Pro Glu Val Lys Phe Asn Trp Tyr Val
50 55 60

Asp Gly Val Glu Val His Asn Ala Lys Thr Lys Pro Arg Glu Glu Gln
65 70 75 80

Tyr Asn Ser Thr Tyr Arg Val Val Ser Val Leu Thr Val Leu His Gln
85 90 95

Asp Trp Leu Asn Gly Lys Glu Tyr Lys Cys Lys Val Ser Asn Lys Ala
100 105 110

Leu Pro Ala Pro Ile Glu Lys Thr Ile Ser Lys Ala Lys Gly Gln Pro
115 120 125

Arg Glu Pro Gln Val Tyr Thr Leu Pro Pro Ser Arg Glu Glu Met Thr
130 135 140

Lys Asn Gln Val Ser Leu Thr Cys Leu Val Lys Gly Phe Tyr Pro Ser
145 150 155 160

Asp Ile Ala Val Glu Trp Glu Ser Asn Gly Gln Pro Glu Asn Asn Tyr
165 170 175

Lys Thr Thr Pro Pro Val Leu Asp Ser Asp Gly Ser Phe Phe Leu Tyr
180 185 190

Ser Lys Leu Thr Val Asp Lys Ser Arg Trp Gln Gln Gly Asn Val Phe
195 200 205

Ser Cys Ser Val Met His Glu Ala Leu His Asn His Tyr Thr Gln Lys
210 215 220

Ser Leu Ser Leu Ser Pro Gly Lys
225 230

<210> 27
<211> 228
<212> PRT
<213> Homo sapiens

<400> 27
Glu Arg Lys Cys Cys Val Glu Cys Pro Pro Cys Pro Ala Pro Pro Val
1 5 10 15

Ala Gly Pro Ser Val Phe Leu Phe Pro Pro Lys Pro Lys Asp Thr Leu
20 25 30

Met Ile Ser Arg Thr Pro Glu Val Thr Cys Val Val Val Asp Val Ser
35 40 45

His Glu Asp Pro Glu Val Gln Phe Asn Trp Tyr Val Asp Gly Met Glu
50 55 60

Val His Asn Ala Lys Thr Lys Pro Arg Glu Glu Gln Phe Asn Ser Thr
65 70 75 80

```
Phe Arg Val Val Ser Val Leu Thr Val Val His Gln Asp Trp Leu Asn
                85                  90                  95

Gly Lys Glu Tyr Lys Cys Lys Val Ser Asn Lys Gly Leu Pro Ala Pro
            100                 105                 110

Ile Glu Lys Thr Ile Ser Lys Thr Lys Gly Gln Pro Arg Glu Pro Gln
        115                 120                 125

Val Tyr Thr Leu Pro Pro Ser Arg Glu Glu Met Thr Lys Asn Gln Val
    130                 135                 140

Ser Leu Thr Cys Leu Val Lys Gly Phe Tyr Pro Ser Asp Ile Ala Val
145                 150                 155                 160

Glu Trp Glu Ser Asn Gly Gln Pro Glu Asn Asn Tyr Lys Thr Thr Pro
                165                 170                 175

Pro Met Leu Asp Ser Asp Gly Ser Phe Phe Leu Tyr Ser Lys Leu Thr
            180                 185                 190

Val Asp Lys Ser Arg Trp Gln Gln Gly Asn Val Phe Ser Cys Ser Val
        195                 200                 205

Met His Glu Ala Leu His Asn His Tyr Thr Gln Lys Ser Leu Ser Leu
    210                 215                 220

Ser Pro Gly Lys
225


<210> 28
<211> 279
<212> PRT
<213> Homo sapiens

<400> 28
Glu Leu Lys Thr Pro Leu Gly Asp Thr Thr His Thr Cys Pro Arg Cys
1               5                   10                  15

Pro Glu Pro Lys Ser Cys Asp Thr Pro Pro Pro Cys Pro Arg Cys Pro
            20                  25                  30

Glu Pro Lys Ser Cys Asp Thr Pro Pro Pro Cys Pro Arg Cys Pro Glu
        35                  40                  45

Pro Lys Ser Cys Asp Thr Pro Pro Pro Cys Pro Arg Cys Pro Ala Pro
    50                  55                  60

Glu Leu Leu Gly Gly Pro Ser Val Phe Leu Phe Pro Pro Lys Pro Lys
```

65 70 75 80

Asp Thr Leu Met Ile Ser Arg Thr Pro Glu Val Thr Cys Val Val Val
85 90 95

Asp Val Ser His Glu Asp Pro Glu Val Gln Phe Lys Trp Tyr Val Asp
100 105 110

Gly Val Glu Val His Asn Ala Lys Thr Lys Pro Arg Glu Glu Gln Tyr
115 120 125

Asn Ser Thr Phe Arg Val Val Ser Val Leu Thr Val Leu His Gln Asp
130 135 140

Trp Leu Asn Gly Lys Glu Tyr Lys Cys Lys Val Ser Asn Lys Ala Leu
145 150 155 160

Pro Ala Pro Ile Glu Lys Thr Ile Ser Lys Thr Lys Gly Gln Pro Arg
165 170 175

Glu Pro Gln Val Tyr Thr Leu Pro Pro Ser Arg Glu Glu Met Thr Lys
180 185 190

Asn Gln Val Ser Leu Thr Cys Leu Val Lys Gly Phe Tyr Pro Ser Asp
195 200 205

Ile Ala Val Glu Trp Glu Ser Ser Gly Gln Pro Glu Asn Asn Tyr Asn
210 215 220

Thr Thr Pro Pro Met Leu Asp Ser Asp Gly Ser Phe Phe Leu Tyr Ser
225 230 235 240

Lys Leu Thr Val Asp Lys Ser Arg Trp Gln Gln Gly Asn Ile Phe Ser
245 250 255

Cys Ser Val Met His Glu Ala Leu His Asn Arg Phe Thr Gln Lys Ser
260 265 270

Leu Ser Leu Ser Pro Gly Lys
275

<210> 29
<211> 229
<212> PRT
<213> Homo sapiens

<400> 29
Glu Ser Lys Tyr Gly Pro Pro Cys Pro Ser Cys Pro Ala Pro Glu Phe
1 5 10 15

Leu Gly Gly Pro Ser Val Phe Leu Phe Pro Pro Lys Pro Lys Asp Thr
20 25 30

Leu Met Ile Ser Arg Thr Pro Glu Val Thr Cys Val Val Val Asp Val
35 40 45

Ser Gln Glu Asp Pro Glu Val Gln Phe Asn Trp Tyr Val Asp Gly Val
50 55 60

Glu Val His Asn Ala Lys Thr Lys Pro Arg Glu Glu Gln Phe Asn Ser
65 70 75 80

Thr Tyr Arg Val Val Ser Val Leu Thr Val Leu His Gln Asp Trp Leu
85 90 95

Asn Gly Lys Glu Tyr Lys Cys Lys Val Ser Asn Lys Gly Leu Pro Ser
100 105 110

Ser Ile Glu Lys Thr Ile Ser Lys Ala Lys Gly Gln Pro Arg Glu Pro
115 120 125

Gln Val Tyr Thr Leu Pro Pro Ser Gln Glu Glu Met Thr Lys Asn Gln
130 135 140

Val Ser Leu Thr Cys Leu Val Lys Gly Phe Tyr Pro Ser Asp Ile Ala
145 150 155 160

Val Glu Trp Glu Ser Asn Gly Gln Pro Glu Asn Asn Tyr Lys Thr Thr
165 170 175

Pro Pro Val Leu Asp Ser Asp Gly Ser Phe Phe Leu Tyr Ser Arg Leu
180 185 190

Thr Val Asp Lys Ser Arg Trp Gln Glu Gly Asn Val Phe Ser Cys Ser
195 200 205

Val Met His Glu Ala Leu His Asn His Tyr Thr Gln Lys Ser Leu Ser
210 215 220

Leu Ser Leu Gly Lys
225

<210> 30
<211> 9
<212> PRT
<213> Artificial Sequence

<220>
<221> source
<223> /note="Description of Artificial Sequence: Synthetic peptide"

<220>
<221> MOD_RES
<222> (2)..(9)
<223> Any amino acid

<400> 30
Cys Xaa Xaa Xaa Xaa Xaa Xaa Xaa Xaa
1               5

<210> 31
<211> 5
<212> PRT
<213> Artificial Sequence

<220>
<221> source
<223> /note="Description of Artificial Sequence: Synthetic peptide"

<400> 31
Leu Glu Trp Ile Gly
1               5

<210> 32
<211> 4
<212> PRT
<213> Artificial Sequence

<220>
<221> source
<223> /note="Description of Artificial Sequence: Synthetic peptide"

<220>
<221> MOD_RES
<222> (3)..(3)
<223> Any amino acid

<400> 32
Trp Gly Xaa Gly
1

<210> 33
<211> 4
<212> PRT
<213> Artificial Sequence

<220>
<221> source
<223> /note="Description of Artificial Sequence: Synthetic peptide"

<220>
<221> MOD_RES
<222> (3)..(3)
<223> Any amino acid

<400> 33
Phe Gly Xaa Gly
1

<210> 34
<211> 5
<212> PRT
<213> Artificial Sequence

<220>
<221> source
<223> /note="Description of Artificial Sequence: Synthetic peptide"

<400> 34
Thr Val Ala Ala Pro
1               5

<210> 35
<211> 6
<212> PRT
<213> Artificial Sequence

<220>
<221> source
<223> /note="Description of Artificial Sequence: Synthetic peptide"

<400> 35
Ala Ser Thr Lys Gly Pro
1               5

<210> 36
<211> 10
<212> PRT
<213> Artificial Sequence

<220>
<221> source
<223> /note="Description of Artificial Sequence: Synthetic peptide"

<400> 36
Gly Gly Gly Gly Ser Gly Gly Gly Gly Ser
1               5                   10

<210> 37
<211> 8
<212> PRT
<213> Artificial Sequence

<220>
<221> source
<223> /note="Description of Artificial Sequence: Synthetic

peptide"

<400> 37
Gly Gly Gly Gly Ser Ala Ala Ala
1               5

<210> 38
<211> 15
<212> PRT
<213> Artificial Sequence

<220>
<221> source
<223> /note="Description of Artificial Sequence: Synthetic
peptide"

<400> 38
Gly Gly Gly Gly Ser Gly Gly Gly Gly Ser Gly Gly Gly Gly Ser
1               5                   10                  15

<210> 39
<211> 186
<212> PRT
<213> Homo sapiens

<400> 39
Gln Asp Gly Asn Glu Glu Met Gly Gly Ile Thr Gln Thr Pro Tyr Lys
1               5                   10                  15

Val Ser Ile Ser Gly Thr Thr Val Ile Leu Thr Cys Pro Gln Tyr Pro
            20                  25                  30

Gly Ser Glu Ile Leu Trp Gln His Asn Asp Lys Asn Ile Gly Gly Asp
        35                  40                  45

Glu Asp Asp Lys Asn Ile Gly Ser Asp Glu Asp His Leu Ser Leu Lys
    50                  55                  60

Glu Phe Ser Glu Leu Glu Gln Ser Gly Tyr Tyr Val Cys Tyr Pro Arg
65                  70                  75                  80

Gly Ser Lys Pro Glu Asp Ala Asn Phe Tyr Leu Tyr Leu Arg Ala Arg
                85                  90                  95

Val Cys Glu Asn Cys Met Glu Met Asp Val Met Ser Val Ala Thr Ile
            100                 105                 110

Val Ile Val Asp Ile Cys Ile Thr Gly Gly Leu Leu Leu Leu Val Tyr
        115                 120                 125

Tyr Trp Ser Lys Asn Arg Lys Ala Lys Ala Lys Pro Val Thr Arg Gly
    130                 135                 140

Ala Gly Ala Gly Gly Arg Gln Arg Gly Gln Asn Lys Glu Arg Pro Pro
145 150 155 160

Pro Val Pro Asn Pro Asp Tyr Glu Pro Ile Arg Lys Gly Gln Arg Asp
165 170 175

Leu Tyr Ser Gly Leu Asn Gln Arg Arg Ile
180 185

<210> 40
<211> 177
<212> PRT
<213> Macaca fascicularis

<400> 40
Gln Asp Gly Asn Glu Glu Met Gly Ser Ile Thr Gln Thr Pro Tyr Gln
1 5 10 15

Val Ser Ile Ser Gly Thr Thr Val Ile Leu Thr Cys Ser Gln His Leu
20 25 30

Gly Ser Glu Ala Gln Trp Gln His Asn Gly Lys Asn Lys Gly Asp Ser
35 40 45

Gly Asp Gln Leu Phe Leu Pro Glu Phe Ser Glu Met Glu Gln Ser Gly
50 55 60

Tyr Tyr Val Cys Tyr Pro Arg Gly Ser Asn Pro Glu Asp Ala Ser His
65 70 75 80

His Leu Tyr Leu Lys Ala Arg Val Cys Glu Asn Cys Met Glu Met Asp
85 90 95

Val Met Ala Val Ala Thr Ile Val Ile Val Asp Ile Cys Ile Thr Leu
100 105 110

Gly Leu Leu Leu Leu Val Tyr Tyr Trp Ser Lys Asn Arg Lys Ala Lys
115 120 125

Ala Lys Pro Val Thr Arg Gly Ala Gly Ala Gly Gly Arg Gln Arg Gly
130 135 140

Gln Asn Lys Glu Arg Pro Pro Pro Val Pro Asn Pro Asp Tyr Glu Pro
145 150 155 160

Ile Arg Lys Gly Gln Gln Asp Leu Tyr Ser Gly Leu Asn Gln Arg Arg
165 170 175

Ile

<210> 41
<211> 5
<212> PRT
<213> Artificial Sequence

<220>
<221> source
<223> /note="Description of Artificial Sequence: Synthetic
      peptide"

<400> 41
Gln Asp Gly Asn Glu
1               5

<210> 42
<211> 537
<212> PRT
<213> Artificial Sequence

<220>
<221> source
<223> /note="Description of Artificial Sequence: Synthetic
      polypeptide"

<400> 42
Met Gly Ser Thr Ala Ile Leu Gly Leu Leu Leu Ala Val Leu Gln Gly
1               5                   10                  15

Gly Arg Ala Glu Val Gln Leu Leu Glu Gln Ser Gly Ala Glu Leu Val
            20                  25                  30

Arg Pro Gly Ala Leu Val Lys Leu Ser Cys Lys Ala Ser Gly Phe Lys
        35                  40                  45

Ile Lys Asp Tyr Phe Val Asn Trp Val Lys Gln Arg Pro Glu Gln Gly
    50                  55                  60

Leu Glu Trp Ile Gly Trp Ile Asp Pro Glu Asn Asp Asn Ser Leu Tyr
65                  70                  75                  80

Gly Pro Asn Phe Gln Asp Lys Ala Ser Ile Thr Ala Asp Thr Ser Ser
                85                  90                  95

Asn Thr Gly Tyr Leu Gln Leu Ser Gly Leu Thr Ser Glu Asp Thr Ala
            100                 105                 110

Val Tyr Tyr Cys Ala Leu Tyr Tyr Gly Ser Arg Gly Asp Ala Met Asp
        115                 120                 125

Tyr Trp Gly Gln Gly Thr Thr Val Thr Val Ser Ser Gly Gly Gly Gly

130 135 140

Ser Gly Gly Gly Gly Ser Gly Gly Gly Gly Ser Glu Leu Val Met Thr
145 150 155 160

Gln Thr Pro Ser Ser Leu Ser Ala Ser Leu Gly Asp Arg Val Thr Ile
165 170 175

Ser Cys Arg Ala Ser Gln Asp Ile Ser Asn Tyr Leu Asn Trp Tyr Gln
180 185 190

Gln Lys Pro Asp Gly Thr Val Lys Leu Leu Ile Tyr Tyr Thr Ser Arg
195 200 205

Leu His Ser Gly Val Pro Ser Arg Phe Ser Gly Ser Gly Ser Gly Thr
210 215 220

Asp Tyr Ser Leu Thr Ile Ser Asn Leu Glu Gln Glu Asp Ile Ala Thr
225 230 235 240

Tyr Phe Cys Gln Gln Gly Asn Thr Leu Pro Leu Thr Phe Gly Ala Gly
245 250 255

Thr Lys Leu Glu Ile Lys Ser Gly Gly Gly Gly Ser Glu Val Gln Leu
260 265 270

Val Glu Ser Gly Gly Gly Leu Val Gln Pro Gly Gly Ser Leu Lys Leu
275 280 285

Ser Cys Ala Ala Ser Gly Phe Thr Phe Asn Ser Tyr Ala Met Asn Trp
290 295 300

Val Arg Gln Ala Pro Gly Lys Gly Leu Glu Trp Val Ala Arg Ile Arg
305 310 315 320

Ser Lys Tyr Asn Asn Tyr Ala Thr Tyr Tyr Ala Asp Ser Val Lys Gly
325 330 335

Arg Phe Thr Ile Ser Arg Asp Asp Ser Lys Asn Thr Ala Tyr Leu Gln
340 345 350

Met Asn Asn Leu Lys Thr Glu Asp Thr Ala Val Tyr Tyr Cys Val Arg
355 360 365

His Gly Asn Phe Gly Asn Ser Tyr Val Ser Trp Trp Ala Tyr Trp Gly
370 375 380

Gln Gly Thr Leu Val Thr Val Ser Ser Gly Gly Gly Gly Ser Gly Gly
385 390 395 400

Gly Gly Ser Gly Gly Gly Gly Ser Gln Thr Val Val Thr Gln Glu Pro
405 410 415

Ser Leu Thr Val Ser Pro Gly Gly Thr Val Thr Leu Thr Cys Gly Ser
420 425 430

Ser Thr Gly Ala Val Thr Ser Gly Asn Tyr Pro Asn Trp Val Gln Gln
435 440 445

Lys Pro Gly Gln Ala Pro Arg Gly Leu Ile Gly Gly Thr Lys Phe Leu
450 455 460

Ala Pro Gly Thr Pro Ala Arg Phe Ser Gly Ser Leu Leu Gly Gly Lys
465 470 475 480

Ala Ala Leu Thr Leu Ser Gly Val Gln Pro Glu Asp Glu Ala Glu Tyr
485 490 495

Tyr Cys Val Leu Trp Tyr Ser Asn Arg Trp Val Phe Gly Gly Gly Thr
500 505 510

Lys Leu Thr Val Leu Ala Ala Ala Asp Tyr Lys Asp Asp Asp Asp Lys
515 520 525

Gly Ser Ser His His His His His His
530 535

<210> 43
<211> 1611
<212> DNA
<213> Artificial Sequence

<220>
<221> source
<223> /note="Description of Artificial Sequence: Synthetic polynucleotide"

<400> 43
atggggtcaa ccgccatcct tggcctcctc ctggctgtcc tgcagggagg gcgcgccgag 60

gtgcagctgc tcgagcagtc tggagctgag cttgtgaggc caggggcctt agtcaagttg 120

tcctgcaaag cttctggctt caaaattaaa gactactttg tgaactgggt gaagcagagg 180

cctgaacagg gcctggagtg gattggatgg attgatcctg agaatgataa tagtttatat 240

ggcccgaact tccaggacaa ggccagtatc acagcagaca catcctccaa cacaggctac 300

ctgcagctca gcggcctgac atctgaggac actgccgtct attactgtgc tctttattac 360

ggaagtaggg gggatgctat ggactactgg ggccaaggga ccacggtcac cgtctcctca 420

ggtggtggtg gttctggcgg cggcggctcc ggtggtggtg gttctgagct cgtgatgacc 480

cagactccat cctccctgtc tgcctctctg ggagacagag tcaccatcag ttgcagggca 540

agtcaggaca ttagcaatta tttaaactgg tatcagcaga aaccagatgg aactgttaaa 600

ctcctgatct actacacatc aagattacac tcaggagtcc catcaaggtt cagtggcagt 660

gggtctggaa cagattattc tctcaccatt agcaacctgg agcaagaaga tattgccact 720

tacttttgcc aacagggtaa tacgcttccg ctcacgttcg gtgctgggac caagcttgag 780

atcaaatccg gaggtggtgg atccgaggtg cagctggtcg agtctggagg aggattggtg 840

cagcctggag ggtcattgaa actctcatgt gcagcctctg gattcacctt caatagctac 900

gccatgaact gggtccgcca ggctccagga aagggtttgg aatgggttgc tcgcataaga 960

agtaaatata ataattatgc aacatattat gccgattcag tgaaaggcag gttcaccatc 1020

tccagagatg attcaaaaaa cactgcctat ctacaaatga acaacttgaa aactgaggac 1080

actgccgtgt actactgtgt gagacatggg aacttcggta atagctacgt ttcctggtgg 1140

gcttactggg gccaagggac tctggtcacc gtctcctcag gtggtggtgg ttctggcggc 1200

ggcggctccg gtggtggtgg ttctcagact gttgtgactc aggaaccttc actcaccgta 1260

tcacctggtg gaacagtcac actcacttgt ggctcctcga ctggggctgt tacatctggc 1320

aactacccaa actgggtcca acaaaaacca ggtcaggcac cccgtggtct aataggtggg 1380

actaagttcc tcgcccccgg tactcctgcc agattctcag gctccctgct tggaggcaag 1440

gctgccctca ccctctcagg ggtacagcca gaggatgagg cagaatatta ctgtgttcta 1500

tggtacagca accgctgggt gttcggtgga ggaaccaaac tgactgtcct agcggccgca 1560

gactacaaag acgatgacga caagggcagt tctcaccatc accatcacca c 1611

<210> 44
<211> 537
<212> PRT
<213> Artificial Sequence

<220>
<221> source
<223> /note="Description of Artificial Sequence: Synthetic
polypeptide"

<400> 44
Met Gly Ser Thr Ala Ile Leu Gly Leu Leu Leu Ala Val Leu Gln Gly
1 5 10 15

Gly Arg Ala Gln Val Gln Leu Gln Glu Ser Gly Pro Gly Leu Val Lys
20 25 30

Pro Ser Gln Thr Leu Ser Leu Thr Cys Thr Val Ser Gly Gly Ser Ile

35 40 45

Ser Ser Gly Ala Tyr Tyr Trp Thr Trp Ile Arg Gln His Pro Gly Lys
50 55 60

Gly Leu Glu Trp Ile Gly Tyr Ile Tyr Tyr Ser Gly Ser Thr Tyr Tyr
65 70 75 80

Asn Pro Ser Leu Lys Ser Arg Val Ser Ile Ser Ile Asp Thr Ser Lys
85 90 95

Asn Gln Phe Ser Leu Lys Leu Ser Ser Val Thr Ala Ala Asp Thr Ala
100 105 110

Val Tyr Tyr Cys Ala Arg Gly Ser Ser Ser Trp Phe Asp Tyr Trp Gly
115 120 125

Gln Gly Thr Leu Val Thr Val Ser Ser Gly Gly Gly Gly Ser Gly Gly
130 135 140

Gly Gly Ser Gly Gly Gly Gly Ser Gln Ser Val Leu Thr Gln Pro Pro
145 150 155 160

Ser Val Ser Glu Ala Pro Arg Gln Arg Val Thr Ile Ser Cys Ser Gly
165 170 175

Ser Ser Ser Asn Ile Gly Asn Asn Ala Val Asn Trp Tyr Gln Gln Leu
180 185 190

Pro Gly Lys Ala Pro Lys Leu Leu Ile Tyr Tyr Asp Asp Met Leu Ser
195 200 205

Ser Gly Val Ser Asp Arg Phe Ser Gly Ser Lys Ser Gly Thr Ser Ala
210 215 220

Ser Leu Ala Ile Ser Gly Leu Gln Ser Glu Asp Glu Ala Asp Tyr Tyr
225 230 235 240

Cys Ala Ala Trp Asp Asp Ser Leu Asn Gly Val Val Phe Gly Gly Gly
245 250 255

Thr Lys Leu Thr Val Leu Ser Gly Gly Gly Gly Ser Glu Val Gln Leu
260 265 270

Val Glu Ser Gly Gly Gly Leu Val Gln Pro Gly Gly Ser Leu Lys Leu
275 280 285

Ser Cys Ala Ala Ser Gly Phe Thr Phe Asn Ser Tyr Ala Met Asn Trp
    290                 295                 300

Val Arg Gln Ala Pro Gly Lys Gly Leu Glu Trp Val Ala Arg Ile Arg
305                 310                 315                 320

Ser Lys Tyr Asn Asn Tyr Ala Thr Tyr Tyr Ala Asp Ser Val Lys Gly
                325                 330                 335

Arg Phe Thr Ile Ser Arg Asp Asp Ser Lys Asn Thr Ala Tyr Leu Gln
            340                 345                 350

Met Asn Asn Leu Lys Thr Glu Asp Thr Ala Val Tyr Tyr Cys Val Arg
        355                 360                 365

His Gly Asn Phe Gly Asn Ser Tyr Val Ser Trp Trp Ala Tyr Trp Gly
    370                 375                 380

Gln Gly Thr Leu Val Thr Val Ser Ser Gly Gly Gly Gly Ser Gly Gly
385                 390                 395                 400

Gly Gly Ser Gly Gly Gly Gly Ser Gln Thr Val Val Thr Gln Glu Pro
                405                 410                 415

Ser Leu Thr Val Ser Pro Gly Gly Thr Val Thr Leu Thr Cys Gly Ser
            420                 425                 430

Ser Thr Gly Ala Val Thr Ser Gly Asn Tyr Pro Asn Trp Val Gln Gln
        435                 440                 445

Lys Pro Gly Gln Ala Pro Arg Gly Leu Ile Gly Gly Thr Lys Phe Leu
    450                 455                 460

Ala Pro Gly Thr Pro Ala Arg Phe Ser Gly Ser Leu Leu Gly Gly Lys
465                 470                 475                 480

Ala Ala Leu Thr Leu Ser Gly Val Gln Pro Glu Asp Glu Ala Glu Tyr
                485                 490                 495

Tyr Cys Val Leu Trp Tyr Ser Asn Arg Trp Val Phe Gly Gly Gly Thr
            500                 505                 510

Lys Leu Thr Val Leu Ala Ala Ala Asp Tyr Lys Asp Asp Asp Asp Lys
        515                 520                 525

Gly Ser Ser His His His His His His
    530                 535

<210> 45
<211> 1611
<212> DNA
<213> Artificial Sequence

<220>
<221> source
<223> /note="Description of Artificial Sequence: Synthetic polynucleotide"

<400> 45
atggggtcaa ccgccatcct tggcctcctc ctggctgtcc tgcagggagg gcgcgcccag 60

gtgcagctgc aggagtcggg cccaggactg gtgaagcctt cacagaccct gtccctcacc 120

tgcactgtct ctggtggctc catcagcagt ggtgcttact actggacctg gatccgccag 180

cacccaggga agggcctgga gtggattggg tacatctatt acagtgggag cacctactac 240

aacccgtccc tcaagagtcg agttagcata tcaatagaca cgtctaagaa ccagttctcc 300

ctgaagctga gctctgtgac tgccgcggac acggccgtgt attactgtgc gcgaggcagc 360

agcagctggt tcgactactg gggccaggga accctggtca ccgtctcctc aggaggcggc 420

ggttcaggcg gaggtggctc tggcggtggc ggaagtcagt ctgtgctgac tcagccaccc 480

tcggtgtctg aagcccccag gcagagggtc accatctcct gttctggaag cagctccaac 540

atcggaaata atgctgtaaa ctggtaccag cagctcccag gaaaggctcc caaactcctc 600

atctattatg atgatatgtt gtcttcaggg gtctcggacc gatttctggc ctccaagtct 660

ggcacctcag cctccctggc catcagtggg ctccagtctg aggatgaggc tgattattac 720

tgtgcagcat gggatgacag cctgaatggt gtggtattcg gcggagggac caagctgacc 780

gtcctatccg gaggtggtgg atccgaggtg cagctggtcg agtctggagg aggattggtg 840

cagcctggag ggtcattgaa actctcatgt gcagcctctg gattcacctt caatagctac 900

gccatgaact gggtccgcca ggctccagga aagggtttgg aatgggttgc tcgcataaga 960

agtaaatata ataattatgc aacatattat gccgattcag tgaaaggcag gttcaccatc 1020

tccagagatg attcaaaaaa cactgcctat ctacaaatga acaacttgaa aactgaggac 1080

actgccgtgt actactgtgt gagacatggg aacttcggta atagctacgt ttcctggtgg 1140

gcttactggg gccaagggac tctggtcacc gtctcctcag gtggtggtgg ttctggcggc 1200

ggcggctccg gtggtggtgg ttctcagact gttgtgactc aggaaccttc actcaccgta 1260

tcacctggtg gaacagtcac actcacttgt ggctcctcga ctggggctgt tacatctggc 1320

aactacccaa actgggtcca acaaaaacca ggtcaggcac cccgtggtct aataggtggg 1380

actaagttcc tcgcccccgg tactcctgcc agattctcag gctccctgct tggaggcaag 1440

gctgccctca ccctctcagg ggtacagcca gaggatgagg cagaatatta ctgtgttcta 1500

tggtacagca accgctgggt gttcggtgga ggaaccaaac tgactgtcct agcggccgca 1560

gactacaaag acgatgacga caagggcagt tctcaccatc accatcacca c 1611

<210> 46
<211> 711
<212> PRT
<213> Artificial Sequence

<220>
<221> source
<223> /note="Description of Artificial Sequence: Synthetic polypeptide"

<400> 46
Met Gly Ser Thr Ala Ile Leu Gly Leu Leu Leu Ala Val Leu Gln Gly
1 5 10 15

Gly Arg Ala Glu Val Gln Leu Val Glu Ser Gly Gly Gly Leu Val Gln
20 25 30

Pro Gly Gly Ser Leu Lys Leu Ser Cys Ala Ala Ser Gly Phe Thr Phe
35 40 45

Asn Ser Tyr Ala Met Asn Trp Val Arg Gln Ala Pro Gly Lys Gly Leu
50 55 60

Glu Trp Val Ala Arg Ile Arg Ser Lys Tyr Asn Asn Tyr Ala Thr Tyr
65 70 75 80

Tyr Ala Asp Ser Val Lys Gly Arg Phe Thr Ile Ser Arg Asp Asp Ser
85 90 95

Lys Asn Thr Ala Tyr Leu Gln Met Asn Asn Leu Lys Thr Glu Asp Thr
100 105 110

Ala Val Tyr Tyr Cys Val Arg His Gly Asn Phe Gly Asn Ser Tyr Val
115 120 125

Ser Trp Trp Ala Tyr Trp Gly Gln Gly Thr Leu Val Thr Val Ser Ser
130 135 140

Ala Ser Thr Lys Gly Pro Ser Val Phe Pro Leu Ala Pro Ser Ser Lys
145 150 155 160

Ser Thr Ser Gly Gly Thr Ala Ala Leu Gly Cys Leu Val Lys Asp Tyr
165 170 175

Phe Pro Glu Pro Val Thr Val Ser Trp Asn Ser Gly Ala Leu Thr Ser
180 185 190

Gly Val His Thr Phe Pro Ala Val Leu Gln Ser Ser Gly Leu Tyr Ser
195 200 205

Leu Ser Ser Val Val Thr Val Pro Ser Ser Ser Leu Gly Thr Gln Thr
210 215 220

Tyr Ile Cys Asn Val Asn His Lys Pro Ser Asn Thr Lys Val Asp Lys
225 230 235 240

Arg Val Glu Pro Lys Ser Cys Asp Lys Thr His Thr Cys Pro Pro Cys
245 250 255

Pro Ala Pro Glu Ala Ala Gly Gly Pro Ser Val Phe Leu Phe Pro Pro
260 265 270

Lys Pro Lys Asp Thr Leu Met Ile Ser Arg Thr Pro Glu Val Thr Cys
275 280 285

Val Val Val Asp Val Ser His Glu Asp Pro Glu Val Lys Phe Asn Trp
290 295 300

Tyr Val Asp Gly Val Glu Val His Asn Ala Lys Thr Lys Pro Arg Glu
305 310 315 320

Glu Gln Tyr Asn Ser Thr Tyr Arg Val Val Ser Val Leu Thr Val Leu
325 330 335

His Gln Asp Trp Leu Asn Gly Lys Glu Tyr Lys Cys Lys Val Ser Asn
340 345 350

Lys Ala Leu Pro Ala Pro Ile Glu Lys Thr Ile Ser Lys Ala Lys Gly
355 360 365

Gln Pro Arg Glu Pro Gln Val Tyr Thr Leu Pro Pro Ser Arg Lys Glu
370 375 380

Met Thr Lys Asn Gln Val Ser Leu Thr Cys Leu Val Lys Gly Phe Tyr
385 390 395 400

Pro Ser Asp Ile Ala Val Glu Trp Glu Ser Asn Gly Gln Pro Glu Asn
405 410 415

Asn Tyr Lys Thr Thr Pro Pro Val Leu Lys Ser Asp Gly Ser Phe Phe
420 425 430

Leu Tyr Ser Lys Leu Thr Val Asp Lys Ser Arg Trp Gln Gln Gly Asn
435 440 445

Val Phe Ser Cys Ser Val Met His Glu Ala Leu His Asn His Tyr Thr
450 455 460

Gln Lys Ser Leu Ser Leu Ser Pro Gly Lys Ala Ala Ala Gly Gly Gly
465 470 475 480

Gly Ser Gly Gly Gly Gly Ser Glu Val Gln Leu Leu Glu Gln Ser Gly
485 490 495

Ala Glu Leu Val Arg Pro Gly Ala Leu Val Lys Leu Ser Cys Lys Ala
500 505 510

Ser Gly Phe Lys Ile Lys Asp Tyr Phe Val Asn Trp Val Lys Gln Arg
515 520 525

Pro Glu Gln Gly Leu Glu Trp Ile Gly Trp Ile Asp Pro Glu Asn Asp
530 535 540

Asn Ser Leu Tyr Gly Pro Asn Phe Gln Asp Lys Ala Ser Ile Thr Ala
545 550 555 560

Asp Thr Ser Ser Asn Thr Gly Tyr Leu Gln Leu Ser Gly Leu Thr Ser
565 570 575

Glu Asp Thr Ala Val Tyr Tyr Cys Ala Leu Tyr Tyr Gly Ser Arg Gly
580 585 590

Asp Ala Met Asp Tyr Trp Gly Gln Gly Thr Thr Val Thr Val Ser Ser
595 600 605

Ala Ser Thr Lys Gly Pro Ser Val Phe Pro Leu Ala Pro Ser Ser Lys
610 615 620

Ser Thr Ser Gly Gly Thr Ala Ala Leu Gly Cys Leu Val Lys Asp Tyr
625 630 635 640

Phe Pro Glu Pro Val Thr Val Ser Trp Asn Ser Gly Ala Leu Thr Ser
645 650 655

Gly Val His Thr Phe Pro Ala Val Leu Gln Ser Ser Gly Leu Tyr Ser
660 665 670

Leu Ser Ser Val Val Thr Val Pro Ser Ser Ser Leu Gly Thr Gln Thr
675 680 685

Tyr Ile Cys Asn Val Asn His Lys Pro Ser Asn Thr Lys Val Asp Lys
690 695 700

Arg Val Glu Pro Lys Ser Cys
705                 710

<210> 47
<211> 2133
<212> DNA
<213> Artificial Sequence

<220>
<221> source
<223> /note="Description of Artificial Sequence: Synthetic
      polynucleotide"

<400> 47
atggggtcaa ccgccatcct tggcctcctc ctggctgtcc tgcagggagg gcgcgccgag 60

gtgcagctgg tcgagtctgg aggaggattg gtgcagcctg gagggtcatt gaaactctca 120

tgtgcagcct ctggattcac cttcaatagc tacgccatga actgggtccg ccaggctcca 180

ggaaagggtt tggaatgggt tgctcgcata agaagtaaat ataataatta tgcaacatat 240

tatgccgatt cagtgaaagg caggttcacc atctccagag atgattcaaa aaacactgcc 300

tatctacaaa tgaacaactt gaaaactgag gacactgccg tgtactactg tgtgagacat 360

gggaacttcg gtaatagcta cgtttcctgg tgggcttact ggggccaagg gactctggtc 420

accgtctcct cagctagcac caagggccca tccgtcttcc ccctggcacc ctcctccaag 480

agcacctctg ggggcacagc ggccctgggc tgcctggtca aggactactt ccccgaaccg 540

gtgacggtgt cgtggaactc aggcgccctg accagcggcg tgcacacctt cccggctgtc 600

ctacagtcct caggactcta ctccctcagc agcgtggtga ccgtgccctc cagcagcttg 660

ggcacccaga cctacatctg caacgtgaat cacaagccca gcaacaccaa ggtggacaag 720

agagttgagc ccaaatcttg tgacaaaact cacacatgcc caccgtgccc agcacctgaa 780

gcagctgggg gaccgtcagt cttcctcttc cccccaaaac ccaaggacac cctcatgatc 840

tcccggaccc ctgaggtcac atgcgtggtg gtggacgtga gccacgaaga ccctgaggtc 900

aagttcaact ggtacgtgga cggcgtggag gtgcataatg ccaagacaaa gccgcgggag 960

gagcagtaca acagcacgta ccgtgtggtc agcgtcctca ccgtcctgca ccaggactgg 1020

ctgaatggca aggagtacaa gtgcaaggtc tccaacaaag ccctcccagc ccccatcgag 1080

aaaaccatct ccaaagccaa agggcagccc cgagaaccac aggtgtacac cctgccccca 1140

tcccggaagg agatgaccaa gaaccaggtc agcctgacct gcctggtcaa aggcttctat 1200

cccagcgaca tcgccgtgga gtgggagagc aatgggcagc cggagaacaa ctacaagacc 1260

acgcctcccg tgctgaagtc cgacggctcc ttcttcctct atagcaagct caccgtggac 1320

aagagcaggt ggcagcaggg gaacgtcttc tcatgctccg tgatgcatga ggctctgcac 1380

aaccactaca cgcagaagag cctctccctg tctccgggta aagcggccgc tggaggcggc 1440

ggttcaggcg gaggtggctc tgaggtgcag ctgctcgagc agtctggagc tgagcttgtg 1500

aggccagggg ccttagtcaa gttgtcctgc aaagcttctg gcttcaaaat taaagactac 1560

tttgtgaact gggtgaagca gaggcctgaa cagggcctgg agtggattgg atggattgat 1620

cctgagaatg ataatagttt atatggcccg aacttccagg acaaggccag tatcacagca 1680

gacacatcct ccaacacagg ctacctgcag ctcagcggcc tgacatctga ggacactgcc 1740

gtctattact gtgctcttta ttacggaagt agggggggatg ctatggacta ctggggccaa 1800

gggaccacgg tcaccgtctc ctcagctagc accaagggcc catccgtctt ccccctggca 1860

ccctcctcca agagcacctc tgggggcaca gcggccctgg gctgcctggt caaggactac 1920

ttccccgaac cggtgacggt gtcgtggaac tcaggcgccc tgaccagcgg cgtgcacacc 1980

ttcccggctg tcctacagtc ctcaggactc tactccctca gcagcgtggt gaccgtgccc 2040

tccagcagct tgggcaccca gacctacatc tgcaacgtga atcacaagcc cagcaacacc 2100

aaggtggaca agagagttga gcccaaatct tgt 2133

<210> 48
<211> 688
<212> PRT
<213> Artificial Sequence

<220>
<221> source
<223> /note="Description of Artificial Sequence: Synthetic polypeptide"

<400> 48
Met Gly Ser Thr Ala Ile Leu Gly Leu Leu Leu Ala Val Leu Gln Gly
1 5 10 15

Gly Arg Ala Gln Thr Val Val Thr Gln Glu Pro Ser Leu Thr Val Ser
20 25 30

Pro Gly Gly Thr Val Thr Leu Thr Cys Gly Ser Ser Thr Gly Ala Val
35 40 45

Thr Ser Gly Asn Tyr Pro Asn Trp Val Gln Gln Lys Pro Gly Gln Ala
50 55 60

Pro Arg Gly Leu Ile Gly Gly Thr Lys Phe Leu Ala Pro Gly Thr Pro
65 70 75 80

Ala Arg Phe Ser Gly Ser Leu Leu Gly Gly Lys Ala Ala Leu Thr Leu
85 90 95

Ser Gly Val Gln Pro Glu Asp Glu Ala Glu Tyr Tyr Cys Val Leu Trp

```
            100                 105                 110

Tyr Ser Asn Arg Trp Val Phe Gly Gly Gly Thr Lys Leu Thr Val Leu
        115                 120                 125

Gly Gln Pro Lys Ala Ala Pro Ser Val Thr Leu Phe Pro Pro Ser Ser
    130                 135                 140

Glu Glu Leu Gln Ala Asn Lys Ala Thr Leu Val Cys Leu Ile Ser Asp
145                 150                 155                 160

Phe Tyr Pro Gly Ala Val Thr Val Ala Trp Lys Ala Asp Ser Ser Pro
                165                 170                 175

Val Lys Ala Gly Val Glu Thr Thr Thr Pro Ser Lys Gln Ser Asn Asn
            180                 185                 190

Lys Tyr Ala Ala Ser Ser Tyr Leu Ser Leu Thr Pro Glu Gln Trp Lys
        195                 200                 205

Ser His Arg Ser Tyr Ser Cys Gln Val Thr His Glu Gly Ser Thr Val
    210                 215                 220

Glu Lys Thr Val Ala Pro Thr Glu Cys Ser Asp Lys Thr His Thr Cys
225                 230                 235                 240

Pro Pro Cys Pro Ala Pro Glu Ala Ala Gly Gly Pro Ser Val Phe Leu
                245                 250                 255

Phe Pro Pro Lys Pro Lys Asp Thr Leu Met Ile Ser Arg Thr Pro Glu
            260                 265                 270

Val Thr Cys Val Val Val Asp Val Ser His Glu Asp Pro Glu Val Lys
        275                 280                 285

Phe Asn Trp Tyr Val Asp Gly Val Glu Val His Asn Ala Lys Thr Lys
    290                 295                 300

Pro Arg Glu Glu Gln Tyr Asn Ser Thr Tyr Arg Val Val Ser Val Leu
305                 310                 315                 320

Thr Val Leu His Gln Asp Trp Leu Asn Gly Lys Glu Tyr Lys Cys Lys
                325                 330                 335

Val Ser Asn Lys Ala Leu Pro Ala Pro Ile Glu Lys Thr Ile Ser Lys
            340                 345                 350
```

Ala Lys Gly Gln Pro Arg Glu Pro Gln Val Tyr Thr Leu Pro Pro Ser
355 360 365

Arg Glu Glu Met Thr Lys Asn Gln Val Ser Leu Thr Cys Leu Val Lys
370 375 380

Gly Phe Tyr Pro Ser Asp Ile Ala Val Glu Trp Glu Ser Asn Gly Gln
385 390 395 400

Pro Glu Asn Asn Tyr Asp Thr Thr Pro Pro Val Leu Asp Ser Asp Gly
405 410 415

Ser Phe Phe Leu Tyr Ser Glu Leu Thr Val Asp Lys Ser Arg Trp Gln
420 425 430

Gln Gly Asn Val Phe Ser Cys Ser Val Met His Glu Ala Leu His Asn
435 440 445

His Tyr Thr Gln Lys Ser Leu Ser Leu Ser Pro Gly Lys Ala Ala Ala
450 455 460

Gly Gly Gly Gly Ser Gly Gly Gly Gly Ser Glu Leu Val Met Thr Gln
465 470 475 480

Thr Pro Ser Ser Leu Ser Ala Ser Leu Gly Asp Arg Val Thr Ile Ser
485 490 495

Cys Arg Ala Ser Gln Asp Ile Ser Asn Tyr Leu Asn Trp Tyr Gln Gln
500 505 510

Lys Pro Asp Gly Thr Val Lys Leu Leu Ile Tyr Tyr Thr Ser Arg Leu
515 520 525

His Ser Gly Val Pro Ser Arg Phe Ser Gly Ser Gly Ser Gly Thr Asp
530 535 540

Tyr Ser Leu Thr Ile Ser Asn Leu Glu Gln Glu Asp Ile Ala Thr Tyr
545 550 555 560

Phe Cys Gln Gln Gly Asn Thr Leu Pro Leu Thr Phe Gly Ala Gly Thr
565 570 575

Lys Leu Glu Ile Lys Arg Thr Val Ala Ala Pro Ser Val Phe Ile Phe
580 585 590

Pro Pro Ser Asp Glu Gln Leu Lys Ser Gly Thr Ala Ser Val Val Cys
595 600 605

Leu Leu Asn Asn Phe Tyr Pro Arg Glu Ala Lys Val Gln Trp Lys Val
610 615 620

Asp Asn Ala Leu Gln Ser Gly Asn Ser Gln Glu Ser Val Thr Glu Gln
625 630 635 640

Asp Ser Lys Asp Ser Thr Tyr Ser Leu Ser Ser Thr Leu Thr Leu Ser
645 650 655

Lys Ala Asp Tyr Glu Lys His Lys Val Tyr Ala Cys Glu Val Thr His
660 665 670

Gln Gly Leu Ser Ser Pro Val Thr Lys Ser Phe Asn Arg Gly Glu Cys
675 680 685

<210> 49
<211> 2064
<212> DNA
<213> Artificial Sequence

<220>
<221> source
<223> /note="Description of Artificial Sequence: Synthetic polynucleotide"

<400> 49
atggggtcaa ccgccatcct tggcctcctc ctggctgtcc tgcagggagg gcgcgcccag 60

actgttgtga ctcaggaacc ttcactcacc gtatcacctg gtggaacagt cacactcact 120

tgtggctcct cgactggggc tgttacatct ggcaactacc caaactgggt ccaacaaaaa 180

ccaggtcagg caccccgtgg tctaataggt gggactaagt tcctcgcccc cggtactcct 240

gccagattct caggctccct gcttggaggc aaggctgccc tcaccctctc aggggtacag 300

ccagaggatg aggcagaata ttactgtgtt ctatggtaca gcaaccgctg ggtgttcggt 360

ggaggaacca aactgactgt cctaggtcag cccaaggctg ccccctcggt cactctgttc 420

ccgccctcct ctgaggagct tcaagccaac aaggccacac tggtgtgtct cataagtgac 480

ttctacccgg gagccgtgac agtggcctgg aaggcagata gcagccccgt caaggcggga 540

gtggagacca ccacaccctc caaacaaagc aacaacaagt acgcggccag cagctatctg 600

agcctgacgc ctgagcagtg gaagtcccac agaagctaca gctgccaggt cacgcatgaa 660

gggagcaccg tggagaagac agtggcccct acagaatgtt cagacaaaac tcacacatgc 720

ccaccgtgcc cagcacctga agcagctggg ggaccgtcag tcttcctctt ccccccaaaa 780

cccaaggaca ccctcatgat ctcccggacc cctgaggtca catgcgtggt ggtggacgtg 840

agccacgaag accctgaggt caagttcaac tggtacgtgg acggcgtgga ggtgcataat 900

gccaagacaa agccgcggga ggagcagtac aacagcacgt accgtgtggt cagcgtcctc 960

```
accgtcctgc accaggactg gctgaatggc aaggagtaca agtgcaaggt ctccaacaaa     1020
gccctcccag cccccatcga gaaaaccatc tccaaagcca aagggcagcc ccgagaacca     1080
caggtgtaca ccctgccccc atcccgggag gagatgacca agaaccaggt cagcctgacc     1140
tgcctggtca aaggcttcta tcccagcgac atcgccgtgg agtgggagag caatgggcag     1200
ccggagaaca actacgacac cacgcctccc gtgctggact ccgacggctc cttcttcctc     1260
tatagcgagc tcaccgtgga caagagcagg tggcagcagg ggaacgtctt ctcatgctcc     1320
gtgatgcatg aggctctgca caaccactac acgcagaaga gcctctccct gtctccgggt     1380
aaagcggccg ctggaggcgg cggttcaggc ggaggtggct ctgagctcgt gatgacccag     1440
actccatcct ccctgtctgc ctctctggga gacagagtca ccatcagttg cagggcaagt     1500
caggacatta gcaattattt aaactggtat cagcagaaac cagatggaac tgttaaactc     1560
ctgatctact acacatcaag attacactca ggagtcccat caaggttcag tggcagtggg     1620
tctggaacag attattctct caccattagc aacctggagc aagaagatat tgccacttac     1680
ttttgccaac agggtaatac gcttccgctc acgttcggtg ctgggaccaa gcttgagatc     1740
aaacgtacgg tggctgcacc atctgtcttc atcttcccgc catctgatga gcagttgaaa     1800
tctggaactg cctctgttgt gtgcctgctg aataacttct atcccagaga ggccaaagta     1860
cagtggaagg tggataacgc cctccaatcg ggtaactccc aggagagtgt cacagagcag     1920
gacagcaagg acagcaccta cagcctcagc agcaccctga cgctgagcaa agcagactac     1980
gagaaacaca aagtctacgc ctgcgaagtc acccatcagg gcctgagctc gcccgtcaca     2040
aagagcttca acaggggaga gtgt                                            2064


<210> 50
<211> 50
<212> PRT
<213> Artificial Sequence

<220>
<221> source
<223> /note="Description of Artificial Sequence: Synthetic
      polypeptide"


<220>
<221> VARIANT
<222> (6)..(50)
<223> /replace=" "

<220>
<221> misc_feature
<222> (1)..(50)
<223> /note="This sequence may encompass 1-10 'Gly Gly Gly Gly Ser'
      repeating units"

<220>
```

<221> misc_feature
<222> (1)..(50)
<223> /note="Variant residues given in the sequence have no preference with respect to those in the annotations for variant positions"

<400> 50
Gly Gly Gly Gly Ser Gly Gly Gly Gly Ser Gly Gly Gly Gly Ser Gly
1               5                   10                  15

Gly Gly Gly Ser Gly Gly Gly Gly Ser Gly Gly Gly Gly Ser Gly Gly
            20                  25                  30

Gly Gly Ser Gly Gly Gly Gly Ser Gly Gly Gly Gly Ser Gly Gly Gly
        35                  40                  45

Gly Ser
    50

<210> 51
<211> 8
<212> PRT
<213> Artificial Sequence

<220>
<221> source
<223> /note="Description of Artificial Sequence: Synthetic peptide"

<400> 51
Gly Pro Leu Gly Ile Ala Gly Gln
1               5

<210> 52
<211> 6
<212> PRT
<213> Artificial Sequence

<220>
<221> source
<223> /note="Description of Artificial Sequence: Synthetic peptide"

<400> 52
Arg Arg Arg Arg Arg Arg
1               5

<210> 53
<211> 16
<212> PRT
<213> Artificial Sequence

<220>
<221> source
<223> /note="Description of Artificial Sequence: Synthetic peptide"

<400> 53
Gly Gly Gly Gly Ser Arg Arg Arg Arg Arg Arg Gly Gly Gly Gly Ser
1 5 10 15

<210> 54
<211> 18
<212> PRT
<213> Artificial Sequence

<220>
<221> source
<223> /note="Description of Artificial Sequence: Synthetic peptide"

<400> 54
Gly Gly Gly Gly Ser Gly Pro Leu Gly Ile Ala Gly Gln Gly Gly Gly
1 5 10 15

Gly Ser

<210> 55
<211> 30
<212> PRT
<213> Artificial Sequence

<220>
<221> source
<223> /note="Description of Artificial Sequence: Synthetic polypeptide"

<400> 55
Gly Gly Gly Gly Ser Gly Gly Gly Gly Ser Gly Leu Glu Val Leu Phe
1 5 10 15

Gln Gly Pro Ser Gly Gly Gly Gly Ser Gly Gly Gly Gly Ser
20 25 30

<210> 56
<211> 30
<212> PRT
<213> Artificial Sequence

<220>
<221> source
<223> /note="Description of Artificial Sequence: Synthetic polypeptide"

<400> 56
Gly Gly Gly Gly Ser Gly Gly Gly Gly Ser Gly Gln Ser Ser Arg His
1 5 10 15

Arg Arg Ala Leu Gly Gly Gly Gly Ser Gly Gly Gly Gly Ser
20 25 30

<210> 57

```
<211> 30
<212> PRT
<213> Artificial Sequence

<220>
<221> source
<223> /note="Description of Artificial Sequence: Synthetic
      polypeptide"

<400> 57
Gly Gly Gly Gly Ser Gly Gly Gly Gly Ser Gly Gly Pro Leu Gly Met
1               5                   10                  15

Leu Ser Gln Ser Gly Gly Gly Gly Ser Gly Gly Gly Gly Ser
            20                  25                  30


<210> 58
<211> 29
<212> PRT
<213> Artificial Sequence

<220>
<221> source
<223> /note="Description of Artificial Sequence: Synthetic
      peptide"

<400> 58
Gly Gly Gly Gly Ser Gly Gly Gly Gly Ser Gly Gly Gly Gly Arg Arg
1               5                   10                  15

Gly Gly Ser Gly Gly Gly Gly Ser Gly Gly Gly Gly Ser
            20                  25


<210> 59
<211> 8
<212> PRT
<213> Artificial Sequence

<220>
<221> source
<223> /note="Description of Artificial Sequence: Synthetic
      peptide"

<400> 59
Ala Val Arg Trp Leu Leu Thr Ala
1               5


<210> 60
<211> 580
<212> PRT
<213> Artificial Sequence

<220>
<221> source
<223> /note="Description of Artificial Sequence: Synthetic
      polypeptide"

<400> 60
```

```
Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Thr Phe Asn Ser Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Gly Arg Phe Thr Ile Ser Arg Asp Asp Ser Lys Asn Thr
65                  70                  75                  80

Ala Tyr Leu Gln Met Asn Asn Leu Lys Thr Glu Asp Thr Ala Val Tyr
                85                  90                  95

Tyr Cys Val Arg His Gly Asn Phe Gly Asn Ser Tyr Val Ser Trp Trp
            100                 105                 110

Ala Tyr Trp Gly Gln Gly Thr Leu Val Thr Val Ser Ser Gly Gly Gly
        115                 120                 125

Gly Ser Gly Gly Gly Gly Ser Glu Leu Val Met Thr Gln Thr Pro Ser
    130                 135                 140

Ser Leu Ser Ala Ser Leu Gly Asp Arg Val Thr Ile Ser Cys Arg Ala
145                 150                 155                 160

Ser Gln Asp Ile Ser Asn Tyr Leu Asn Trp Tyr Gln Gln Lys Pro Asp
                165                 170                 175

Gly Thr Val Lys Leu Leu Ile Tyr Tyr Thr Ser Arg Leu His Ser Gly
            180                 185                 190

Val Pro Ser Arg Phe Ser Gly Ser Gly Ser Gly Thr Asp Tyr Ser Leu
        195                 200                 205

Thr Ile Ser Asn Leu Glu Gln Glu Asp Ile Ala Thr Tyr Phe Cys Gln
    210                 215                 220

Gln Gly Asn Thr Leu Pro Leu Thr Phe Gly Ala Gly Thr Lys Leu Glu
225                 230                 235                 240

Ile Lys Ala Ser Thr Lys Gly Pro Ser Val Phe Pro Leu Ala Pro Cys
                245                 250                 255
```

Ser Arg Ser Thr Ser Glu Ser Thr Ala Ala Leu Gly Cys Leu Val Lys
260 265 270

Asp Tyr Phe Pro Glu Pro Val Thr Val Ser Trp Asn Ser Gly Ala Leu
275 280 285

Thr Ser Gly Val His Thr Phe Pro Ala Val Leu Gln Ser Ser Gly Leu
290 295 300

Tyr Ser Leu Ser Ser Val Val Thr Val Pro Ser Ser Asn Phe Gly Thr
305 310 315 320

Gln Thr Tyr Thr Cys Asn Val Asp His Lys Pro Ser Asn Thr Lys Val
325 330 335

Asp Lys Thr Val Gly Gly Gly Gly Ser Ala Ala Ala Glu Pro Lys Ser
340 345 350

Ser Asp Lys Thr His Thr Cys Pro Pro Cys Pro Ala Pro Glu Ala Ala
355 360 365

Gly Gly Pro Ser Val Phe Leu Phe Pro Pro Lys Pro Lys Asp Thr Leu
370 375 380

Met Ile Ser Arg Thr Pro Glu Val Thr Cys Val Val Val Asp Val Ser
385 390 395 400

His Glu Asp Pro Glu Val Lys Phe Asn Trp Tyr Val Asp Gly Val Glu
405 410 415

Val His Asn Ala Lys Thr Lys Pro Arg Glu Glu Gln Tyr Asn Ser Thr
420 425 430

Tyr Arg Val Val Ser Val Leu Thr Val Leu His Gln Asp Trp Leu Asn
435 440 445

Gly Lys Glu Tyr Lys Cys Lys Val Ser Asn Lys Ala Leu Pro Ala Pro
450 455 460

Ile Glu Lys Thr Ile Ser Lys Ala Lys Gly Gln Pro Arg Glu Pro Gln
465 470 475 480

Val Tyr Thr Leu Pro Pro Ser Arg Glu Glu Met Thr Lys Asn Gln Val
485 490 495

Ser Leu Thr Cys Leu Val Lys Gly Phe Tyr Pro Ser Asp Ile Ala Val
500 505 510

Glu Trp Glu Ser Asn Gly Gln Pro Glu Asn Asn Tyr Asp Thr Thr Pro
515 520 525

Pro Val Leu Asp Ser Asp Gly Ser Phe Phe Leu Tyr Ser Asp Leu Thr
530 535 540

Val Asp Lys Ser Arg Trp Gln Gln Gly Asn Val Phe Ser Cys Ser Val
545 550 555 560

Met His Glu Ala Leu His Asn His Tyr Thr Gln Lys Ser Leu Ser Leu
565 570 575

Ser Pro Gly Lys
580

<210> 61
<211> 590
<212> PRT
<213> Artificial Sequence

<220>
<221> source
<223> /note="Description of Artificial Sequence: Synthetic polypeptide"

<400> 61
Glu Val Gln Leu Leu Glu Gln Ser Gly Ala Glu Leu Val Arg Pro Gly
1 5 10 15

Ala Leu Val Lys Leu Ser Cys Lys Ala Ser Gly Phe Lys Ile Lys Asp
20 25 30

Tyr Phe Val Asn Trp Val Lys Gln Arg Pro Glu Gln Gly Leu Glu Trp
35 40 45

Ile Gly Trp Ile Asp Pro Glu Asn Asp Asn Ser Leu Tyr Gly Pro Asn
50 55 60

Phe Gln Asp Lys Ala Ser Ile Thr Ala Asp Thr Ser Ser Asn Thr Gly
65 70 75 80

Tyr Leu Gln Leu Ser Gly Leu Thr Ser Glu Asp Thr Ala Val Tyr Tyr
85 90 95

Cys Ala Leu Tyr Tyr Gly Ser Arg Gly Asp Ala Met Asp Tyr Trp Gly
100 105 110

Gln Gly Thr Thr Val Thr Val Ser Ser Gly Gly Gly Gly Ser Gly Gly
115 120 125

Gly Gly Ser Gln Thr Val Val Thr Gln Glu Pro Ser Leu Thr Val Ser
130 135 140

Pro Gly Gly Thr Val Thr Leu Thr Cys Gly Ser Ser Thr Gly Ala Val
145 150 155 160

Thr Ser Gly Asn Tyr Pro Asn Trp Val Gln Gln Lys Pro Gly Gln Ala
165 170 175

Pro Arg Gly Leu Ile Gly Gly Thr Lys Phe Leu Ala Pro Gly Thr Pro
180 185 190

Ala Arg Phe Ser Gly Ser Leu Leu Gly Gly Lys Ala Ala Leu Thr Leu
195 200 205

Ser Gly Val Gln Pro Glu Asp Glu Ala Glu Tyr Tyr Cys Val Leu Trp
210 215 220

Tyr Ser Asn Arg Trp Val Phe Gly Gly Gly Thr Lys Leu Thr Val Leu
225 230 235 240

Gly Gln Pro Lys Ala Ala Pro Ser Val Thr Leu Phe Pro Pro Ser Ser
245 250 255

Glu Glu Leu Gln Ala Asn Lys Ala Thr Leu Val Cys Leu Ile Ser Asp
260 265 270

Phe Tyr Pro Gly Ala Val Thr Val Ala Trp Lys Ala Asp Ser Ser Pro
275 280 285

Val Lys Ala Gly Val Glu Thr Thr Thr Pro Ser Lys Gln Ser Asn Asn
290 295 300

Lys Tyr Ala Ala Ser Ser Tyr Leu Ser Leu Thr Pro Glu Gln Trp Lys
305 310 315 320

Ser His Arg Ser Tyr Ser Cys Gln Val Thr His Glu Gly Ser Thr Val
325 330 335

Glu Lys Thr Val Ala Pro Thr Glu Cys Ser Ala Ala Ala Glu Pro Lys
340 345 350

Ser Ser Asp Lys Thr His Thr Cys Pro Pro Cys Pro Ala Pro Glu Ala
355 360 365

Ala Gly Gly Pro Ser Val Phe Leu Phe Pro Pro Lys Pro Lys Asp Thr

370 375 380

Leu Met Ile Ser Arg Thr Pro Glu Val Thr Cys Val Val Val Asp Val
385 390 395 400

Ser His Glu Asp Pro Glu Val Lys Phe Asn Trp Tyr Val Asp Gly Val
405 410 415

Glu Val His Asn Ala Lys Thr Lys Pro Arg Glu Glu Gln Tyr Asn Ser
420 425 430

Thr Tyr Arg Val Val Ser Val Leu Thr Val Leu His Gln Asp Trp Leu
435 440 445

Asn Gly Lys Glu Tyr Lys Cys Lys Val Ser Asn Lys Ala Leu Pro Ala
450 455 460

Pro Ile Glu Lys Thr Ile Ser Lys Ala Lys Gly Gln Pro Arg Glu Pro
465 470 475 480

Gln Val Tyr Thr Leu Pro Pro Ser Arg Lys Glu Met Thr Lys Asn Gln
485 490 495

Val Ser Leu Thr Cys Leu Val Lys Gly Phe Tyr Pro Ser Asp Ile Ala
500 505 510

Val Glu Trp Glu Ser Asn Gly Gln Pro Glu Asn Asn Tyr Lys Thr Thr
515 520 525

Pro Pro Val Leu Lys Ser Asp Gly Ser Phe Phe Leu Tyr Ser Lys Leu
530 535 540

Thr Val Asp Lys Ser Arg Trp Gln Gln Gly Asn Val Phe Ser Cys Ser
545 550 555 560

Val Met His Glu Ala Leu His Asn His Tyr Thr Gln Lys Ser Leu Ser
565 570 575

Leu Ser Pro Gly Lys Ala Ala Ala His His His His His His
580 585 590

<210> 62
<211> 707
<212> PRT
<213> Artificial Sequence

<220>
<221> source
<223> /note="Description of Artificial Sequence: Synthetic

polypeptide"

<400> 62

Met Gly Ser Thr Ala Ile Leu Gly Leu Leu Leu Ala Val Leu Gln Gly
1               5                   10                  15

Gly Arg Ala Glu Val Gln Leu Leu Glu Gln Ser Gly Ala Glu Leu Val
            20                  25                  30

Arg Pro Gly Ala Leu Val Lys Leu Ser Cys Lys Ala Ser Gly Phe Lys
        35                  40                  45

Ile Lys Asp Tyr Phe Val Asn Trp Val Lys Gln Arg Pro Glu Gln Gly
    50                  55                  60

Leu Glu Trp Ile Gly Trp Ile Asp Pro Glu Asn Asp Asn Ser Leu Tyr
65                  70                  75                  80

Gly Pro Asn Phe Gln Asp Lys Ala Ser Ile Thr Ala Asp Thr Ser Ser
                85                  90                  95

Asn Thr Gly Tyr Leu Gln Leu Ser Gly Leu Thr Ser Glu Asp Thr Ala
            100                 105                 110

Val Tyr Tyr Cys Ala Leu Tyr Tyr Gly Ser Arg Gly Asp Ala Met Asp
        115                 120                 125

Tyr Trp Gly Gln Gly Thr Thr Val Thr Val Ser Ser Ala Ser Thr Lys
    130                 135                 140

Gly Pro Ser Val Phe Pro Leu Ala Pro Ser Ser Lys Ser Thr Ser Gly
145                 150                 155                 160

Gly Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Ser Leu Gly Thr Gln Thr Tyr Ile Cys Asn
    210                 215                 220

Val Asn His Lys Pro Ser Asn Thr Lys Val Asp Lys Arg Val Glu Pro
225                 230                 235                 240

Lys Ser Cys Asp Lys Thr His Thr Cys Pro Pro Cys Pro Ala Pro Glu
245 250 255

Ala Ala Gly Gly Pro Ser Val Phe Leu Phe Pro Pro Lys Pro Lys Asp
260 265 270

Thr Leu Met Ile Ser Arg Thr Pro Glu Val Thr Cys Val Val Val Asp
275 280 285

Val Ser His Glu Asp Pro Glu Val Lys Phe Asn Trp Tyr Val Asp Gly
290 295 300

Val Glu Val His Asn Ala Lys Thr Lys Pro Arg Glu Glu Gln Tyr Asn
305 310 315 320

Ser Thr Tyr Arg Val Val Ser Val Leu Thr Val Leu His Gln Asp Trp
325 330 335

Leu Asn Gly Lys Glu Tyr Lys Cys Lys Val Ser Asn Lys Ala Leu Pro
340 345 350

Ala Pro Ile Glu Lys Thr Ile Ser Lys Ala Lys Gly Gln Pro Arg Glu
355 360 365

Pro Gln Val Tyr Thr Leu Pro Pro Ser Arg Lys Glu Met Thr Lys Asn
370 375 380

Gln Val Ser Leu Thr Cys Leu Val Lys Gly Phe Tyr Pro Ser Asp Ile
385 390 395 400

Ala Val Glu Trp Glu Ser Asn Gly Gln Pro Glu Asn Asn Tyr Lys Thr
405 410 415

Thr Pro Pro Val Leu Lys Ser Asp Gly Ser Phe Phe Leu Tyr Ser Lys
420 425 430

Leu Thr Val Asp Lys Ser Arg Trp Gln Gln Gly Asn Val Phe Ser Cys
435 440 445

Ser Val Met His Glu Ala Leu His Asn His Tyr Thr Gln Lys Ser Leu
450 455 460

Ser Leu Ser Pro Gly Lys Ala Ala Ala Arg Arg Arg Arg Arg Arg Glu
465 470 475 480

Val Gln Leu Val Glu Ser Gly Gly Gly Leu Val Gln Pro Gly Gly Ser
485 490 495

```
Leu Lys Leu Ser Cys Ala Ala Ser Gly Phe Thr Phe Asn Ser Tyr Ala
            500                 505                 510

Met Asn Trp Val Arg Gln Ala Pro Gly Lys Gly Leu Glu Trp Val Ala
        515                 520                 525

Arg Ile Arg Ser Lys Tyr Asn Asn Tyr Ala Thr Tyr Tyr Ala Asp Ser
    530                 535                 540

Val Lys Gly Arg Phe Thr Ile Ser Arg Asp Asp Ser Lys Asn Thr Ala
545                 550                 555                 560

Tyr Leu Gln Met Asn Asn Leu Lys Thr Glu Asp Thr Ala Val Tyr Tyr
                565                 570                 575

Cys Val Arg His Gly Asn Phe Gly Asn Ser Tyr Val Ser Trp Trp Ala
            580                 585                 590

Tyr Trp Gly Gln Gly Thr Leu Val Thr Val Ser Ser Ala Ser Thr Lys
        595                 600                 605

Gly Pro Ser Val Phe Pro Leu Ala Pro Ser Ser Lys Ser Thr Ser Gly
    610                 615                 620

Gly Thr Ala Ala Leu Gly Cys Leu Val Lys Asp Tyr Phe Pro Glu Pro
625                 630                 635                 640

Val Thr Val Ser Trp Asn Ser Gly Ala Leu Thr Ser Gly Val His Thr
                645                 650                 655

Phe Pro Ala Val Leu Gln Ser Ser Gly Leu Tyr Ser Leu Ser Ser Val
            660                 665                 670

Val Thr Val Pro Ser Ser Ser Leu Gly Thr Gln Thr Tyr Ile Cys Asn
        675                 680                 685

Val Asn His Lys Pro Ser Asn Thr Lys Val Asp Lys Arg Val Glu Pro
    690                 695                 700

Lys Ser Cys
705
```

<210> 63
<211> 2121
<212> DNA
<213> Artificial Sequence

<220>

<221> source
<223> /note="Description of Artificial Sequence: Synthetic polynucleotide"

<400> 63
atggggtcaa ccgccatcct tggcctcctc ctggctgtcc tgcagggagg gcgcgccgag 60

gtgcagctgc tcgagcagtc tggagctgag cttgtgaggc caggggcctt agtcaagttg 120

tcctgcaaag cttctggctt caaaattaaa gactactttg tgaactgggt gaagcagagg 180

cctgaacagg gcctggagtg gattggatgg attgatcctg agaatgataa tagtttatat 240

ggcccgaact tccaggacaa ggccagtatc acagcagaca catcctccaa cacaggctac 300

ctgcagctca gcggcctgac atctgaggac actgccgtct attactgtgc tctttattac 360

ggaagtaggg gggatgctat ggactactgg ggccaaggga ccacggtcac cgtctcctca 420

gctagcacca agggcccatc cgtcttcccc ctggcaccct cctccaagag cacctctggg 480

ggcacagcgg ccctgggctg cctggtcaag gactacttcc ccgaaccggt gacggtgtcg 540

tggaactcag gcgccctgac cagcggcgtg cacaccttcc cggctgtcct acagtcctca 600

ggactctact ccctcagcag cgtggtgacc gtgccctcca gcagcttggg cacccagacc 660

tacatctgca acgtgaatca caagcccagc aacaccaagg tggacaagag agttgagccc 720

aaatcttgtg acaaaactca cacatgccca ccgtgcccag cacctgaagc agctggggga 780

ccgtcagtct tcctcttccc cccaaaaccc aaggacaccc tcatgatctc ccggacccct 840

gaggtcacat gcgtggtggt ggacgtgagc cacgaagacc ctgaggtcaa gttcaactgg 900

tacgtggacg gcgtggaggt gcataatgcc aagacaaagc cgcgggagga gcagtacaac 960

agcacgtacc gtgtggtcag cgtcctcacc gtcctgcacc aggactggct gaatggcaag 1020

gagtacaagt gcaaggtctc caacaaagcc ctcccagccc ccatcgagaa aaccatctcc 1080

aaagccaaag ggcagccccg agaaccacag gtgtacaccc tgcccccatc ccggaaggag 1140

atgaccaaga accaggtcag cctgacctgc ctggtcaaag gcttctatcc cagcgacatc 1200

gccgtggagt gggagagcaa tgggcagccg gagaacaact acaagaccac gcctcccgtg 1260

ctgaagtccg acggctcctt cttcctctat agcaagctca ccgtggacaa gagcaggtgg 1320

cagcagggga acgtcttctc atgctccgtg atgcatgagg ctctgcacaa ccactacacg 1380

cagaagagcc tctccctgtc tccgggtaaa gcggccgctc ggcgaagacg tcgcagggag 1440

gtgcagctgg tcgagtctgg aggaggattg gtgcagcctg gagggtcatt gaaactctca 1500

tgtgcagcct ctggattcac cttcaatagc tacgccatga actgggtccg ccaggctcca 1560

ggaaagggtt tggaatgggt tgctcgcata agaagtaaat ataataatta tgcaacatat 1620

tatgccgatt cagtgaaagg caggttcacc atctccagag atgattcaaa aaacactgcc 1680

tatctacaaa tgaacaactt gaaaactgag gacactgccg tgtactactg tgtgagacat 1740

gggaacttcg gtaatagcta cgtttcctgg tgggcttact ggggccaagg gactctggtc 1800

accgtctcct cagctagcac caagggccca tccgtcttcc ccctggcacc ctcctccaag 1860

agcacctctg ggggcacagc ggccctgggc tgcctggtca aggactactt ccccgaaccg 1920

gtgacggtgt cgtggaactc aggcgccctg accagcggcg tgcacacctt cccggctgtc 1980

ctacagtcct caggactcta ctccctcagc agcgtggtga ccgtgccctc cagcagcttg 2040

ggcacccaga cctacatctg caacgtgaat cacaagccca gcaacaccaa ggtggacaag 2100

agagttgagc ccaaatcttg t 2121

<210> 64
<211> 709
<212> PRT
<213> Artificial Sequence

<220>
<221> source
<223> /note="Description of Artificial Sequence: Synthetic
polypeptide"

<400> 64
Met Gly Ser Thr Ala Ile Leu Gly Leu Leu Leu Ala Val Leu Gln Gly
1 5 10 15

Gly Arg Ala Glu Val Gln Leu Leu Glu Gln Ser Gly Ala Glu Leu Val
20 25 30

Arg Pro Gly Ala Leu Val Lys Leu Ser Cys Lys Ala Ser Gly Phe Lys
35 40 45

Ile Lys Asp Tyr Phe Val Asn Trp Val Lys Gln Arg Pro Glu Gln Gly
50 55 60

Leu Glu Trp Ile Gly Trp Ile Asp Pro Glu Asn Asp Asn Ser Leu Tyr
65 70 75 80

Gly Pro Asn Phe Gln Asp Lys Ala Ser Ile Thr Ala Asp Thr Ser Ser
85 90 95

Asn Thr Gly Tyr Leu Gln Leu Ser Gly Leu Thr Ser Glu Asp Thr Ala
100 105 110

Val Tyr Tyr Cys Ala Leu Tyr Tyr Gly Ser Arg Gly Asp Ala Met Asp
115 120 125

Tyr Trp Gly Gln Gly Thr Thr Val Thr Val Ser Ser Ala Ser Thr Lys
130 135 140

Gly Pro Ser Val Phe Pro Leu Ala Pro Ser Ser Lys Ser Thr Ser Gly

145 150 155 160

Gly Thr Ala Ala Leu Gly Cys Leu Val Lys Asp Tyr Phe Pro Glu Pro
165 170 175

Val Thr Val Ser Trp Asn Ser Gly Ala Leu Thr Ser Gly Val His Thr
180 185 190

Phe Pro Ala Val Leu Gln Ser Ser Gly Leu Tyr Ser Leu Ser Ser Val
195 200 205

Val Thr Val Pro Ser Ser Ser Leu Gly Thr Gln Thr Tyr Ile Cys Asn
210 215 220

Val Asn His Lys Pro Ser Asn Thr Lys Val Asp Lys Arg Val Glu Pro
225 230 235 240

Lys Ser Cys Asp Lys Thr His Thr Cys Pro Pro Cys Pro Ala Pro Glu
245 250 255

Ala Ala Gly Gly Pro Ser Val Phe Leu Phe Pro Pro Lys Pro Lys Asp
260 265 270

Thr Leu Met Ile Ser Arg Thr Pro Glu Val Thr Cys Val Val Val Asp
275 280 285

Val Ser His Glu Asp Pro Glu Val Lys Phe Asn Trp Tyr Val Asp Gly
290 295 300

Val Glu Val His Asn Ala Lys Thr Lys Pro Arg Glu Glu Gln Tyr Asn
305 310 315 320

Ser Thr Tyr Arg Val Val Ser Val Leu Thr Val Leu His Gln Asp Trp
325 330 335

Leu Asn Gly Lys Glu Tyr Lys Cys Lys Val Ser Asn Lys Ala Leu Pro
340 345 350

Ala Pro Ile Glu Lys Thr Ile Ser Lys Ala Lys Gly Gln Pro Arg Glu
355 360 365

Pro Gln Val Tyr Thr Leu Pro Pro Ser Arg Lys Glu Met Thr Lys Asn
370 375 380

Gln Val Ser Leu Thr Cys Leu Val Lys Gly Phe Tyr Pro Ser Asp Ile
385 390 395 400

Ala Val Glu Trp Glu Ser Asn Gly Gln Pro Glu Asn Asn Tyr Lys Thr
405 410 415

Thr Pro Pro Val Leu Lys Ser Asp Gly Ser Phe Phe Leu Tyr Ser Lys
420 425 430

Leu Thr Val Asp Lys Ser Arg Trp Gln Gln Gly Asn Val Phe Ser Cys
435 440 445

Ser Val Met His Glu Ala Leu His Asn His Tyr Thr Gln Lys Ser Leu
450 455 460

Ser Leu Ser Pro Gly Lys Ala Ala Ala Gly Pro Leu Gly Ile Ala Gly
465 470 475 480

Gln Glu Val Gln Leu Val Glu Ser Gly Gly Gly Leu Val Gln Pro Gly
485 490 495

Gly Ser Leu Lys Leu Ser Cys Ala Ala Ser Gly Phe Thr Phe Asn Ser
500 505 510

Tyr Ala Met Asn Trp Val Arg Gln Ala Pro Gly Lys Gly Leu Glu Trp
515 520 525

Val Ala Arg Ile Arg Ser Lys Tyr Asn Asn Tyr Ala Thr Tyr Tyr Ala
530 535 540

Asp Ser Val Lys Gly Arg Phe Thr Ile Ser Arg Asp Asp Ser Lys Asn
545 550 555 560

Thr Ala Tyr Leu Gln Met Asn Asn Leu Lys Thr Glu Asp Thr Ala Val
565 570 575

Tyr Tyr Cys Val Arg His Gly Asn Phe Gly Asn Ser Tyr Val Ser Trp
580 585 590

Trp Ala Tyr Trp Gly Gln Gly Thr Leu Val Thr Val Ser Ser Ala Ser
595 600 605

Thr Lys Gly Pro Ser Val Phe Pro Leu Ala Pro Ser Ser Lys Ser Thr
610 615 620

Ser Gly Gly Thr Ala Ala Leu Gly Cys Leu Val Lys Asp Tyr Phe Pro
625 630 635 640

Glu Pro Val Thr Val Ser Trp Asn Ser Gly Ala Leu Thr Ser Gly Val
645 650 655

His Thr Phe Pro Ala Val Leu Gln Ser Ser Gly Leu Tyr Ser Leu Ser
660 665 670

Ser Val Val Thr Val Pro Ser Ser Ser Leu Gly Thr Gln Thr Tyr Ile
675 680 685

Cys Asn Val Asn His Lys Pro Ser Asn Thr Lys Val Asp Lys Arg Val
690 695 700

Glu Pro Lys Ser Cys
705

<210> 65
<211> 2127
<212> DNA
<213> Artificial Sequence

<220>
<221> source
<223> /note="Description of Artificial Sequence: Synthetic polynucleotide"

<400> 65
atggggtcaa ccgccatcct tggcctcctc ctggctgtcc tgcagggagg gcgcgccgag 60

gtgcagctgc tcgagcagtc tggagctgag cttgtgaggc caggggcctt agtcaagttg 120

tcctgcaaag cttctggctt caaaattaaa gactactttg tgaactgggt gaagcagagg 180

cctgaacagg gcctggagtg gattggatgg attgatcctg agaatgataa tagtttatat 240

ggcccgaact tccaggacaa ggccagtatc acagcagaca catcctccaa cacaggctac 300

ctgcagctca gcggcctgac atctgaggac actgccgtct attactgtgc tctttattac 360

ggaagtaggg gggatgctat ggactactgg ggccaaggga ccacggtcac cgtctcctca 420

gctagcacca agggcccatc cgtcttcccc ctggcaccct cctccaagag cacctctggg 480

ggcacagcgg ccctgggctg cctggtcaag gactacttcc ccgaaccggt gacggtgtcg 540

tggaactcag gcgccctgac cagcggcgtg cacaccttcc cggctgtcct acagtcctca 600

ggactctact ccctcagcag cgtggtgacc gtgccctcca gcagcttggg cacccagacc 660

tacatctgca acgtgaatca caagcccagc aacaccaagg tggacaagag agttgagccc 720

aaatcttgtg acaaaactca cacatgccca ccgtgcccag cacctgaagc agctggggga 780

ccgtcagtct tcctcttccc cccaaaaccc aaggacaccc tcatgatctc ccggacccct 840

gaggtcacat gcgtggtggt ggacgtgagc cacgaagacc ctgaggtcaa gttcaactgg 900

tacgtggacg gcgtggaggt gcataatgcc aagacaaagc cgcgggagga gcagtacaac 960

agcacgtacc gtgtggtcag cgtcctcacc gtcctgcacc aggactggct gaatggcaag 1020

gagtacaagt gcaaggtctc caacaaagcc ctcccagccc ccatcgagaa aaccatctcc 1080

aaagccaaag ggcagccccg agaaccacag gtgtacaccc tgcccccatc ccggaaggag 1140

atgaccaaga accaggtcag cctgacctgc ctggtcaaag gcttctatcc cagcgacatc 1200

gccgtggagt gggagagcaa tgggcagccg gagaacaact acaagaccac gcctcccgtg 1260

ctgaagtccg acggctcctt cttcctctat agcaagctca ccgtggacaa gagcaggtgg 1320

cagcagggga acgtcttctc atgctccgtg atgcatgagg ctctgcacaa ccactacacg 1380

cagaagagcc tctccctgtc tccgggtaaa gcggccgctg gaccgttggg tatcgctggc 1440

caggaggtgc agctggtcga gtctggagga ggattggtgc agcctggagg gtcattgaaa 1500

ctctcatgtg cagcctctgg attcaccttc aatagctacg ccatgaactg ggtccgccag 1560

gctccaggaa agggtttgga atgggttgct cgcataagaa gtaaatataa taattatgca 1620

acatattatg ccgattcagt gaaaggcagg ttcaccatct ccagagatga ttcaaaaaac 1680

actgcctatc tacaaatgaa caacttgaaa actgaggaca ctgccgtgta ctactgtgtg 1740

agacatggga acttcggtaa tagctacgtt tcctggtggg cttactgggg ccaagggact 1800

ctggtcaccg tctcctcagc tagcaccaag ggcccatccg tcttccccct ggcaccctcc 1860

tccaagagca cctctggggg cacagcggcc ctgggctgcc tggtcaagga ctacttcccc 1920

gaaccggtga cggtgtcgtg gaactcaggc gccctgacca gcggcgtgca caccttcccg 1980

gctgtcctac agtcctcagg actctactcc ctcagcagcg tggtgaccgt gccctccagc 2040

agcttgggca cccagaccta catctgcaac gtgaatcaca agcccagcaa caccaaggtg 2100

gacaagagag ttgagcccaa atcttgt 2127

<210> 66
<211> 8
<212> PRT
<213> Artificial Sequence

<220>
<221> source
<223> /note="Description of Artificial Sequence: Synthetic peptide"

<400> 66
Gly Pro Leu Gly Ile Ala Gly Gln
1               5

<210> 67
<211> 125
<212> PRT
<213> Artificial Sequence

<220>
<221> source
<223> /note="Description of Artificial Sequence: Synthetic polypeptide"

<400> 67
Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Thr Phe Asn Lys Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Lys Asn Thr
65                  70                  75                  80

Ala Tyr Leu Gln Met Asn Asn Leu Lys Thr Glu Asp Thr Ala Val Tyr
                85                  90                  95

Tyr Cys Val Arg His Gly Asn Phe Gly Asn Ser Tyr Ile Ser Tyr Trp
            100                 105                 110

Ala Tyr Trp Gly Gln Gly Thr Leu Val Thr Val Ser Ser
        115                 120                 125

<210> 68
<211> 375
<212> DNA
<213> Artificial Sequence

<220>
<221> source
<223> /note="Description of Artificial Sequence: Synthetic
      polynucleotide"

<400> 68
gaggtgcagc tggtcgagtc tggaggagga ttggtgcagc ctggagggtc attgaaactc 60

tcatgtgcag cctctggatt caccttcaat aagtacgcca tgaactgggt ccgccaggct 120

ccaggaaagg gtttggaatg ggttgctcgc ataagaagta aatataataa ttatgcaaca 180

tattatgccg attcagtgaa agacaggttc accatctcca gagatgattc aaaaaacact 240

gcctatctac aaatgaacaa cttgaaaact gaggacactg ccgtgtacta ctgtgtgaga 300

catgggaact tcggtaatag ctacatatcc tactgggctt actggggcca agggactctg 360

gtcaccgtct cctca 375

<210> 69
<211> 109
<212> PRT
<213> Artificial Sequence

<220>
<221> source
<223> /note="Description of Artificial Sequence: Synthetic
polypeptide"

<400> 69
Gln Thr Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Ser Gly
            20                  25                  30

Asn Tyr Pro Asn Trp Val Gln Gln Lys Pro Gly Gln Ala Pro Arg Gly
        35                  40                  45

Leu Ile Gly Gly Thr Lys Phe Leu Ala Pro Gly Thr Pro Ala Arg Phe
    50                  55                  60

Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Val Leu Trp Tyr Ser Asn
                85                  90                  95

Arg Trp Val Phe Gly Gly Gly Thr Lys Leu Thr Val Leu
            100                 105

<210> 70
<211> 327
<212> DNA
<213> Artificial Sequence

<220>
<221> source
<223> /note="Description of Artificial Sequence: Synthetic
polynucleotide"

<400> 70
cagactgttg tgactcagga accttcactc accgtatcac ctggtggaac agtcacactc 60

acttgtggct cctcgactgg ggctgttaca tctggcaact acccaaactg ggtccaacaa 120

aaaccaggtc aggcaccccg tggtctaata ggtgggacta agttcctcgc ccccggtact 180

cctgccagat tctcaggctc cctgcttgga ggcaaggctg ccctcaccct ctcaggggta 240

cagccagagg atgaggcaga atattactgt gttctatggt acagcaaccg ctgggtgttc 300

ggtggaggaa ccaaactgac tgtccta 327

<210> 71
<211> 8
<212> PRT
<213> Artificial Sequence

<220>
<221> source
<223> /note="Description of Artificial Sequence: Synthetic
peptide"

<400> 71
Ala Pro Met Ala Glu Gly Gly Gly
1               5

<210> 72
<211> 8
<212> PRT
<213> Artificial Sequence

<220>
<221> source
<223> /note="Description of Artificial Sequence: Synthetic
peptide"

<400> 72
Glu Ala Gln Gly Asp Lys Ile Ile
1               5

<210> 73
<211> 8
<212> PRT
<213> Artificial Sequence

<220>
<221> source
<223> /note="Description of Artificial Sequence: Synthetic
peptide"

<400> 73
Leu Ala Phe Ser Asp Ala Gly Pro
1               5

<210> 74
<211> 7
<212> PRT
<213> Artificial Sequence

<220>
<221> source
<223> /note="Description of Artificial Sequence: Synthetic
peptide"

<400> 74
Tyr Val Ala Asp Ala Pro Lys
1               5

<210> 75
<211> 5
<212> PRT
<213> Artificial Sequence

<220>
<221> source

<223> /note="Description of Artificial Sequence: Synthetic peptide"

<400> 75
Ser Gly Arg Ser Ala
1               5

<210> 76
<211> 6
<212> PRT
<213> Artificial Sequence

<220>
<221> source
<223> /note="Description of Artificial Sequence: Synthetic peptide"

<400> 76
Gly Ser Gly Arg Ser Ala
1               5

<210> 77
<211> 5
<212> PRT
<213> Artificial Sequence

<220>
<221> source
<223> /note="Description of Artificial Sequence: Synthetic peptide"

<400> 77
Ser Gly Lys Ser Ala
1               5

<210> 78
<211> 5
<212> PRT
<213> Artificial Sequence

<220>
<221> source
<223> /note="Description of Artificial Sequence: Synthetic peptide"

<400> 78
Ser Gly Arg Ser Ser
1               5

<210> 79
<211> 5
<212> PRT
<213> Artificial Sequence

<220>
<221> source
<223> /note="Description of Artificial Sequence: Synthetic peptide"

```
<400> 79
Ser Gly Arg Arg Ala
1               5


<210> 80
<211> 5
<212> PRT
<213> Artificial Sequence

<220>
<221> source
<223> /note="Description of Artificial Sequence: Synthetic
      peptide"

<400> 80
Ser Gly Arg Asn Ala
1               5


<210> 81
<211> 5
<212> PRT
<213> Artificial Sequence

<220>
<221> source
<223> /note="Description of Artificial Sequence: Synthetic
      peptide"

<400> 81
Ser Gly Arg Lys Ala
1               5


<210> 82
<211> 6
<212> PRT
<213> Artificial Sequence

<220>
<221> source
<223> /note="Description of Artificial Sequence: Synthetic
      peptide"

<400> 82
Gln Arg Gly Arg Ser Ala
1               5


<210> 83
<211> 6
<212> PRT
<213> Artificial Sequence

<220>
<221> source
<223> /note="Description of Artificial Sequence: Synthetic
      peptide"

<400> 83
Thr Gln Gly Ala Ala Ala
1               5
```

<210> 84
<211> 6
<212> PRT
<213> Artificial Sequence

<220>
<221> source
<223> /note="Description of Artificial Sequence: Synthetic peptide"

<400> 84
Gly Ala Ala Ala Ala Ala
1               5

<210> 85
<211> 6
<212> PRT
<213> Artificial Sequence

<220>
<221> source
<223> /note="Description of Artificial Sequence: Synthetic peptide"

<400> 85
Gly Ala Gly Ala Ala Gly
1               5

<210> 86
<211> 6
<212> PRT
<213> Artificial Sequence

<220>
<221> source
<223> /note="Description of Artificial Sequence: Synthetic peptide"

<400> 86
Ala Ala Ala Ala Ala Gly
1               5

<210> 87
<211> 6
<212> PRT
<213> Artificial Sequence

<220>
<221> source
<223> /note="Description of Artificial Sequence: Synthetic peptide"

<400> 87
Leu Cys Gly Ala Ala Ile
1               5

<210> 88

<211> 6
<212> PRT
<213> Artificial Sequence

<220>
<221> source
<223> /note="Description of Artificial Sequence: Synthetic peptide"

<400> 88
Phe Ala Gln Ala Leu Gly
1               5

<210> 89
<211> 6
<212> PRT
<213> Artificial Sequence

<220>
<221> source
<223> /note="Description of Artificial Sequence: Synthetic peptide"

<400> 89
Leu Leu Gln Ala Asn Pro
1               5

<210> 90
<211> 6
<212> PRT
<213> Artificial Sequence

<220>
<221> source
<223> /note="Description of Artificial Sequence: Synthetic peptide"

<400> 90
Leu Ala Ala Ala Asn Pro
1               5

<210> 91
<211> 6
<212> PRT
<213> Artificial Sequence

<220>
<221> source
<223> /note="Description of Artificial Sequence: Synthetic peptide"

<400> 91
Leu Tyr Gly Ala Gln Phe
1               5

<210> 92
<211> 6
<212> PRT
<213> Artificial Sequence

<220>
<221> source
<223> /note="Description of Artificial Sequence: Synthetic peptide"

<400> 92
Leu Ser Gln Ala Gln Gly
1 5

<210> 93
<211> 6
<212> PRT
<213> Artificial Sequence

<220>
<221> source
<223> /note="Description of Artificial Sequence: Synthetic peptide"

<400> 93
Ala Ser Ala Ala Ser Gly
1 5

<210> 94
<211> 6
<212> PRT
<213> Artificial Sequence

<220>
<221> source
<223> /note="Description of Artificial Sequence: Synthetic peptide"

<400> 94
Phe Leu Gly Ala Ser Leu
1 5

<210> 95
<211> 6
<212> PRT
<213> Artificial Sequence

<220>
<221> source
<223> /note="Description of Artificial Sequence: Synthetic peptide"

<400> 95
Ala Tyr Gly Ala Thr Gly
1 5

<210> 96
<211> 6
<212> PRT
<213> Artificial Sequence

<220>
<221> source

<223> /note="Description of Artificial Sequence: Synthetic peptide"

<400> 96
Leu Ala Gln Ala Thr Gly
1 5

<210> 97
<211> 5
<212> PRT
<213> Artificial Sequence

<220>
<221> source
<223> /note="Description of Artificial Sequence: Synthetic peptide"

<400> 97
Arg Arg Arg Arg Arg
1 5

<210> 98
<211> 10
<212> PRT
<213> Artificial Sequence

<220>
<221> source
<223> /note="Description of Artificial Sequence: Synthetic peptide"

<400> 98
Gly Gln Ser Ser Arg His Arg Arg Ala Leu
1 5 10

<210> 99
<211> 10
<212> PRT
<213> Artificial Sequence

<220>
<221> source
<223> /note="Description of Artificial Sequence: Synthetic peptide"

<400> 99
Gly Gly Pro Leu Gly Met Leu Ser Gln Ser
1 5 10

<210> 100
<211> 6
<212> PRT
<213> Artificial Sequence

<220>
<221> source
<223> /note="Description of Artificial Sequence: Synthetic peptide"

```
<400> 100
Pro Leu Gly Leu Ala Gly
1               5


<210> 101
<211> 6
<212> PRT
<213> Artificial Sequence

<220>
<221> source
<223> /note="Description of Artificial Sequence: Synthetic
      peptide"

<400> 101
Leu Ala Ala Ala Asn Pro
1               5
```

## Description

## CROSS-REFERENCE TO RELATED APPLICATIONS

This application claims the benefit of US Provisional Application 61/882,428, filed September 25, 2013, which is incorporated herein in its entirety.

## FIELD

This application is in the field of antibody engineering.

## BACKGROUND

Bispecific antibodies have promise as therapeutics in a variety of indications. Bispecific antibodies having a standard IgG format can be challenging to produce because they include four different polypeptide chains. The efficacy of a smaller, more easily-produced bispecific molecule has been clinically demonstrated in non-Hodgkin's lymphoma. Bargou et al. (2008), Science 321(5891): 974-977. Daily administration was used to achieve these results, presumably because of the short *in vivo* half life of this small, single chain molecule. *Id.* Hence, there is a need in the art for bispecific therapeutics with favorable pharmacokinetic properties, as well as therapeutic efficacy and a format that makes them straightforward to produce.

## SUMMARY

Herein is described an antibody comprising two polypeptide chains: the first polypeptide chain comprising the following regions, which may or may not be separated linkers, in the following order in an N- to C-terminal direction: V-1---C-1---hinge---CH2-1---CH3-1---V-2---C-2; and the second polypeptide chain comprising the following regions, which may or may not be separated by linkers, in the following order in an N- to C-terminal direction: V-3---C-3---hinge---CH2-2---CH3-2---V-4---C-4; wherein the V-1 to V-4 are immunoglobulin variable regions; wherein the C-1 to C-4 are immunoglobulin constant regions, and the C-1 and C-3 and also the C-2 and C-4 form pairs of a heavy chain constant region 1 (CH1) and a light chain constant region (CL) and/or if C-1 is a CH1, then C-3 is a CL and vice versa, and if C-2 is a CH1, then C-4 is a CL and vice versa; and wherein the CH2-1 and CH2-2 are each a heavy chain constant region 2 and the CH3-1 and CH3-2 are each a heavy chain constant region 3. *See* Figure 1. In one aspect, the V-1 can be a heavy chain variable region (VH), the C-1 can be a CH1, the V-2 can be a VH, the C-2 can be a CH1, the V-3 can be a light chain variable region (VL), the C-3 can be a light chain constant region (CL), the V-4 can be a VL, and the C-4 can be a CL. In another aspect, the V-1 can be a VH, the C-1 can be a CH1, the V-2 can be a VL, the C-2 can be a CL, the V-3 can be a VL, the C-3 can be a CL, the V-4 can be a VH, and the C-4 can be a CH1. In a further embodiment, the V-1 can be a VH, the C-1 can be a CH1, the V-2 can be a VH, the C-2 can be a CL, the V-3 can be a VL, the C-3 can be a CL, the V-4 can be a VL, and the C-4 can be a CH1. In another embodiment, the V-1 can be a VH, the C-1 can be a CL, the V-2 can be a VH, the C-2 can be a CH1, the V-3 can be a VL, the C-3 can be a CH1, the V-4 can be a VL, and the C-4 can be a CL. In another embodiment, the V-1 can be a VL, the C-1 can be a CL, the V-2 can be a VL, the C-2 can be a CL, the V-3 can be a VH, the C-3 can be a CH1, the V-4 can be a VH, and the C-4 can be a CH1. In still another aspect, the V-1 can be a VL, the C-1 can be a CL, the V-2 can be a VH, the C-2 can be a CH1, the V-3 can be a VH, the C-3 can be a CH1, the V-4 can be a VL, and the C-4 can be a CL. In another embodiment, the V-1 can be a VL, the C-1 can be a CH1, the V-2 can be a VH, the C-2 can be a CH1, the V-3 can be a VH, the C-3 can be a CL, the V-4 can be a VL, and the C-4 can be a CL. In still another embodiment, the V-1 can be a VL, the C-1 can be a CH1, the V-2 can be a VL, the C-2 can be a CH1, the V-3 can be a VH, the C-3 can be a CL, the V-4 can be a VH, and the C-4 can be a CL. In a further embodiment, the V-1 can be a VH, the C-1 can be a CH1, the V-2 can be a VL, the C-2 can be a CH1, the V-3 can be a VL, the C-3 can be a CL, the V-4 can be a VH, and the C-4 can be a CL. In one further aspect, the V-1 can be a VL, the C-1 can be a CH1, the V-2 can be a VH, the C-2 can be a CL, the V-3 can be a VH, the C-3 can be a CL, the V-4 can be a VL, and the C-4 can be a CH1.

In some embodiments of the V-C-Fc-V-C antibodies described above and below, the first polypeptide chain can comprise a linker between the CH3-1 and the V-2 and the second polypeptide chain can comprise a linker between the CH3-2 and the V-4. In some embodiments, either the linker between CH3-1 and V-2 or the linker between CH3-2 and V-4 comprises a cleavage site for a protease, which can be a furin, a matrix metalloproteinase, or a protease expressed by a cancer cell. The linkers between the CH3-1 and the V-2 and between the CH3-2 and the V-4 each can be, for example, from 5 to 35 or from 5 to 30 amino acids long.

In the V-C-Fc-V-C antibodies described above and below, the CH3-1 and the CH3-2 can have different amino acid sequences and can comprise charge pair substitutions. The CH3-1 can, for example, comprise the charge pair substitutions K409D or K409E and K392D or K392E, and the CH3-2 can, for example, comprise the charge pair substitutions D399K or D399R and D356K or D356R. Alternatively, the CH3-2 can, for example, comprise the charge pair substitutions K409D or K409E and K392D or K392E, and the CH3-1 can, for example, comprise the charge pair substitutions D399K or D399R and D356K or D356R.

In some embodiments of the V-C-Fc-V-C-antibodies described above and below, the V-1 and the V-3 can bind to an immune effector cell when they are part of an IgG and/or scFv antibody, and the V-2 and the V-4 can bind to a target cell when they are part of an IgG and/or scFv antibody. In other embodiments, the V-2 and the V-4 can bind to an immune effector cell when they are part of an IgG and/or scFv antibody, and the V-1 and the V-3 can bind to a target cell when they are part of an IgG and/or scFv antibody. The immune effector cell can, for example, be a T cell, and the effector cell molecule can be human CD3 epsilon chain (CD3ε). In some embodiments, the target cell can be a cancer cell.

In some embodiments of the V-C-Fc-V-C antibody described above and below, the C-1 to C4, the hinge regions, and the CH2-1, CH2-2, CH3-1, and CH3-2 can be human IgG constant regions or human lambda or kappa constant regions, which human sequences comprise not more than 15 insertions, deletions, or substitutions of a single amino acid per 100 amino acids relative to a naturally-occurring human amino acid sequence. The human IgG constant regions can be human IgG1, IgG2, or IgG4 constant regions. In another aspect, the first and second polypeptide chains can comprise one or more alterations that reduce Fc gamma receptor (Fc γ R) binding, such as L234A, L235A, and/or any substitution at N297. In a further aspect, the first and second polypeptide chains can comprise one or more alterations that enhance(s) ADCC, such as an insertion of the amino acid sequence of any one of SEQ ID NOs:13-24 between positions 384 and 385 according to the EU numbering system as shown in Table 2 in CH3-1 and/or CH3-2.

In a further embodiment, herein is described a V-C-Fc-V-C antibody comprising a first polypeptide chain having the formula V-1---C-1---hinge---CH2-1---CH3-1---L-1---V-2---C-2 and a second polypeptide chain having the formula V-3---C-3---hinge---CH2-2---CH3-2---L-2---V-4---C-4; wherein the V-1, V-2, V-3, and V-4 are immunoglobulin variable regions having different amino acid sequences; wherein either the V-1 or the V-3 is a VH, and if the V-1 is a VH, then the V-3 is a VL, and if the V-3 is a VH, then the V-1 is a VL; wherein either the V-2 or the V-4 is a VH, and if the V-2 is a VH, then the V-4 is a VL, and if the V-4 is a VH, then the V-2 is a VL; wherein the L-1 and L-2 are linkers; wherein either the C-1 or the C-3 is a CH1, and if the C-1 is a CH1, then the C-3 is a CL, and if the C-3 is a CH1, then the C-1 is a CL; wherein either the C-2 or the C-4 is a CH1, and if the C-2 is a CH1, then the C-4 is a CL, and if the C-4 is a CH1, then the C-2 is a CL; and wherein the antibody can bind to a target cell, which can be a cancer cell, an infected cell, or a cell mediating an autoimmune or fibrotic disease, and to an immune effector cell. Alternatively, herein is described an antibody comprising a first polypeptide chain having the following formula: V-1---L-3---C-1---L-4---hinge---CH2-1---CH3-1---L-1---V-2---L-5-C-2 and a second polypeptide chain having the following formula: V-3-L6---C-3---L7---hinge---CH2-2---CH3-2--L-2---V-4---L-8---C-4, wherein the V-1, V-2, V-3, and V-4 are immunoglobulin variable regions having different amino acid sequences, wherein either the V-1 or the V-3 is a VH, and if the V-1 is a VH, then the V-3 is a VL, and if the V-3 is a VH, then the V-1 is a VL, wherein either the V-2 or the V-4 is a VH, and if the V-2 is a VH, then the V-4 is a VL, and if the V-4 is a VH, then the V-2 is a VL, wherein the L-1, L-2, L-3, L-4, L-5, L-6, L-7, and L-8 are linkers and L-3, L-4, L-5, L-6, L-7, and L-8 can be present or absent, wherein either the C-1 or the C-3 is a CH1, and if the C-1 is a CH1, then the C-3 is a CL, and if the C-3 is a CH1, then the C-1 is a CL, wherein either the C-2 or the C-4 is a CH1, and if the C-2 is a CH1, then the C-4 is a CL, and if the C-4 is a CH1, then the C-2 is a CL, and wherein the antibody binds to a cancer cell and to an immune effector cell. The first and the second polypeptide chains can each comprise a charge pair substitution. For example, the CH3-1 can comprise the charge pair substitutions K409D or K409E and K392D or K392E, and the CH3-2 can comprise the charge pair substitutions D399K or D399R and E356K or E356R. Alternatively, the CH3-2 can comprise the charge pair substitutions K409D or K409E and K392D or K392E, and the CH3-1 can comprise the charge pair substitutions D399K or D399R and D356K or D356R. Alternatively or in addition, the CH3-1 and the CH3-2 can comprise other charge pair substitutions. In another aspect, the first and second polypeptide chains can comprise one or more alterations that inhibit Fc γ R binding, such as, for example, L234A, L235A, and any substitution at N297. In some embodiments, the immune effector cell can be a T cell, and the antibody can bind to human CD3ε. In some embodiments, the target cell is a cancer cell. In such embodiments, the V-1 and the V-3 may bind to the cancer cell when they are part of an IgG and/or an scFv antibody, and the V-2 and the V-4 may bind to the immune effector cell when they are part of an IgG and/or scFv antibody. Alternatively the V-2 and the V-4 may bind to the cancer cell when they are part of an IgG and/or an scFv antibody and the V-1 and the V-3 may bind to the immune effector cell when they are part of an IgG and/or scFv antibody. In some embodiments, the L-1 can comprise a protease cleavage site while the L-2 does not, or vice versa.

In some of the embodiments of a V-C-Fc-V-C antibody described above and/or below, the antibody can comprise the amino acid sequences of SEQ ID NOs: 1 and 3, SEQ ID NOs: 5 and 7, SEQ ID NOs: 9 and 11, SEQ ID NOs: 46 and 48, SEQ ID NOs:11 and 62, SEQ ID NOs:11 and 64, or SEQ ID NOs:67 and 69.

In another aspect, herein is described a pharmaceutical composition comprising any of the V-C-Fc-V-C antibodies described above and/or below.

Also provided herein are one or more nucleic acid(s) encoding any of the V-C-Fc-V-C antibodies, or portions thereof, described above and/or below. Such nucleic acids can include the nucleotide sequences of SEQ ID NOs:2 and 4, SEQ ID NOs:6 and 8, SEQ ID NOs:10 and 12, SEQ ID NOs:47 and 49, SEQ ID NOs:12 and 63, SEQ ID NOs:12 and 65, or SEQ ID NOs:68 and 70. Also described are one or more vector(s) containing such nucleic acid(s) and a host cell containing such nucleic acids or vectors. Also described herein is a method of making an antibody comprising culturing such a host cell under conditions such that the nucleic acid(s) is(are) expressed, and recovering the antibody from the cell mass or the culture medium.

In further embodiments, herein is described a method of treatment comprising administering to a cancer patient a therapeutically effect dose of any of the V-C-Fc-V-C antibodies described above or below. The cancer can be a hematologic malignancy. Chemotherapy, a non-chemotherapeutic anti-neoplastic agent, or radiation may have been administered before, after, or concurrently with the antibody. In some embodiments, the antibody can bind to FOLR1 or HER2 and CD3ε.

In another aspect, herein is described a method of treatment comprising administering to a patient having an infectious disease any of the V-C-Fc-V-C antibodies described above and/or below. In a further aspect, herein is provided a method of treatment comprising administering to a patient having asthma, systemic lupus erythematosus, or a fibrotic disease any of the V-C-Fc-V-C antibodies described above and/or below. In still another aspect, herein is described the use of any of the V-C-Fc-V-C antibodies described above and/or below as a medicament for treating a cancer, an infectious disease, an autoimmune disease, asthma, or a fibrotic disease. Further described herein is use of any of the V-C-Fc-V-C antibodies described above and/or below in the manufacture of a medicament for treating a cancer, an infectious disease, an autoimmune disease, asthma, or a fibrotic disease.

## BRIEF DESCRIPTION OF THE FIGURES

**Figure 1:** Diagram of a V-C-Fc-V-C antibody. The various regions are shown as follows: oval filled with heavy horizontal lines, immunoglobulin variable region (labeled V-1 through V-4); unfilled rectangle, immunoglobulin constant region (labeled C-1 through C-4), which can be either a CH1 or a CL region; oval filled with diagonal lines, CH2 region (labeled CH2-1 and CH2-2); oval filled with windowpane checks, CH3 region (labeled CH3-1 and CH3-2); vertical line between the most N-terminal immunoglobulin constant region and the CH2 region, hinge region; horizontal or nearly horizontal line, disulfide bond; vertical line between the CH3 region and the following immunoglobulin variable region, linker; and short vertical line following one of the C-terminal immunoglobulin constant regions, a portion of a hinge region. The position of the Fc region is indicated.

**Figure 2:** Diagrams of particular embodiments of V-C-Fc-V-C antibodies. In panels A, B, C, and D, respectively, are diagrammed V-C-Fc-V-C antibodies called aCD3-Fc-aFOLR1, aFOLR1-Fc-aCD3, aHER2-Fc-aCD3, and aCD3-Fc-aHER2. The construction of nucleic acids encoding these and other antibodies are described in Example 1. Amino acid sequences of the two polypeptide chains that constitute each of these are provided in SEQ ID NOs: 1 and 3 (aFOLR1-Fc-aCD3), SEQ ID NOs: 5 and 7 (aCD3-Fc-aFOLR1), SEQ ID NOs: 9 and 11 (aHER2-Fc-aCD3), and SEQ ID NOs:46 and 48 (aCD3-Fc-aHER2). The various regions are shown as follows: oval filled with heavy horizontal lines, VH region; rectangle filled with heavy horizontal lines, CH1 region; vertical line between the CH1 or CL region and the CH2 region, hinge region; horizontal or nearly horizontal line, disulfide bond; oval filled with diagonal lines, CH2 region; oval filled with windowpane checks, CH3 region; vertical line between the CH3 region and the following immunoglobulin variable region, linker; oval filled with heavy vertical lines, VL region; rectangle filled with heavy vertical lines, CL region; and vertical line following the most carboxyterminal CH1 region, a portion of the hinge.

**Figure 3:** Binding of aFOLR1-Fc-aCD3 or sc-aFOLR1-sc-aCD3 to cells expressing FOLR1 or CD3. The binding of aFOLR1-Fc-aCD3 and sc-aFOLR1-sc-aCD3 to T47D tumor cells, which express FOLR1, and T cells, which express CD3, was analyzed by fluorescence activated cell sorting (FACS) as described in Example 2. The x axes show the intensity of fluorescence detected (MFI), and the y axes show the number of cells. Data from T47D cells and T cells are shown in the top and bottom two panels, respectively, as indicated. Data from aFOLR1-Fc-aCD3 and sc-aFOLR1-sc-aCD3 are shown in the left and right two panels, respectively, as indicated.

**Figure 4:** T cell dependent cell cytolysis induced by aFOLR1-Fc-aCD3 or sc-aFOLR1-sc-aCD3. Assays were performed as described in Example 2 using the following three different target cell lines: panel A, T47D, which expresses about 101,000 molecules of FOLR1 on the surface of each cell; panel B, Cal-51, which expresses about 148,000 molecules of FOLR1 on the surface of each cell; and panel C, BT474, which expresses no detectable FOLRI on the surface of the cells. Filled circles represent data from aFOLR1-Fc-aCD3 (which is described in Example 1), and open circles represent data from sc-aFOLR1-sc-aCD3, which is described in Example 2. The x axis indicates the concentration of protein used, and the y axis indicates the percent of target cells lysed.

**Figure 5:** T cell dependent cell cytolysis induced by aFOLR1-Fc-aCD3, aCD3-Fc-aFOLR1, or sc-aFOLR1-sc-aCD3.

Assays were performed as described in Example 2 using Cal-51 cells, as indicated. Symbols indicate data from assays containing the following bispecific molecules: open circles with dashed line, sc-aFOLR1-sc-aCD3; filled squares with solid line, aCD3-Fc-aFOLR1; and open squares with dashed line, aFOLR1-Fc-aCD3. The x axis indicates the concentration of protein used, and the y axis indicates the percent of target cells lysed.

**Figure 6:** Binding of aHER2-Fc-aCD3 or sc-HER2-sc-aCD3 to cells expressing HER2 or CD3. The binding of aHER2-Fc-aCD3 and sc-aHER2-sc-aCD3 to SKOV-3 tumor cells, which express HER2, and T cells, which express CD3, was analyzed by FACS as described in Example 3. Data from SKOV-3 cells and T cells are shown in the top and bottom two panels, respectively, as indicated. Data from aHER2-Fc-aCD3 and sc-aHER2-sc-aCD3 are shown in the left and right two panels, respectively, as indicated. The x axes show the intensity of fluorescence detected (MFI), and the y axes show the number of cells.

**Figure 7:** T cell dependent cell cytolysis induced by aHER2-Fc-aCD3 or sc-aHER2-sc-aCD3. Assays were performed as described in Example 3 using the following three different target cell lines: top panel, SKOV-3, which expresses about 530,000 molecules of HER2 on the surface of each cell; middle panel, BT474, which expresses about 300,000 molecules of HER2 on the surface of each cell; and bottom panel, SHP77, which expresses no detectable HER2 on the surface of the cells. Filled circles represent data from aHER2-Fc-aCD3, which is described in Example 1, and open circles represent data from the sc-aHER2-sc-aCD3, which is described in Example 3. The x axis indicates the concentration (pM) of protein used, and the y axis indicates the percent of target cells lysed.

**Figure 8:** T cell dependent cell cytolysis induced by aHER2-Fc-aCD3, aCD3-Fc-aHER2, or sc-aHER2-sc-aCD3. Assays were performed as described in Example 3, except that JIMT-1 cells, which express about 181,000 molecules of HER2 per cell, were used as target cells. Symbols indicate data from assays containing the following bispecific molecules: open circles with dashed line, sc-aHER2-sc-aCD3; filled squares with solid line, aCD3-Fc-aHER2; and open squares with dashed line, aHER2-Fc-aCD3. The x axis indicates the concentration of protein used, and the y axis indicates the percent of target cells lysed.

**Figure 9:** Activation of T cells by aHER2-Fc-aCD3 or sc-aHER2-sc-aCD3. In the top panel the y axis shows the percent of T cells in a population incubated with either aHER2-Fc-aCD3 (filled circles) or sc-HER2-sc-aCD3 (open circles) that are dividing. In the bottom panel the y axis shows the percent of cells expressing CD25 in a population of T cells incubated with either aHER2-Fc-aCD3 (filled circles) or sc-aHER2-sc-aCD3 (open circles). The x axis shows the concentration of protein used (pM). Methods are described in Example 3.

**Figure 10:** Pharmacokinetics following intravenous injection. Two different bispecific antibodies, a V-C-Fc-V-C (aHER2-Fc-aCD3) and a single chain antibody (sc-aHER2-sc-aCD3), were injected intravenously into mice, and concentrations were assessed over time as described in Example 4. Symbols indicate data from animals injected with sc-aHER2-sc-aCD3 (filled circles), or aHER2-Fc-aCD3 (filed squares).

**Figure 11:** Pharmacokinetics following subcutaneous injection. Two different bispecific antibodies, a V-C-Fc-V-C (aHER2-Fc-aCD3) and a single chain antibody (sc-aHER2-sc-aCD3), were injected intravenously into mice, and concentrations were assessed over time as described in Example 4. Symbols indicate data from animals injected with sc-aHER2-sc-aCD3 (filled circles), or aHER2-Fc-aCD3 (filed squares).

**Figure 12A:** Binding of V-C-Fc-V-Cs with and without protease cleavage sites to T cells. Methods are described in Example 2. The x axis shows the concentration (nM) of protein used in the assay, and the y axis shows the geometric mean of the fluorescence detected in the FACS at that concentration. Symbols signify data from assays containing the following proteins: filled circles, the positive control heterodimeric antibody described in Example 5, comprising SEQ ID NOs:60 and 61; unfilled circles and unfilled squares, aHer2-Fc-aCD3, comprising SEQ ID NOs:9 and 11; filled triangles, aHer2-Fc-Fur-aCD3, comprising SEQ ID NOs: 11 and 62; and unfilled triangles, aHer2-Fc-MMP2-aCD3, comprising SEQ ID NOs: 11 and 64.

**Figure 12B:** Binding of V-C-Fc-V-Cs with and without protease cleavage sites to T47D cells. Methods are described in Example 2. The x axis shows the concentration of protein used in the assay, and the y axis shows the geometric mean of the fluorescence detected in the FACS at that concentration. Symbols signify as stated in the description of Figure 12A.

**Figure 13:** Cytolysis of of T47D cells in the presence of T cells and V-C-Fc-V-Cs with and without protease cleavage sites or control proteins. Cytolysis assays using T47D cells as target cells are described in Example 2. The x axis shows the concentration of protein used and the y axis shows the percent of target cells lysed. Symbols signify as stated in the description of Figure 12A.

## BRIEF DESCRIPTION OF THE SEQUENCE LISTINGS

| SEQ ID NO | Description |
|---|---|
| SEQ ID NO:1 | Amino acid sequence of one polypeptide chain of aFOLR1-Fc-aCD3 with signal sequence |
| SEQ ID NO:2 | Nucleic acid sequence encoding SEQ ID NO:1 |
| SEQ ID NO:3 | Amino acid sequence of another polypeptide chain of aFOLR1-Fc-aCD3 with signal sequence |
| SEQ ID NO:4 | Nucleic acid sequence encoding SEQ ID NO:3 |
| SEQ ID NO:5 | Amino acid sequence of one polypeptide chain of aCD3-Fc-aFOLR1 with signal sequence |
| SEQ ID NO:6 | Nucleic acid sequence encoding SEQ ID NO:5 |
| SEQ ID NO:7 | Amino acid sequence of another polypeptide chain of aCD3-Fc-aFOLR1 with signal sequence |
| SEQ ID NO:8 | Nucleic acid sequence encoding SEQ ID NO:7 |
| SEQ ID NO:9 | Amino acid sequence of one polypeptide chain of aHER2-Fc-aCD3 with signal sequence |
| SEQ ID NO:10 | Nucleic acid sequence encoding SEQ ID NO:9 |
| SEQ ID NO:11 | Amino acid sequence of a polypeptide chain of aHER2-Fc-aCD3, aHER2-Fc-Fur-aCD3, or aHER2-Fc-MMP2-aCD3, with signal sequence |
| SEQ ID NO:12 | Nucleic acid sequence encoding SEQ ID NO:11 |
| SEQ ID NO:13 | Amino acid sequence of peptide that extends half life of an Fc region |
| SEQ ID NO:14 | Amino acid sequence of peptide that extends half life of an Fc region |
| SEQ ID NO:15 | Amino acid sequence of peptide that extends half life of an Fc region |
| SEQ ID NO:16 | Amino acid sequence of peptide that extends half life of an Fc region |
| SEQ ID NO:17 | Amino acid sequence of peptide that extends half life of an Fc region |
| SEQ ID NO:18 | Amino acid sequence of peptide that extends half life of an Fc region |
| SEQ ID NO:19 | Amino acid sequence of peptide that extends half life of an Fc region |
| SEQ ID NO:20 | Amino acid sequence of peptide that extends half life of an Fc region |
| SEQ ID NO:21 | Amino acid sequence of peptide that extends half life of an Fc region |
| SEQ ID NO:22 | Amino acid sequence of peptide that extends half life of an Fc region |
| SEQ ID NO:23 | Amino acid sequence of peptide that extends half life of an Fc region |
| SEQ ID NO:24 | Amino acid sequence of peptide that extends half life of an Fc region |
| SEQ ID NO:25 | Amino acid sequence of human fibronectin 3 domain engineered to bind serum albumin |
| SEQ ID NO:26 | Amino acid sequence of human IgG1 Fc polypeptide chain |
| SEQ ID NO:27 | Amino acid sequence of human IgG2 Fc polypeptide chain |
| SEQ ID NO:28 | Amino acid sequence of human IgG3 Fc polypeptide chain |
| SEQ ID NO:29 | Amino acid sequence of human IgG4 Fc polypeptide chain |
| SEQ ID NO:30 | Amino acid sequence preceding heavy chain CDR1 |
| SEQ ID NO:31 | Amino acid sequence preceding heavy chain CDR2 |
| SEQ ID NO:32 | Amino acid sequence following heavy chain CDR3 |
| SEQ ID NO:33 | Amino acid sequence following light chain CDR3 |
| SEQ ID NO:34 | Amino acid sequence of a linker |
| SEQ ID NO:35 | Amino acid sequence of a linker |
| SEQ ID NO:36 | Amino acid sequence of a linker |
| SEQ ID NO:37 | Amino acid sequence of a linker |
| SEQ ID NO:38 | Amino acid sequence of a linker |

(continued)

| SEQ ID NO | Description |
|---|---|
| SEQ ID NO:39 | Mature amino acid sequence of CD3ε of *Homo sapiens* |
| SEQ ID NO:40 | Mature amino acid sequence of CD3ε of *Macaca fascicularis* |
| SEQ ID NO:41 | Amino acid sequence of a portion of an epitope on human CD3ε |
| SEQ ID NO:42 | Amino acid sequence of sc-aHER2-sc-aCD3 with signal sequence |
| SEQ ID NO:43 | Nucleic acid sequence encoding SEQ ID NO:42 |
| SEQ ID NO:44 | Amino acid sequence of sc-aFOLR1-sc-aCD3 with signal sequence |
| SEQ ID NO:45 | Nucleic acid sequence encoding SEQ ID NO:44 |
| SEQ ID NO:46 | Amino acid sequence of one polypeptide chain of aCD3-Fc-aHER2 with signal sequence |
| SEQ ID NO:47 | Nucleic acid sequence encoding SEQ ID NO:46 |
| SEQ ID NO:48 | Amino acid sequence of another polypeptide chain of aCD3-Fc-aHER2 with signal sequence |
| SEQ ID NO:49 | Nucleic acid sequence encoding SEQ ID NO:48 |
| SEQ ID NO:50 | Amino acid sequence of a linker |
| SEQ ID NO:51 | Amino acid sequence of a linker that can be cleaved by a metalloproteinase |
| SEQ ID NO:52 | Amino acid sequence of a linker that can be cleaved by a furin |
| SEQ ID NO:53 | Amino acid sequence of a linker that can be cleaved by a protease |
| SEQ ID NO:54 | Amino acid sequence of a linker that can be cleaved by a protease |
| SEQ ID NO:55 | Amino acid sequence of a linker that can be cleaved by a protease |
| SEQ ID NO:56 | Amino acid sequence of a linker that can be cleaved by a protease |
| SEQ ID NO:57 | Amino acid sequence of a linker that can be cleaved by a protease |
| SEQ ID NO:58 | Amino acid sequence of a linker that can be cleaved by a protease |
| SEQ ID NO:59 | Amino acid sequence that can be cleaved by matrix metalloproteinase 9 (MMP9) |
| SEQ ID NO:60 | Amino acid sequence of one polypeptide chain of aCD3/HER2 heterodimer (P136797.3) |
| SEQ ID NO:61 | Amino acid sequence of the other polypeptide chain of aCD3/HER2 heterodimer (P136797.3) |
| SEQ ID NO:62 | Amino acid sequence a second polypeptide chain of aHER2-Fc-Fur-aCD3 (PL-35586) with signal sequence |
| SEQ ID NO:63 | Nucleic acid sequence encoding SEQ ID NO:62 |
| SEQ ID NO:64 | Amino acid sequence a second polypeptide chain of aHER2-Fc-MMP2-aCD3 (PL-35589) with signal sequence |
| SEQ ID NO:65 | Nucleic acid sequence encoding SEQ ID NO:64 |
| SEQ ID NO:66 | Amino acid sequence of an MMP2 cleavage site |
| SEQ ID NO:67 | Amino acid sequence of an anti-CD3 VH region |
| SEQ ID NO:68 | Nucleic acid sequence encoding SEQ ID NO:67 |
| SEQ ID NO:69 | Amino acid sequence of an anti-CD3 VL region |
| SEQ ID NO:70 | Nucleic acid sequence encoding SEQ ID NO:69 |
| SEQ ID NO:71 | Amino acid sequence of a meprin a or β cleavage site |
| SEQ ID NO:72 | Amino acid sequence of a meprin a or β cleavage site |
| SEQ ID NO:73 | Amino acid sequence of a meprin a or β cleavage site |
| SEQ ID NO:74 | Amino acid sequence of a meprin α or β cleavage site |

(continued)

| SEQ ID NO | Description |
|---|---|
| SEQ ID NO:75 | Amino acid sequence of a urokinase-type plasminogen activator (u-PA) cleavage site |
| SEQ ID NO:76 | Amino acid sequence of a u-PA cleavage site |
| SEQ ID NO:77 | Amino acid sequence of a u-PA cleavage site |
| SEQ ID NO:78 | Amino acid sequence of a u-PA cleavage site |
| SEQ ID NO:79 | Amino acid sequence of a u-PA cleavage site |
| SEQ ID NO:80 | Amino acid sequence of a u-PA cleavage site |
| SEQ ID NO:81 | Amino acid sequence of a u-PA cleavage site |
| SEQ ID NO:82 | Amino acid sequence of a tissue plasminogen activator (tPA) cleavage site |
| SEQ ID NO:83 | Amino acid sequence of a cathepsin B cleavage site |
| SEQ ID NO:84 | Amino acid sequence of a cathepsin B cleavage site |
| SEQ ID NO:85 | Amino acid sequence of a cathepsin B cleavage site |
| SEQ ID NO:86 | Amino acid sequence of a cathepsin B cleavage site |
| SEQ ID NO:87 | Amino acid sequence of a cathepsin B cleavage site |
| SEQ ID NO:88 | Amino acid sequence of a cathepsin B cleavage site |
| SEQ ID NO:89 | Amino acid sequence of a cathepsin B cleavage site |
| SEQ ID NO:90 | Amino acid sequence of a cathepsin B cleavage site |
| SEQ ID NO:91 | Amino acid sequence of a cathepsin B cleavage site |
| SEQ ID NO:92 | Amino acid sequence of a cathepsin B cleavage site |
| SEQ ID NO:93 | Amino acid sequence of a cathepsin B cleavage site |
| SEQ ID NO:94 | Amino acid sequence of a cathepsin B cleavage site |
| SEQ ID NO:95 | Amino acid sequence of a cathepsin B cleavage site |
| SEQ ID NO:96 | Amino acid sequence of a cathepsin B cleavage site |
| SEQ ID NO:97 | Amino acid sequence of a furin cleavage site |
| SEQ ID NO:98 | Amino acid sequence of a furin cleavage site |
| SEQ ID NO:99 | Amino acid sequence of an MMP2 cleavage site |
| SEQ ID NO:100 | Amino acid sequence of an MMP2 cleavage site |
| SEQ ID NO:101 | Amino acid sequence of a cathepsin B cleavage site |

## DETAILED DESCRIPTION

Described herein is a new format for a bispecific antibody. It is a heterodimeric molecule containing two different polypeptide chains. Each polypeptide chain comprises an Fc polypeptide chain, which is between two different segments, each containing a heavy or light chain variable (VH or VL) region and either a heavy chain constant region 1 (CH1) or a light chain constant region (CL). This format is diagramed in Figure 1. Together, the two chains contain two different binding sites, each of which comprises a VH and a VL region, and each of which binds to a different molecule, optionally a protein. In some embodiments, one of the proteins is expressed on the surface of an immune effector cell, such as a T cell, an NK cell, a macrophage, or a neutrophil and the other protein is expressed on the surface of a target cell, for example a cancer cell, a cell infected by a pathogen such as a virus, or a cell that mediates a fibrotic, autoimmune, or inflammatory disease. Since a V-C-Fc-V-C antibody, as described herein, has only one binding site for each of the molecules or proteins it binds to (*i.e.*, it binds "monovalently" to each molecule or protein), its binding cannot oligomerize the molecules or proteins it binds to on a cell surface in some situations, *e.g.*, if it is bound to only one of its target proteins. For example, if it binds to CD3 on the surface of a T cell, CD3 will not be oligomerized on the T cell surface.

Oligomerization of CD3 can cause a generalized activation of a T cell, which can be undesirable. The V-C-Fc-V-C antibody described herein can tether an immune effector cell to a target cell, forming a close physical association between the cells, thereby eliciting a specific cytolytic activity against the target cell rather than a generalized inflammatory response. For example, when an immune effector cell protein, for example CD3 on a T cell, and a target cell protein are simultaneously engaged by a V-C-Fc-V-C, the CD3 can be oligomerized on the T cell, which is thereby activated to kill the target cell to which it is tethered. The mechanism of action may be similar to that explored in detail for other bispecific antibodies. *See, e.g.,* Haas et al. (2009), Immunobiology 214(6): 441-453. Further, the V-C-Fc-V-C antibodies can comprise at least one, optionally two, half life-extending moieties. Thus, they can have favorable pharmacokinetic properties and can be relatively easy to manufacture since they contain only two different polypeptide chains.

***Definitions***

Antibodies comprise various domains or regions. For example, an antibody in the IgG format found in nature comprises four polypeptide chains, two heavy chains and two light chains. Each heavy chain comprises a VH region followed by a first heavy chain constant (CH1) region, a hinge region, a second heavy chain constant (CH2) region, and a third heavy chain constant (CH3) region. Each light chain comprises a light chain variable (VL) region and a light chain constant (CL) region. These regions are described in, *e.g*., Carayannopoulos and Capra, Immunoglobulins: Structure and Function, pp. 283-314, in Fundamental Immunology, 3rd Edition, Paul, ed., Raven Press, New York, 1993, the relevant portions of which, *i.e.,* pp. 283-304, are incorporated herein by reference.

An **"antibody,"** as meant herein, is a protein containing at least one VH or VL region, in many cases a VH and a VL region. Thus, the term "antibody" encompasses molecules having a variety of formats, including single chain Fv antibodies (scFv, which contain VH and VL regions joined by a linker), Fab, $F(ab)_2'$, Fab', scFv:Fc antibodies (as described in Carayannopoulos and Capra, Ch. 9 in FUNDAMENTAL IMMUNOLOGY, 3rd ed., Paul, ed., Raven Press, New York, 1993, pp. 284-286) or full length antibodies containing two full length heavy and two full length light chains, such as naturally-occurring IgG antibodies found in mammals. *Id.* Such IgG antibodies can be of the IgG1, IgG2, IgG3, or IgG4 isotype and can be human antibodies. The portions of Carayannopoulos and Capra that describe the structure of antibodies are incorporated herein by reference. Further, the term "antibody" includes dimeric antibodies containing two heavy chains and no light chains such as the naturally-occurring antibodies found in camels and other dromedary species and sharks. *See, e.g.,* Muldermans et al., 2001, J. Biotechnol. 74:277-302; Desmyter et al., 2001, J. Biol. Chem. 276:26285-90; Streltsov et al. (2005), Protein Science 14: 2901-2909. An antibody can be **"monospecific"** (that is, binding to only one kind of antigen), **"bispecific"** (that is, binding to two different antigens), or **"multispecific"** (that is, binding to more than one different antigen). Further, an antibody can be monovalent, bivalent, or multivalent, meaning that it can bind to one, two, or multiple antigen molecules at once, respectively. An antibody binds **"monovalently"** to a particular protein when one molecule of the antibody binds to only one molecule of the protein, even though the antibody may also bind to a different protein as well. That is, an antibody binds "monovalently," as meant herein, to two different proteins when it binds to only one molecule of each protein. Such an antibody is "bispecific" and binds to each of two different proteins "monovalently." An antibody can be **"monomeric,"** *i.e*., comprising a single polypeptide chain. An antibody can comprise multiple polypeptide chains (**"multimeric"**) or can comprise two (**"dimeric"**), three (**"trimeric"**), or four (**"tetrameric"**) polypeptide chains. If multimeric, an antibody can be a homomultimer, *i.e.,* containing more than one molecule of only one kind of polypeptide chain, including homodimers, homotrimer, or homotetramers. Alternatively, a multimeric antibody can be a heteromultimer, *i.e.,* containing more than one different kind of polypeptide chain, including heterodimers, heterotrimers, or heterotetramers. An antibody can have a variety of possible formats including, for example, monospecific monovalent antibodies (as described in International Application WO 2009/089004 and US Publication 2007/0105199, the relevant portions of which are incorporated herein by reference) that may inhibit or activate the molecule to which they bind, bivalent monospecific or bispecific dimeric Fv-Fc, scFv-Fc, or diabody Fc, monospecific monovalent scFv-Fc/Fc's, the multispecific binding proteins and dual variable domain immunoglobulins described in US Publication 2009/0311253 (the relevant portions of which are incorporated herein by reference), the V-C-Fc-V-C antibodies described herein, and the many formats for bispecific antibodies described in Chapters 1, 4, 5, 6, 7, 8, 9, 10, 11, 12, 13, 14 of BISPECIFIC ANTIBODIES, Kontermann, ed., Springer, 2011 (which chapters are incorporated herein by reference), among many other possible antibody formats.

A **"cancer cell antigen,"** as meant herein, is a protein expressed on the surface of a cancer cell. Some cancer cell antigens are also expressed on some normal cells, and some are specific to cancer cells. Cancer cell antigens can be highly expressed on the surface of a cancer cell. There are a wide variety of cancer cell antigens. Examples of cancer cell antigens include, without limitation, the following human proteins: epidermal growth factor receptor (EGFR), EGFRvIII (a mutant form of EGFR), melanoma-associated chondroitin sulfate proteoglycan (MCSP), mesothelin (MSLN), folate receptor 1 (FOLR1), and human epidermal growth factor 2 (HER2), among many others.

**"Chemotherapy,"** as used herein, means the treatment of a cancer patient with a **"chemotherapeutic agent"** that has cytotoxic or cytostatic effects on cancer cells. A **"chemotherapeutic agent"** specifically targets cells engaged in cell division and not cells that are not engaged in cell division. Chemotherapeutic agents directly interfere with processes that are intimately tied to cell division such as, for example, DNA replication, RNA synthesis, protein synthesis, the assembly, disassembly, or function of the mitotic spindle, and/or the synthesis or stability of molecules that play a role in these processes, such as nucleotides or amino acids. A chemotherapeutic agent therefore has cytotoxic or cytostatic effects on both cancer cells and other cells that are engaged in cell division. Chemotherapeutic agents are well-known in the art and include, for example: alkylating agents (*e.g.* busulfan, temozolomide, cyclophosphamide, lomustine (CCNU), methyllomustine, streptozotocin, *cis*-diamminedi-chloroplatinum, aziridinylbenzo-quinone, and thiotepa); inorganic ions (*e.g.* cisplatin and carboplatin); nitrogen mustards (*e.g.* melphalan hydrochloride, ifosfamide, chlorambucil, and mechlorethamine HCl); nitrosoureas (*e.g.* carmustine (BCNU)); anti-neoplastic antibiotics (*e.g.* adriamycin (doxorubicin), daunomycin, mitomycin C, daunorubicin, idarubicin, mithramycin, and bleomycin); plant derivatives (*e.g.* vincristine, vinblastine, vinorelbine, paclitaxel, docetaxel, vindesine, VP-16, and VM-26); antimetabolites (*e.g.* methotrexate with or without leucovorin, 5-fluorouracil with or without leucovorin, 5-fluorodeoxyuridine, 6-mercaptopurine, 6-thioguanine, cytarabine, 5-azacytidine, hydroxyurea, deoxycoformycin, gemcitabine, and fludarabine); podophyllotoxins (*e.g.* etoposide, irinotecan, and topotecan); as well as actinomycin D, dacarbazine (DTIC), mAMSA, procarbazine, hexamethylmelamine, pentamethylmelamine, L-asparaginase, and mitoxantrone, among many known in the art. *See e.g.* Cancer: Principles and Practice of Oncology, 4th Edition, DeVita et al., eds., J.B. Lippincott Co., Philadelphia, PA (1993), the relevant portions of which are incorporated herein by reference. Alkylating agents and nitrogen mustard act by alkylating DNA, which restricts uncoiling and replication of strands. Methotrexate, cytarabine, 6-mercaptopurine, 5-fluorouracil, and gemcitabine interfere with nucleotide synthesis. Plant derivatives such a paclitaxel and vinblastine are mitotic spindle poisons. The podophyllotoxins inhibit topoisomerases, thus interfering with DNA replication. Antibiotics doxorubicin, bleomycin, and mitomycin interfere with DNA synthesis by intercalating between the bases of DNA (inhibiting uncoiling), causing strand breakage, and alkylating DNA, respectively. Other mechanisms of action include carbamoylation of amino acids (lomustine, carmustine), and depletion of asparagine pools (asparaginase). Merck Manual of Diagnosis and Therapy, 17th Edition, Section 11, Hematology and Oncology, 144. Principles of Cancer Therapy, Table 144-2 (1999). Specifically included among chemotherapeutic agents are those that directly affect the same cellular processes that are directly affected by the chemotherapeutic agents listed above.

A **"cleavage site for a protease,"** as meant herein, is an amino acid sequence that can be cleaved by a protease, such as, for example, a matrix metalloproteinase or a furin. Examples of such sites include Gly-Pro-Leu-Gly-Ile-Ala-Gly-Gln (SEQ ID NO:51) or Ala-Val-Arg-Trp-Leu-Leu-Thr-Ala (SEQ ID NO:59), which can cleaved by metalloproteinases, and Arg-Arg-Arg-Arg-Arg-Arg (SEQ ID NO:52), which is cleaved by a furin. Linkers including such cleavage site include, without limitation, linkers comprising the amino acid sequences of any one of SEQ ID NOs: 51-59. Further protease cleavage sites include, without limitation, the amino acid sequences of SEQ ID NOs:71-101. In therapeutic applications, the protease cleavage site can be cleaved by a protease that is produced by target cells, for example cancer cells or infected cells, or pathogens.

A drug or treatment is **"concurrently"** administered with a V-C-Fc-V-C antibody, as meant herein, if it is administered in the same general time frame as the antibody, optionally, on an ongoing basis. For example, if a patient is taking Drug A once a week on an ongoing basis and the V-C-Fc-V-C once every six months on an ongoing basis, Drug A and the V-C-Fc-V-C are concurrently administered, whether or not they are ever administered on the same day. Similarly, if the V-C-Fc-V-C is taken once per week on an ongoing basis and Drug A is administered only once or a few times on a daily basis, Drug A and the V-C-Fc-V-C are concurrently administered as meant herein. Similarly, if both Drug A and the V-C-Fc-V-C are administered for short periods of time either once or multiple times within a one month period, they are administered concurrently as meant herein as long as both drugs are administered within the same month. The V-C-Fc-V-C antibodies described herein can be administered before, after, or concurrently with a chemotherapeutic agent, a non-chemotherapeutic anti-neoplastic agent, radiation, or any other cancer treatment.

A **"conservative amino acid substitution,"** as meant herein, is a substitution of an amino acid with another amino acid with similar properties. Properties considered include chemical properties such as charge and hydrophobicity. Table 1 below lists substitutions for each amino acid that are considered to be conservative substitutions as meant herein.

**Table 1: Conservative Amino Acid Substitutions**

| Original Residue | Conservative Substitutions |
|---|---|
| Ala | Val, Leu, Ile |
| Arg | Lys, Gln, Asn |
| Asn | Gln |
| Asp | Glu |
| Cys | Ser, Ala |

(continued)

| Original Residue | Conservative Substitutions |
|---|---|
| Gln | Asn |
| Glu | Asp |
| Gly | Pro, Ala |
| His | Asn, Gln, Lys, Arg |
| Ile | Leu, Val, Met, Ala, Phe, Norleucine |
| Leu | Norleucine, Ile, Val, Met, Ala, Phe |
| Lys | Arg, Gln, Asn |
| Met | Leu, Phe, Ile |
| Phe | Leu, Val, Ile, Ala, Tyr |
| Pro | Ala |
| Ser | Thr, Ala, Cys |
| Thr | Ser |
| Trp | Tyr, Phe |
| Tyr | Trp, Phe, Thr, Ser |
| Val | Ile, Met, Leu, Phe, Ala, Norleucine |

A **"Fab fragment,"** as meant herein is a portion of a full length tetrameric antibody, for example an IgG antibody, resulting from cleavage of the antibody within or near the hinge region. A Fab fragment is at least a dimer containing a polypeptide chain comprising a VH and a CH1 region and another polypeptide comprising a VL and a CL region. In some embodiments, the polypeptide chains of a Fab fragment also comprise part or all of a hinge region. In some embodiments, the polypeptide chains of a Fab fragment can contain some or all of the cysteine residues in the hinge region. In such Fab fragments, two Fab fragments can be linked by one or more disulfide bonds to form tetrameric, bivalent Fab fragments known as $F(ab)'_2$ fragments. *See* Carayannopoulos and Capra, Immunoglobulins: Structure and Function, Ch. 9, pp. 283-314, at 284-285, in FUNDAMENTAL IMMUNOLOGY, THIRD EDITION, Paul, ed., Raven Press, New York, 1993, which is incorporated herein by reference. A **"half Fab fragment,"** as meant herein comprises a single polypeptide chain comprising a VH and a CH1 region, optionally plus part or all of a hinge region, or a VL and a CL region, optionally plus part or all of a hinge region.

As meant herein, an **"Fc region"** is a dimer consisting of two polypeptide chains joined by one or more disulfide bonds, each chain comprising part or all of a hinge domain plus a CH2 and a CH3 domain. Each of the polypeptide chains is referred to as an **"Fc polypeptide chain."** To distinguish the two Fc polypeptide chains, in some instances one is referred to herein as an **"A chain"** and the other is referred to as a **"B chain."** More specifically, the Fc regions contemplated for use with the present invention are IgG Fc regions, which can be mammalian, for example human, IgG1, IgG2, IgG3, or IgG4 Fc regions. Among human IgG1 Fc regions, at least two allelic types are known. In other embodiments, the amino acid sequences of the two Fc polypeptide chains can vary from those of a mammalian Fc polypeptide by no more than 15 substitutions, insertions, and/or deletions of a single amino acid per 100 amino acids of sequence relative to a mammalian Fc polypeptide amino acid sequence. In some embodiments, such variations can be "heterodimerizing alterations" that facilitate the formation of heterodimers over homodimers, an Fc alteration that extends half life, an alteration that reduces Fc gamma receptor (Fc$\gamma$R) binding, and/or an alteration that enhances ADCC.

An **"Fc alteration that extends half life,"** as meant herein is an alteration within an Fc polypeptide chain that lengthens the *in vivo* half life of a protein that contains the altered Fc polypeptide chain as compared to the half life of a similar protein containing the same Fc polypeptide, except that it does not contain the alteration. Such alterations can be included in an Fc polypeptide chain that is part of a V-C-Fc-V-C antibody as described herein. The alterations M252Y, S254T, and T256E (methionine at position 252 changed to tyrosine; serine at position 254 changed to threonine; and threonine at position 256 changed to glutamic acid; numbering according to EU numbering as shown in Table 2) are Fc alterations that extend half life and can be used together, separately or in any combination. These alterations and a number of others are described in detail in U.S. Patent 7,083,784. The portions of U.S. Patent 7,083,784 that describe such alterations are incorporated herein by reference. Similarly, M428L and N434S are Fc alterations that extend half life and can be used together, separately or in any combination. These alterations and a number of others are described in detail in U.S. Patent Application Publication 2010/0234575 and U.S. Patent 7,670,600. The portions of U.S. Patent Application Publication 2010/0234575 and U.S. Patent 7,670,600 that describe such alterations are incorporated herein by reference. In addition, any substitution at one or more of the following sites can be considered an Fc alteration that extends half life as meant herein: 250, 251, 252, 259, 307, 308, 332, 378, 380, 428, 430, 434, and 436. Each of these alterations or combinations of these alterations can be used to extend the half life of a V-C-Fc-V-C antibody as described herein. Other alterations that can be used to extend half life are described in detail in International Application Publication WO 2013/096221. The portions of this application that describe such alterations are incorporated herein by reference. Some specific embodiments described in this application include insertions between positions 384 and 385 (EU numbering as shown in Table 2) that extend half life, including the following amino acid sequences: GGCVFNMFNCGG (SEQ ID NO:13), GGCHLPFAVCGG (SEQ ID NO:14), GGCGHEYMWCGG (SEQ ID NO:15), GGCWPLQDYCGG(SEQ ID NO:16), GGCMQMNKWCGG (SEQ ID NO:17), GGCDGRTKYCGG (SEQ ID NO:18), GGCALYPTNCGG (SEQ ID NO:19), GGCGKHWHQCGG (SEQ ID NO:20), GGCHSFKHFCGG (SEQ ID NO:21), GGCQGMWTWCGG (SEQ ID NO:22), GGCAQQWHHEYCGG (SEQ ID NO:23), and GGCERFHHACGG (SEQ ID NO:24), among others. V-C-Fc-V-C antibodies containing such insertions are contemplated.

A **"half life-extending moiety,"** as meant herein, is a molecule that extends the *in vivo* half life of a protein to which it is attached as compared to the *in vivo* half life of the protein without the half life-extending moiety. Methods for measuring half life are well known in the art. For example, a method for ascertaining half life is disclosed in Example 9. A half life-extending moiety can be a polypeptide, for example an Fc polypeptide chain or a polypeptide that can bind to albumin. The amino acid sequence of a domain of human fibronectin type III (Fn3) that has been engineered to bind to albumin is provided in SEQ ID NO:25, and various human IgG Fc polypeptide sequences are given in SEQ ID NOs:26-29. In alternate embodiments, a half life-extending moiety can be a non-polypeptide molecule. For example, a polyethylene glycol (PEG) molecule can be a half life-extending moiety.

**"Heterodimerizing alterations"** generally refer to alterations in the A and B chains of an Fc region that facilitate the formation of heterodimeric Fc regions, that is, Fc regions in which the A chain and the B chain of the Fc region do not have identical amino acid sequences. Such alterations can be included in an Fc polypeptide chain that is part of a V-C-Fc-V-C antibody as described herein. Heterodimerizing alterations can be asymmetric, that is, an A chain having a certain alteration can pair with a B chain having a different alteration. These alterations can facilitate heterodimerization and disfavor homodimerization. Whether hetero- or homodimers have formed can be assessed by size differences as determined by polyacrylamide gel electrophoresis in some situations or by other appropriate means such as differing charges or biophysical characteristics, including binding by antibodies or other molecules that recognize certain portions of the heterodimer including molecular tags. One example of such paired heterodimerizing alterations are the so-called "knobs and holes" substitutions. *See, e.g.,* US Patent 7,695,936 and US Patent Application Publication 2003/0078385, the portions of which describe such mutations are incorporated herein by reference. As meant herein, an Fc region that contains one pair of knobs and holes substitutions, contains one substitution in the A chain and another in the B chain. For example, the following knobs and holes substitutions in the A and B chains of an IgG1 Fc region have been found to increase heterodimer formation as compared with that found with unmodified A and B chains: 1) Y407T in one chain and T366Y in the other; 2) Y407A in one chain and T366W in the other; 3) F405A in one chain and T394W in the other; 4) F405W in one chain and T394S in the other; 5) Y407T in one chain and T366Y in the other; 6) T366Y and F405A in one chain and T394W and Y407T in the other; 7) T366W and F405W in one chain and T394S and Y407A in the other; 8) F405W and Y407A in one chain and T366W and T394S in the other; and 9) T366W in one polypeptide of the Fc and T366S, L368A, and Y407V in the other. This way of notating mutations can be explained as follows. The amino acid (using the one letter code) normally present at a given position in the CH3 region using the EU numbering system (which is presented in Edelman et al. (1969), Proc. Natl. Acad. Sci. 63: 78-85; *see also* Table 2 below) is followed by the EU position, which is followed by the alternate amino acid that is present at that position. For example, Y407T means that the tyrosine normally present at EU position 407 is replaced by a threonine. Alternatively or in addition to such alterations, substitutions creating new disulfide bridges can facilitate heterodimer formation. *See, e.g.,* US Patent Application Publication 2003/0078385, the portions of which describe such mutations are incorporated herein by reference. Such alterations in an IgG1 Fc region include, for example, the following substitutions: Y349C in one Fc polypeptide chain and S354C in the other; Y349C in one Fc polypeptide chain and E356C in the other; Y349C in one Fc polypeptide chain and E357C in the other; L351C in one Fc polypeptide chain and S354C in the other; T394C in one Fc polypeptide chain and E397C in the other; or D399C in one Fc polypeptide chain and K392C in the other. Similarly, substitutions changing the charge of a one or more residue, for example, in the $C_H3$-$C_H3$ interface, can enhance heterodimer formation as explained in US Patent Application Publication 2010/0286374, the portions of which describe such substitutions are incorporated herein by reference. Such substitutions are referred to herein as **"charge pair substitutions,"** and an Fc region containing one pair of charge pair substitutions contains one substitution in the A chain and a different substitution in the B chain. General examples of charge pair substitutions include the following: 1) K409D or K409E in one chain plus D399K or D399R in the other; 2) K392D or K392E in one chain plus D399K or D399R in the other; 3) K439D or K439E in one chain plus E356K or E356R in the other; and 4) K370D or K370E in one chain plus E357K or E357R in the other. In addition, the substitutions R355D, R355E, K360D, or K360R in both chains can stabilize heterodimers when used with other heterodimerizing alterations. Specific charge pair substitutions can be used either alone or with other charge pair substitutions. Specific examples of single pairs of charge pair substitutions and combinations thereof include the following: 1) K409E in one chain plus D399K in the other; 2) K409E in one chain plus D399R in the other; 3) K409D in one chain plus D399K in the other; 4) K409D in one chain plus D399R in the other; 5) K392E in one chain plus D399R in the other; 6) K392E in one chain plus D399K in the other; 7) K392D in one chain plus D399R in the other; 8) K392D in one chain plus D399K in the other; 9) K409D and K360D in one chain plus D399K and E356K in the other; 10) K409D and K370D in one chain plus D399K and E357K in the other; 11) K409D and K392D in one chain plus D399K, E356K, and E357K in the other; 12) K409D and K392D on one chain and D399K on the other; 13) K409D and K392D on one chain plus D399K and E356K on the other; 14) K409D and K392D on one chain plus D399K and D357K on the other; 15) K409D and K370D on one chain plus D399K and D357K on the other; 16) D399K on one chain plus K409D and K360D on the other; and 17) K409D and K439D on one chain plus D399K and E356K on the other. Any of these heterodimerizing alterations can be used in the Fc regions of the V-C-Fc-V-C antibodies described herein.

An **"alteration that inhibits Fc γ R binding,"** as meant herein, is one or more insertions, deletions, or substitutions within an Fc polypeptide chain that inhibits the binding of Fc γ RIIA, Fc γ RIIB, and/or Fc γ RIIIA as measured, for example, by an ALPHALISA®-based competition binding assay (PerkinElmer, Waltham, MA). Such alterations can be included in an Fc polypeptide chain that is part of a V-C-Fc-V-C antibody, as described herein. More specifically, alterations that inhibit Fc gamma receptor (Fc γ R) binding include L234A, L235A, or any alteration that inhibits glycosylation at N297, including any substitution at N297. In addition, along with alterations that inhibit glycosylation at N297, additional alterations that stabilize a dimeric Fc region by creating additional disulfide bridges are also contemplated. Further examples of alterations that inhibit Fc γ R binding include a D265A alteration in one Fc polypeptide chain and an A327Q alteration in the other Fc polypeptide chain. Any such alterations can be used in the Fc regions of the V-C-Fc-V-C antibodies described herein.

An **"alteration that enhances ADCC,"** as meant herein is one or more insertions, deletions, or substitutions within an Fc polypeptide chain that enhances antibody dependent cell-mediated cytotoxicity (ADCC). Such alterations can be included in an Fc polypeptide chain that is part of a V-C-Fc-V-C antibody as described herein. Many such alterations are described in International Patent Application Publication WO 2012/125850. Portions of this application that describe such alterations are incorporated herein by reference. Such alterations can be included in an Fc polypeptide chain that is part of a V-C-Fc-V-C antibody as described herein. ADCC assays can be performed as follows. Cell lines that express high and lower amounts of a cancer cell antigen on the cell surface can be used as target cells. These target cells can be labeled with carboxyfluorescein succinimidyl ester (CFSE) and then washed once with phosphate buffered saline (PBS) before being deposited into 96-well microtiter plates with V-shaped wells. Purified immune effector cells, for example T cells or NK cells, can be added to each well. A monospecific antibody that binds to the cancer antigen and contains the alteration(s) being tested and an isotype-matched control antibody can be diluted in a 1:3 series and added to the wells. The cells can be incubated at 37°C with 5% $CO_2$ for 3.5 hrs. The cells can be spun down and re-suspended in 1x FACS buffer (1x phosphate buffered saline (PBS) containing 0.5% fetal bovine serum (FBS)) with the dye TO-PRO®-3 iodide (Molecular Probes, Inc. Corporation, Oregon, USA), which stains dead cells, before analysis by fluorescence activated cell sorting (FACS). The percentage of cell killing can be calculated using the following formula:

*(percent tumor cell lysis with bispecific – percent tumor cell lysis without bispecific)/*

*(percent total cell lysis – percent tumor cell lysis without bispecific)*

Total cell lysis is determined by lysing samples containing effector cells and labeled target cells without a bispecific molecule with cold 80% methanol. Exemplary alterations that enhance ADCC include the following alterations in the A and B chains of anFc region: (a) the A chain comprises Q311M and K334V substitutions and the B chain comprises L234Y, E294L, and Y296W substitutions or vice versa; (b) the A chain comprises E233L, Q311M, and K334V substitutions and the B chain comprises L234Y, E294L, and Y296W substitutions or vice versa; (c) the A chain comprises L234I, Q311M, and K334V substitutions and the B chain comprises L234Y, E294L, and Y296W substitutions or vice versa; (d) the A chain comprises S298T and K334V substitutions and the B chain comprises L234Y, K290Y, and Y296W substitutions or vice versa; (e) the A chain comprises A330M and K334V substitutions and the B chain comprises L234Y, K290Y, and Y296W substitutions or vice versa; (f) the A chain comprises A330F and K334V substitutions and the B chain comprises L234Y, K290Y, and Y296W substitutions or vice versa; (g) the A chain comprises Q311M, A330M, and K334V substitutions and the B chain comprises L234Y, E294L, and Y296W substitutions or vice versa; (h) the A chain comprises Q311M, A330F, and K334V substitutions and the B chain comprises L234Y, E294L, and Y296W substitutions or vice versa; (i) the A chain comprises S298T, A330M, and K334V substitutions and the B chain comprises L234Y, K290Y, and Y296W substitutions or vice versa; (j) the A chain comprises S298T, A330F, and K334V substitutions and the B chain comprises L234Y, K290Y, and Y296W substitutions or vice versa; (k) the A chain comprises S239D, A330M, and K334V substitutions and the B chain comprises L234Y, K290Y, and Y296W substitutions or vice versa; (l) the A chain comprises S239D, S298T, and K334V substitutions and the B chain comprises L234Y, K290Y, and Y296W substitutions or vice versa; (m) the A chain comprises a K334V substitution and the B chain comprises Y296W and S298C substitutions or vice versa; (n) the A chain comprises a K334V substitution and the B chain comprises L234Y, Y296W, and S298C substitutions or vice versa; (o) the A chain comprises L235S, S239D, and K334V substitutions and the B chain comprises L234Y, K290Y, and Y296W, substitutions or vice versa; (p) the A chain comprises L235S, S239D, and K334V substitutions and the B chain comprises L234Y, Y296W, and S298C substitutions or vice versa; (q) the A chain comprises Q311M and K334V substitutions and the B chain comprises L234Y, F243V, and Y296W substitutions or vice versa; (r) the A chain comprises Q311M and K334V substitutions and the B chain comprises L234Y, K296W, and S298C substitutions or vice versa; (s) the A chain comprises S239D and K334V substitutions and the B chain comprises L234Y, K290Y, and Y296W substitutions or vice versa; (t) the A chain comprises S239D and K334V substitutions and the B chain comprises L234Y, Y296W, and S298C substitutions or vice versa; (u) the A chain comprises F243V and K334V substitutions and the B chain comprises L234Y, K290Y, and Y296W, substitutions or vice versa; (v) the A chain comprises F243V and K334V substitutions and the B chain comprises L234Y, Y296W, and S298C substitutions or vice versa; (w) the A chain comprises E294L and K334V substitutions and the B chain comprises L234Y, K290Y, and Y296W substitutions or vice versa; (x) the A chain comprises E294L and K334V substitutions and the B chain comprises L234Y, Y296W, and S298C substitutions or vice versa; (y) the A chain comprises A330M and K334V substitutions and the B chain comprises L234Y and Y296W substitutions or vice versa; or (z) the A chain comprises A330M and K334V substitutions and the B chain comprises K290Y and Y296W substitutions or vice versa. Any such alterations can be used in the Fc regions of the V-C-Fc-V-C antibodies described herein.

A "C-1, C-2, C-3, or C-4," as meant in the context of a V-C-Fc-V-C, is an immunoglobulin constant region, which follows an immunoglobulin variable region. C-1 follows a variable region at the amino terminal end of the first polypeptide chain of a V-C-Fc-V-C, and C-2 is at the carboxy terminal end. C-3 follows a variable region at the amino terminal end of the second polypeptide chain of a V-C-Fc-V-C, and C-4 is at the carboxy terminal end. C-1, C-2, C-3, and C-4 can have the same or different amino acid sequences, and they are either CL or CH1 regions.

A **"human"** protein or region of a protein, such as an immunoglobulin region or domain, has essentially the same amino acid sequence as the human protein or region thereof found in nature, with the proviso that the "human" protein sequence may contain some minor sequence variations relative to a human sequence found in nature. Such minor variations, not to exceed 15, 14, 13, 12, 11, 10, 9, 8, 7, 6, 5, 4, 3, 2, or 1 substitution(s), insertion(s), or deletion(s) of a single amino acid per 100 amino acids of sequence (taking into consideration the entire length of the protein or region), can include, for example, charge pair substitutions, as described herein, or one or more alterations that enhance ADCC, also as described herein, among other possible minor variations.

An **"IgG antibody,"** as meant herein, is an antibody consisting essentially of two immunoglobulin IgG heavy chains and two immunoglobulin light chains, which can be kappa or lambda light chains. More specifically, the heavy chains contain a VH, a CH1, a hinge region, a CH2, and a CH3, while the light chains contain a VL and a CL. Numerous sequences of such immunoglobulin regions are known in the art. *See, e.g.,* Kabat et al. in SEQUENCES OF IMMUNOLOGICAL INTEREST, Public Health Service N.I.H., Bethesda, MD, 1991. Sequences of regions from IgG antibodies disclosed in Kabat *et al.* are incorporated herein by reference.

An **"immune effector cell,"** as meant herein, is a cell that is involved in the mediation of a cytolytic immune response, including, for example, T cells, NK cells, macrophages, or neutrophils. The V-C-Fc-V-C antibodies described herein bind to an antigen that is part of a molecule, optionally a protein, expressed on the surface of an immune effector cell. Such molecules are referred to herein as **"effector cell molecules"** or, in the case of proteins, **"effector cell proteins."**

An **"immunoglobulin heavy chain,"** as meant herein, consists essentially of a VH region, a CH1 region, a hinge region, a CH2 region, a CH3 region in that order, and, optionally, a region downstream of the CH3 region in some isotypes. Close variants of an immunoglobulin heavy chain containing no more than 15 amino acid substitutions, insertions, and/or deletions of a single amino acid per 100 amino acids relative to a known or naturally occurring immunoglobulin heavy chain amino acid sequence are encompassed within what is meant by an immunoglobulin heavy chain.

A **"immunoglobulin light chain,"** as meant herein, consists essentially of a VL and a CL. Close variants of an immunoglobulin light chain containing no more than 15 amino acid substitutions, insertions, and/or deletions of a single amino acid per 100 amino acids relative to a known or naturally occurring immunoglobulin light chain amino acid sequence are encompassed within what is meant by an immunoglobulin light chain.

An **"immunoglobulin variable region,"** as meant herein, is a VH region, a VL region, or a variant thereof. Close variants of an immunoglobulin variable region containing no more than 15 amino acid substitutions, insertions, and/or deletions of a single amino acid per 100 amino acids relative to a known or naturally occurring immunoglobulin variable region amino acid sequence are encompassed within what is meant by an immunoglobulin variable region. Many examples of VH and VL regions are known in the art, such as, for example, those disclosed by Kabat et al. in SEQUENCES OF IMMUNOLOGICAL INTEREST, Public Health Service N.I.H., Bethesda, MD, 1991. Based on the extensive sequence commonalities in the less variable portions of the VH and VL regions, the position within a sequence of more variable regions, and the predicted tertiary structure, one of skill in the art can recognize an immunoglobulin variable region by its sequence. *See, e.g.,* Honegger and Pluckthun (2001), J. Mol. Biol. 309: 657-670.

An immunoglobulin variable region contains three hypervariable regions, known as complementarity determining region 1 (CDR1), complementarity determining region 2 (CDR2), and complementarity determining region 3 (CDR3). These regions form the antigen binding site of an antibody. The CDRs are embedded within the less variable framework regions (FR1-FR4). The order of these subregions within an immunoglobulin variable region is as follows: FR1-CDR1-FR2-CDR2-FR3-CDR3-FR4. Numerous sequences of immunoglobulin variable regions are known in the art. *See, e.g.,* Kabat et al., SEQUENCES OF PROTEINS OF IMMUNOLOGICAL INTEREST, Public Health Service N.I.H., Bethesda, MD, 1991.

CDRs can be located in a VH region sequence in the following way. CDR1 starts at approximately residue 31 of the mature VH region and is usually about 5-7 amino acids long, and it is almost always preceded by a Cys-Xxx-Xxx-Xxx-Xxx-Xxx-Xxx-Xxx-Xxx (SEQ ID NO:30) (where "Xxx" is any amino acid). The residue following the heavy chain CDR1 is almost always a tryptophan, often a Trp-Val, a Trp-Ile, or a Trp-Ala. Fourteen amino acids are almost always between the last residue in CDR1 and the first in CDR2, and CDR2 typically contains 16 to 19 amino acids. CDR2 may be immediately preceded by Leu-Glu-Trp-Ile-Gly (SEQ ID NO:31) and may be immediately followed by Lys/Arg-Leu/Ile/Val/Phe/Thr/Ala-Thr/Ser/Ile/Ala. Other amino acids may precede or follow CDR2. Thirty two amino acids are almost always between the last residue in CDR2 and the first in CDR3, and CDR3 can be from about 3 to 25 residues long. A Cys-Xxx-Xxx almost always immediately precedes CDR3, and a Trp-Gly-Xxx-Gly (SEQ ID NO: 32) almost always follows CDR3.

Light chain CDRs can be located in a VL region in the following way. CDR1 starts at approximately residue 24 of the mature antibody and is usually about 10 to 17 residues long. It is almost always preceded by a Cys. There are almost always 15 amino acids between the last residue of CDR1 and the first residue of CDR2, and CDR2 is almost always 7 residues long. CDR2 is typically preceded by Ile-Tyr, Val-Tyr, Ile-Lys, or Ile-Phe. There are almost always 32 residues between CDR2 and CDR3, and CDR3 is usually about 7 to 10 amino acids long. CDR3 is almost always preceded by Cys and usually followed by Phe-Gly-Xxx-Gly (SEQ ID NO:33).

A **"linker,"** as meant herein, is a peptide that links two polypeptides, which can be two immunoglobulin variable regions in the context of a V-C-Fc-V-C antibody. A linker can be from 2-40 amino acids in length. In some embodiments, a linker can be about 2-35, 2-30, or 3-25 amino acids long. In some embodiments, a linker can be a peptide no more than about 35, 30, 25, 14, 13, 12, 11, 10, 9, 8, 7, 6, or 5 amino acids long. In other embodiments, a linker can be about 5-35, 5-30, 4-20, 10-20, or 20-35 amino acids long. In other embodiments, a linker can be at least about, 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acids long and/or no more than about 40, 35, 30, 25, 20, or 15 amino acids long. Exemplary linkers include, for example, the amino acid sequences TVAAP (SEQ ID NO:34), ASTKGP (SEQ ID NO:35),GGGGSGGGGS (SEQ ID NO:36), GGGGSAAA (SEQ ID NO:37), GGGGSGGGGSGGGGS (SEQ ID NO:38), $(GGGGS)_n$ (SEQ ID NO:50; where n is a number from 1 to 10), and AAA, among many others. In some embodiments linkers in different parts of the V-C-Fc-V-C molecule can have different sequences and/or different lengths. Different lengths of the linkers between the CH3 regions and the variable regions that follow them might allow these variable regions to be more accessible to allow better antigen binding. In some embodiments the linker between the CH3 region and the second segment containing a variable region plus a CL or CH1 region on one polypeptide chain of a V-C-Fc-V-C can contain an amino acid sequence that can be cleaved by a protease, for example, a furin protease, which can be present in a host cell, a metalloproteinase, for example, matrix metalloproteinase 2 (MMP2) or MMP9, a membrane anchored serine protease, which can be present of the surface of a target cell, or any other appropriate protease. Examples of such linkers include, without limitation, the following sequences: GGGGSRRRRRRGGGGS (SEQ ID NO:53), GGGGSGPLGIAGQGGGGS (SEQ ID NO:54), GGGGSGGGGSGLEVLFQGPSGGGGSGGGGS (SEQ ID NO:55), GGGGSGGGGSGQSSRHRRALGGGGSGGGGS (SEQ ID NO:56), GGGGSGGGGSGGPLGMLSQSGGGGSGGGGS (SEQ ID NO:57), GGGGSGGGGSGGGGRRGGSGGGGSGGGGS (SEQ ID NO:58), and AVRWLLTA (SEQ ID NO:59).

A V-C-Fc-V-C antibody **"mediates cytolysis of a target cell by an immune effector cell,"** as meant herein, when addition of an amount from 0.001 pM to 20000 pM of the V-C-Fc-V-C antibody to a cell cytolysis assay as described herein effectively elicits cytolysis of the target cells.

**"Non-chemotherapeutic anti-neoplastic agents"** are chemical agents, compounds, or molecules having cytotoxic or cytostatic effects on cancer cells other than chemotherapeutic agents. Non-chemotherapeutic antineoplastic agents may, however, be targeted to interact directly with molecules that indirectly affect cell division such as cell surface receptors, including receptors for hormones or growth factors. However, non-chemotherapeutic antineoplastic agents do not interfere directly with processes that are intimately linked to cell division such as, for example, DNA replication, RNA synthesis, protein synthesis, or mitotic spindle function, assembly, or disassembly. Examples of non-chemotherapeutic anti-neoplastic agents include inhibitors of Bcl2, inhibitors of farnesyltransferase, anti-estrogenic agents such as tamoxifen, anti-androgenic compounds, interferon, arsenic, retinoic acid, retinoic acid derivatives, antibodies targeted to tumor-specific antigens, and inhibitors of the Bcr-Abl tyrosine kinase (e.g., the small molecule STI-571 marketed under the trade name GLEEVEC™ by Novartis, New York and New Jersey, USA and Basel, Switzerland), among many possible non-chemotherapeutic anti-neoplastic agents.

A **"signal sequence,"** is a hydrophobic amino acid sequence that mediates insertion of a secreted or transmembrane protein through the membrane bounding the endoplasmic reticulum (ER) in a eukaryotic cell. "Signal sequences," as meant herein are amino-terminal hydrophobic sequences which are usually enzymatically removed following the insertion of part or all of the protein through the ER membrane into the lumen of the ER. Thus, it is known in the art that a signal sequence can be present as part of a precursor form of a secreted or transmembrane protein, but will generally be absent from the mature form of the protein. When a protein is said to comprise a signal sequence, it is to be understood that, although a precursor form of the protein does contain the signal sequence, a mature form of the protein will likely not contain the signal sequence. Signal sequences contain a residue adjacent to and immediately upstream from the cleavage site (position -1) and another residue at position -3, which are important for this enzymatic cleavage. Nielsen et al. (1997), Protein Eng. 10(1):1-6; von Heijne (1983), Eur. J. Biochem. 133:17-21; von Heijne (1985), J. Mol. Biol. 184:99-105, the portions of which describe signal sequences and how to identify them are incorporated herein by reference. Signal sequences can be identified as described by Nielsen et al. *(supra)*. Examples of signal peptides or sequences that are functional in mammalian host cells include the following: the signal sequence for interleukin-7 (IL-7) described in US Patent 4,965,195; the signal sequence for interleukin-2 receptor described in Cosman et al. ((1984), Nature 312:768); the interleukin-4 receptor signal peptide described in EP Patent 0 367 566; the type I interleukin-1 receptor signal sequence described in US Patent 4,968,607; the type II interleukin-1 receptor signal peptide described in EP Patent 0 460 846; the signal sequence of human IgK; and the signal sequence of human growth hormone. The relevant portions of these references are incorporated herein by reference. Many other signal sequences are known in the art. Many amino acid sequences described herein include signal sequences, for example, SEQ ID NOs:1, 3, 5, 7, 9, 11, 41, 44, 46, 48, 62, and 64.

A **"target cell"** is a cell that a V-C-Fc-V-C antibody, as described herein, binds to and that is involved in mediating a disease. In some cases, a target cell can be a cell that is ordinarily involved in mediating an immune response, but is also involved in the mediation of a disease. For example in B cell lymphoma, a B cell, which is ordinarily involved in mediating immune response, can be a target cell. In some embodiments, a target cell is a cancer cell, a cell infected with a pathogen, or a cell involved in mediating an autoimmune or inflammatory disease. The V-C-Fc-V-C antibody can bind to the target cell via binding to an antigen on a **"target cell molecule,"** which can be a **"target cell protein,"** which is a molecule that is displayed on the surface of the target cell, possibly a protein that may be highly expressed. Alternatively, a "target cell" of a V-C-Fc-V-C can be a pathogen, which can be, without limitation, a virus, a bacterium, a fungus, a protozoan, or a helminth.

**"Tumor burden"** refers to the number of viable cancer cells, the number of tumor sites, and/or the size of the tumor(s) in a patient suffering from a cancer. A reduction in tumor burden can be observed, for example, as a reduction in the amount of a tumor-associated antigen or protein in a patient's blood or urine, a reduction in the number of tumor cells or tumor sites, and/or a reduction in the size of one or more tumors.

A **"therapeutically effective amount"** of a V-C-Fc-V-C antibody as described herein is an amount that has the effect of, for example, reducing or eliminating the tumor burden of a cancer patient or reducing or eliminating the symptoms of any disease condition that the protein is used to treat. A therapeutically effective amount need not completely eliminate all symptoms of the condition, but may reduce severity of one or more symptoms or delay the onset of more serious symptoms or a more serious disease that can occur with some frequency following the treated condition.

**"Treatment"** of any disease mentioned herein encompasses an alleviation of at least one symptom of the disease, a reduction in the severity of the disease, or the delay or prevention of disease progression to more serious symptoms that may, in some cases, accompany the disease or lead to at least one other disease. Treatment need not mean that the disease is totally cured. A useful therapeutic agent needs only to reduce the severity of a disease, reduce the severity of one or more symptoms associated with the disease or its treatment, or delay the onset of more serious symptoms or a more serious disease that can occur with some frequency following the treated condition.

When it is said that a named VH/VL pair of immunoglobulin variable regions can bind to a target cell or an immune effector cell **"when they are part of an IgG antibody or scFv antibody,"** it is meant that an IgG antibody that contains the named VH region in both heavy chains and the named VL region in both light chains or the scFv that contains the VH/VL pair can bind to the target cell or the immune effector cell. A binding assay is described in Example 2. One of skill in the art could construct an IgG or scFv antibody containing the desired sequences given the knowledge in the art.

### *V-C-Fc-V-C Antibodies*

In the most general sense, a V-C-Fc-V-C antibody as described herein comprises two polypeptide chains having different amino acid sequences, which, together, can bind to two different antigens. Such a molecule can have a half life between a few hours and a few days or from a few days to one or more weeks. A diagram of a structure of a V-C-Fc-V-C antibody is shown in Figure 1. Optionally, linkers can occur between any of the regions diagrammed in Figure 1. The first polypeptide chain can comprise a first segment comprising a first variable region followed by a CH1 or CL region followed by an Fc polypeptide chain comprising part or all of a hinge region, a CH2 region, and a CH3 region, which can optionally be followed by a linker and then a second segment comprising a second variable region followed by a CH1 or CL region, which, optionally, can be followed by part or all of a hinge region. The second polypeptide chain of this embodiment can comprise a third segment comprising a third variable region followed by a CL or CH1 region followed by an Fc polypeptide chain comprising part or all of a hinge region, a CH2 region, and a CH3 region, which is followed by an optional linker and then a fourth segment comprising a fourth variable region followed by a CL or CH1 region, which, optionally, can be followed by part of all of a hinge region. If the first segment comprises a CH1 region, then the third segment can comprise a CL region. Similarly, if the first segment comprises a CL region, then the third segment can comprise a CH1 region. Also, if the second segment comprises a CH1 region, then the fourth segment can comprise a CL region. Similarly, if the second segment comprises a CL region, then the fourth segment can comprise a CH1 region. Also, if the first variable region is a VH region, then the third variable region can be a VL region, and if the first variable region is a VL region, then the third variable can be a VH region. Similarly, if the second variable region is a VH region, then the fourth variable region can be a VL region, and if the second variable region is a VL region, then the fourth variable region can be a VH region. Optionally, the VH/VL pairs containing the first and third variable regions and the second and fourth variable regions can each bind to a target cell, which may be a pathogen including a virus, or an immune effector cell when they are part of an IgG antibody or scFv antibody. In some embodiments, the V-C-Fc-V-C antibody can bind to an immune effector cell and a target cell.

In one embodiment shown in Figure 2, the first polypeptide chain (at left of panel A, B, C, or D in Figure 2) can comprise a first segment comprising a first VH-1 region followed by a CH1-1 region followed by an Fc polypeptide chain comprising part or all of a hinge region, a CH2-1 region, and a CH3-1 region, which is followed by a linker and then a second segment comprising a VH-2 region followed by a CH1-2 region and, optionally, part or all of a hinge region. The second polypeptide chain (at right of panel A, B, C, or D in Figure 2) of this embodiment can comprise a third segment comprising a VL-1 region and a CL-1 region followed by an Fc polypeptide chain comprising part or all of a hinge region, a CH2-2 region, and a CH3-2 region, which is followed by a linker and then a fourth segment comprising a second VL-2 region followed by a CL-2 region and, optionally, part or all of a hinge region. In these designations, the numbers following the dash, i.e., "-1" or "-2", indicate that the indicated domains are distinct domains that can have different amino acids sequence. For example VH-1 and VH-2 are both heavy chain variable regions, but they are in distinct positions within the V-C-Fc-V-C antibody and can have different amino acid sequences. Similar considerations apply for VL-1, VL-2, CH1-1, CH1-2, etc.

In another embodiment, the first polypeptide chain can comprise a first segment comprising a VH-1 region and a CH1-1 region followed by an Fc polypeptide chain comprising part or all of a hinge region, a CH2-1 region, and a CH3-1 region, which is followed by a linker and then a second segment comprising a VL-2 region followed by a CL-2 region and, optionally, part or all of a hinge region.. The second polypeptide chain of this embodiment can comprise a third segment comprising a VL-1 region and a CL-1 region followed by an Fc polypeptide chain comprising part or all of a hinge region, a CH2-2 region, and a CH3-2 region, which is followed by a linker and then a fourth segment comprising a VH-2 region followed by a CH1-2 region and, optionally, part or all of a hinge region.

In a further embodiment, the first polypeptide chain can comprise a first segment comprising a VL-1 region and a CH1-1 region followed by an Fc polypeptide chain comprising part or all of a hinge region, a CH2-1 region, and a CH3-1 region, which is followed by a linker and then a second segment comprising a VH-2 region followed by a CH1-2 region and, optionally, part or all of a hinge region.. The second polypeptide chain of this embodiment can comprise a third segment comprising a VH-1 region and a CL-1 region followed by an Fc polypeptide chain comprising part or all of a hinge region, a CH2-2 region, and a CH3-2 region, which is followed by a linker and then a fourth segment comprising a VL-2 region followed by a CL-2 region and, optionally, part or all of a hinge region.

In a further embodiment, the first polypeptide chain can comprise a first segment comprising a VL-1 region and a CH1-1 region followed by an Fc polypeptide chain comprising part or all of a hinge region, a CH2-1 region, and a CH3-1 region, which is followed by a linker and then a second segment comprising a VL-2 region followed by a CH1-2 region and, optionally, part or all of a hinge region.. The second polypeptide chain of this embodiment can comprise a third segment comprising a VH-1 region and a CL-1 region followed by an Fc polypeptide chain comprising part or all of a hinge region, a CH2-2 region, and a CH3-2 region, which is followed by a linker and then a fourth segment comprising a VH-2 region followed by a CL-2 region and, optionally, part or all of a hinge region.

In still another embodiment, the first polypeptide chain can comprise a first segment comprising a VH-1 region and a CH1-1 region followed by an Fc polypeptide chain comprising part or all of a hinge region, a CH2-1 region, and a CH3-1 region, which is followed by a linker and then a second segment comprising a VL-2 region followed by a CH1-2 region and, optionally, part or all of a hinge region.. The second polypeptide chain of this embodiment can comprise a third segment comprising a VL-1 region and a CL-1 region followed by an Fc polypeptide chain comprising part or all of a hinge region, a CH2-2 region, and a CH3-2 region, which is followed by a linker and then a fourth segment comprising a VH-2 region followed by a CL-2 region and, optionally, part or all of a hinge region.

In any of the embodiments described above, the identities of the first polypeptide chain and the second polypeptide chain can be switched.

The VH-1/VL-1 and VH-2/VL-2 pairs of immunoglobulin variable regions can each bind to a target cell, including a pathogen, or an immune effector cell when they are part of an IgG antibody or scFv antibody. The V-C-Fc-V-C antibody can bind to an immune effector cell and a target cell.

An optional linker between an Fc polypeptide chain and the second or fourth segment, i.e., between the CH3-1 or CH3-2 region and the VH-2 or VL-2 region, can be present or absent and can have the same or a different amino acid sequence in the two polypeptide chains of a V-C-Fc-V-C antibody. These linkers can play a role in the structure and function of the antibody. If the linker is short, i.e., less than 12 amino acids long, it will limit flexibility. If the linker is at least 15 amino acids long, it will allow much more flexibility. In some embodiments, these linkers on the two polypeptide chains of the V-C-Fc-V-C can have different lengths and/or sequences, which can make the VL-2/VH-2 more accessible for binding.

Further, optional linkers can occur between any of the domains in a V-C-Fc-V-C.

In addition, a linker downstream from an Fc polypeptide chain in one, but not both, polypeptide chains can comprise an amino acid sequence cleavable by a protease. In embodiments where such a linker is cleaved, the segment downstream from the Fc region can be more accessible and can exhibit higher affinity for the molecule it binds to than it would if the linker were not cleaved. This can correlate with higher activity in a binding and/or a cell killing assay as described in the Examples below. The linker can be cleavable by any protease, for example, a furin or a matrix metalloproteinase. If the protease is a furin, it may be present in a host cell used to produce the V-C-Fc-V-C, and the V-C-Fc-V-C may be secreted from the host cell in a cleaved form. Alternatively, protease digestion can occur after the V-C-Fc-V-C has been produced or purified and before it is administered to a patient. As a further alternative, a V-C-Fc-V-C can contain a protease site that is cleaved in *vivo* following administration to a patient, for example by a metalloproteinase secreted by a cancer cell. Such a V-C-Fc-V-C has the advantage of being fully active only in the presence of appropriate target cells. Exemplary sequences of linkers containing protease cleavage sites are provided in SEQ ID NOs: 51-59 and 71-101.

A V-C-Fc-V-C antibody as described herein can contain an additional, or alternative, half-life extending moiety besides the Fc region. This moiety could be, for example, be albumin, an albumin fragment, a moiety that binds to albumin or to the neonatal Fc receptor (FcRn), a derivative of fibronectin that has been engineered to bind albumin (e.g., having the amino acid sequence of SEQ ID NO:25) or a fragment thereof, a peptide, a single domain protein fragment, or other polypeptide that can increase serum half life. In alternate embodiments, a half life-extending moiety can be a non-polypeptide molecule such as, for example, polyethylene glycol (PEG).

Sequences of human IgG1, IgG2, IgG3, and IgG4 Fc polypeptides that could be used in a V-C-Fc-V-C antibody are provided in SEQ ID NOs:26-29. Variants of these sequences containing one or more heterodimerizing alterations, one or more Fc alteration that extends half life, one or more alteration that enhances ADCC, and/or one or more alteration that inhibits Fc gamma receptor (Fc$\gamma$R) binding are also contemplated, as are other close variants containing not more than 15, 10, 8, 5, or 3 deletions, insertions, or substitutions of a single amino acid per 100 amino acids of sequence.

As explained above, an Fc polypeptide chain comprises all or part of a hinge region followed by a CH2 and a CH3 region. The Fc polypeptide chain can be of mammalian (for example, human, mouse, rat, rabbit, dromedary, or new or old world monkey), avian, or shark origin. In addition, as explained above, an Fc polypeptide chain can have a limited number alterations. For example, an Fc polypeptide chain can comprise one or more heterodimerizing alterations, one or more alteration(s) that inhibit(s) binding to Fc $\gamma$R, or one or more alteration(s) that increase(s) binding to FcRn. Exemplary amino acid sequences of pairs of polypeptide chains that make up a V-C-Fc-V-C antibody containing an Fc region include, without limitation, the following pairs of sequences: SEQ ID NOs: 1 and 3; SEQ ID NOs: 5 and 7; SEQ ID NOs: 9 and 11, SEQ ID NOs: 46 and 48, SEQ ID NOs: 11 and 62, and SEQ ID NOs: 11 and 64.

In some embodiments the amino acid sequences of the Fc polypeptide chains can be mammalian, for example human amino acid sequences. The isotype of the Fc polypeptide can be IgG, such as IgG1, IgG2, IgG3, or IgG4, IgA, IgD, IgE, or IgM. Table 2 below shows an alignment of the amino acid sequences of human IgG1, IgG2, IgG3, and IgG4 sequences.

Table 2: Amino acid sequences of human IgG Fc polypeptide chains

| | |
|---|---|
| IgG1 | ---------------------------------------------- |
| IgG2 | ---------------------------------------------- |
| IgG3 | ELKTPLGDTTHTCPRCPEPKSCDTPPPCPRCPEPKSCDTPPPCPRCP |
| IgG4 | ---------------------------------------------- |

(continued)

| | |
|---|---|
| IgG1 | 225 * 235 * 245 * 255 * 265 * 275 * |
| | **EPKSCDKTHTCPPCPAPELLGGPSVFLFPPKPKDTLMISRTPEVTCVVVDVSHEDPEVKF** |
| IgG2 | ERKCCVE---CPPCPAPPVA-GPSVFLFPPKPKDTLMISRTPEVTCVWDVSHEDPEVQF |
| IgG3 | EPKSCDTPPPCPRCPAPELLGGPSVFLFPPKPKDTLMISRTPEVTCVVVDVSHEDPEVQF |
| IgG4 | ESKYG---PPCPSCPAPEFLGGPSVFLFPPKPKDTLMISRTPEVTCVVVDVSQEDPEVQF |
| IgG1 | 285 * 295 * 305 * 315 * 325 * 335 * |
| | **NWYVDGVEVHNAKTKPREEQYNSTYRVVSVLTVLHQDWLNGKEYKCKVSNKALPAPIEKT** |
| IgG2 | NWYVDGMEVHNAKTKPREEQFNSTFRVVSVLTVVHQDWLNGKEYKCKVSNKGLPAPIEKT |
| IgG3 | KWYVDGVEVHNAKTKPREEQYNSTFRVVSVLTVLHQDWLNGKEYKCKVSNKALPAPIEKT |
| IgG4 | NWYVDGVEVHNAKTKPREEQFNSTYRVVSVLTVLHQDWLNGKEYKCKVSNKGLPSSIEKT |
| IgG1 | 345 * 355 * 365 * 375 * 385 * 395 * |
| | **ISKAKGQPREPQVYTLPPSREEMTKNQVSLTCLVKGFYPSDIAVEWESNGQPENNYKTTP** |
| IgG2 | ISKTKGQPREPQVYTLPPSREEMTKNQVSLTCLVKGFYPSDIAVEWESNGQPENNYKTTP |
| IgG3 | ISKTKGQPREPQVYTLPPSREEMTKNQVSLTCLVKGFYPSDIAVEWESSGQPENNYNTTP |
| IgG4 | ISKAKGQPREPQVYTLPPSQEEMTKNQVSLTCLVKGFYPSDIAVEWESNGQPENNYKTTP |
| IgG1 | 405 * 415 * 425 * 435 * 445 * |
| | **PVLDSDGSFFLYSKLTVDKSRWQQGNVFSCSVMHEALHNHYTQKSLSLSPGK (SEQ ID NO:26)** |
| IgG2 | PMLDSDGSFFLYSKLTVDKSRWQQGNVFSCSVMHEALHNHYTQKSLSLSPGK (SEQ ID NO:27) |
| IgG3 | PMLDSDGSFFLYSKLTVDKSRWQQGNIFSCSVMHEALHNRFTQKSLSLSPGK (SEQ ID NO:28) |
| IgG4 | PVLDSDGSFFLYSRLTVDKSRWQEGNVFSCSVMHEALHNHYTQKSLSLSLGK (SEQ ID NO:29) |

The numbering shown in Table 2 is according the EU system of numbering, which is based on the sequential numbering of the constant region of an IgG1 antibody. Edelman et al. (1969), Proc. Natl. Acad. Sci. 63: 78-85. Thus, it does not accommodate the additional length of the IgG3 hinge well. It is nonetheless used here to designate positions in an Fc polypeptide chain because it is still commonly used in the art to refer to positions in Fc polypeptide chains. The hinge regions of the IgG1, IgG2, and IgG4 Fc polypeptides extend from about position 216 to about 230. It is clear from the alignment that the IgG2 and IgG4 hinge regions are each three amino acids shorter than the IgG1 hinge. The IgG3 hinge is much longer, extending for an additional 47 amino acids upstream. The CH2 region extends from about position 231 to 340, and the CH3 region extends from about position 341 to 447.

Naturally occurring amino acid sequences of Fc polypeptide chains can be varied slightly. Such variations can include no more than 15, 10, 8, 5, or 3 insertions, deletions, or substitutions of a single amino acid per 100 amino acids of sequence of a naturally occurring Fc polypeptide chain. If there are substitutions, they can be conservative amino acid substitutions, as defined above. The Fc polypeptide chains within the first and second polypeptide chains of a V-C-Fc-V-C antibody can differ in amino acid sequence. In some embodiments, they can include "heterodimerizing alterations," for example, charge pair substitutions, as defined above, that facilitate heterodimer formation. Further, the Fc polypeptide chain portions of a V-C-Fc-V-C antibody can also contain alterations that inhibit Fc γR binding. Such mutations are described above and in Xu et al. (2000), Cell. Immunol. 200(1): 16-26, the relevant portions of which are incorporated herein by reference. The Fc polypeptide chain portions can also include an "Fc alteration that extends half life," as described above, including those described in, *e.g.*, US Patents 7,037,784, 7,670,600, and 7,371,827, US Patent Application Publication 2010/0234575, and International Application PCT/US2012/070146, the relevant portions of all of which are incorporated herein by reference. Further, an Fc polypeptide can comprise "alterations that enhance ADCC," as defined above.

A V-C-Fc-V-C antibody as described herein can bind to an immune effector cell through an antigen that can be part of an effector cell molecule and can bind to a target cell through an antigen that can be part of a target cell molecule or protein. Some effector cell molecules, which can be effector cell proteins, are described below. Similarly, possible target cell molecules, which can be target cell proteins, are also described below. A V-C-Fc-V-C antibody can bind to any combination of an effector cell molecule and a target cell molecule, which can be engaged noncovalently by the V-C-Fc-V-C antibody. Alternatively, a V-C-Fc-V-C antibody may bind to an antigen displayed on target and/or effector cells that is not part of a protein.

*Nucleic Acids Encoding V-C-Fc-V-C Antibodies*

Provided are nucleic acids encoding the V-C-Fc-V-C antibodies described herein. Numerous nucleic acid sequences encoding immunoglobulin regions including VH, VL, hinge, CH1, CH2, CH3, and CH4 regions are known in the art. *See, e.g.,* Kabat et al. in SEQUENCES OF IMMUNOLOGICAL INTEREST, Public Health Service N.I.H., Bethesda, MD, 1991. Using the guidance provided herein, one of skill in the art could combine such nucleic acid sequences and/or other nucleic acid sequences known in the art to create nucleic acid sequences encoding the V-C-Fc-V-C antibodies described herein. Exemplary pairs of nucleic acids encoding V-C-Fc-V-C antibodies include the following: SEQ ID NOs:2 and 4; SEQ ID NOs:6 and 8; SEQ ID NOs:10 and 12; SEQ ID NOs:47 and 49, SEQ ID NOs:12 and 63, and SEQ ID NOs:12 and 65.

In addition, nucleic acid sequences encoding V-C-Fc-V-C antibodies described herein can be determined by one of skill in the art based on the amino acid sequences provided herein and knowledge in the art. Numerous sequences of immunoglobulin regions are known in the art and can be amplified and joined together using polymerase chain reaction. Besides more traditional methods of producing cloned DNA segments encoding a particular amino acid sequence, companies such as DNA 2.0 (Menlo Park, CA, USA) and Blue Heron (Bothell, WA, USA), among others, now routinely produce chemically synthesized, gene-sized DNAs of any desired sequence to order, thus streamlining the process of producing such DNAs.

***Methods of Making V-C-Fc-V-C Antibodies***

The V-C-Fc-V-C antibodies described herein can be made using methods well known in the art. For example, nucleic acids encoding the two polypeptide chains of a V-C-Fc-V-C antibody can be introduced into a cultured host cell by a variety of known methods, such as, for example, transformation, transfection, electroporation, bombardment with nucleic acid-coated microprojectiles, etc. In some embodiments, the nucleic acids encoding the V-C-Fc-V-C antibodies can be inserted into a vector appropriate for expression in the host cells before being introduced into the host cells. Typically such vectors can contain sequence elements enabling expression of the inserted nucleic acids at the RNA and protein levels. Such vectors are well known in the art, and many are commercially available. The host cells containing the nucleic acids can be cultured under conditions so as to enable the cells to express the nucleic acids, and the resulting V-C-Fc-V-C antibodies can be collected from the cell mass or the culture medium. Alternatively, the V-C-Fc-V-C antibodies can be produced in *vivo,* for example in plant leaves (*see*, *e.g.,* Scheller et al. (2001), Nature Biotechnol. 19: 573-577 and references cited therein), bird eggs (see, *e.g.,* Zhu et al. (2005), Nature Biotechnol. 23: 1159-1169 and references cited therein), or mammalian milk (see, *e.g.,* Laible et al. (2012), Reprod. Fertil. Dev. 25(1): 315).

A variety of cultured host cells can be used including, for example, bacterial cells such as *Escherichia coli or Bacilis steorothermophilus,* fungal cells such as *Saccharomyces cerevisiae* or *Pichia pastoris,* insect cells such as lepidopteran insect cells including *Spodoptera frugiperda* cells, or mammalian cells such as Chinese hamster ovary (CHO) cells, baby hamster kidney (BHK) cells, monkey kidney cells, HeLa cells, human hepatocellular carcinoma cells, or human embryonic kidney (HEK) 293 cells, among many others.

***Immune Effector Cells and Effector Cell Molecules***

A V-C-Fc-V-C antibody as described herein can bind to a molecule expressed on the surface of an immune effector cell (called **"effector cell molecule"** herein) and to another molecule expressed on the surface of a target cell (called a **"target cell molecule"** herein). Hence, a V-C-Fc-V-C can bind to a target cell and an effector cell, as shown in the Examples below. The immune effector cell can be, for example, a T cell, an NK cell, a macrophage, or a neutrophil. In some embodiments the effector cell molecule is a protein included in the T cell receptor (TCR)-CD3 complex. The TCR-CD3 complex is a heteromultimer comprising a heterodimer comprising TCR$\alpha$ and TCR$\beta$ or TCR$\gamma$ and TCR$\delta$ plus various CD3 chains from among the CD3 zeta (CD3$\zeta$) chain, CD3 epsilon (CD3$\varepsilon$) chain, CD3 gamma (CD3$\gamma$) chain, and CD3 delta (CD3$\delta$) chain. In some embodiments, a V-C-Fc-V-C antibody binds to a human and/or cynomolgus monkey CD3$\varepsilon$ chain (the mature amino acid sequence of which is disclosed in SEQ ID NO:39), which may be part of a multimeric protein. Alternatively, an effector cell protein can be human and/or cynomolgus monkey TCR$\alpha$, TCR$\beta$, TCR$\delta$, TCR$\gamma$, CD3 beta (CD3$\beta$) chain, CD3$\gamma$ chain, CD3$\delta$ chain, or CD3$\zeta$ chain.

Moreover, in some embodiments, the V-C-Fc-V-C antibody can also bind to a CD3$\varepsilon$ chain from another species, such as mouse, rat, rabbit, new world monkey, and/or old world monkey species. Such species include, without limitation, the following mammalian species: *Mus musculus; Rattus rattus; Rattus norvegicus;* the cynomolgus monkey, Macaca *fascicularis;* the hamadryas baboon, *Papio hamadryas;* the Guinea baboon, *Papio papio;* the olive baboon, *Papio anubis;* the yellow baboon, *Papio cynocephalus;* the Chacma baboon, *Papio ursinus; Callithrix jacchus; Saguinus Oedipus;* and *Saimiri sciureus.* The mature amino acid sequence of the CD3$\varepsilon$ chain of cynomolgus monkey is provided in SEQ ID NO:40. As is known in the art of development of protein therapeutics, having a therapeutic that can have comparable activity in humans and species commonly used for preclinical testing, such as mice and monkeys, can simplify and speed drug development. In the long and expensive process of bringing a drug to market, such advantages can be critical.

In more particular embodiments, the V-C-Fc-V-C antibody can bind to an epitope within the first 27 amino acids of a CD3ε chain, which may be a human CD3ε chain and/or a CD3ε chain from different species, optionally one of the mammalian species listed above. The epitope that the antibody binds to can be part of an amino acid sequence selected from the group consisting of SEQ ID NO:39 and SEQ ID NO:40. The epitope can contain the amino acid sequence Gln-Asp-Gly-Asn-Glu (SEQ ID NO:41). The advantages of an antibody that binds such an epitope are explained in detail in U.S. Patent Application Publication 2010/0183615, the relevant portions of which are incorporated herein by reference. The epitope to which an antibody binds can be determined by alanine scanning, which is described in, e.g., U.S. Patent Application Publication 2010/0150918, the relevant portions of which are incorporated herein by reference.

Where a T cell is the immune effector cell, effector cell molecules, which can be effector cell proteins, to which a V-C-Fc-V-C antibody can bind include proteins that are part of a TCR-CD3 complex including, without limitation, the CD3α chain, the CD3β chain, the CD3y, the CD3δ chain, the CD3ε chain, the CD3ζ chain, the CD3η chain, TCRα, TCRβ, TCRγ, and TCRδ. Where an NK cell or a cytotoxic T cell is an immune effector cell, NKG2D. CD352, NKp46, or CD16a can be an effector cell protein. Where a CD8+ T cell is an immune effector cell, 4-1BB can be an effector cell protein. Alternatively, a V-C-Fc-V-C antibody could bind to other effector cell proteins expressed on T cells, NK cells, macrophages, or neutrophils.

***Target Cells and Target Cell Molecules***

As explained above, a V-C-Fc-V-C antibody as described herein can bind to an effector cell and a target cell or a pathogen via an effector cell molecule and a target cell molecule, respectively. In some embodiments, the effector cell molecule and the target cell molecule can be proteins expressed on the surface of these cells, i.e., "effector cell proteins" and "target cell proteins." The target cell molecule can, for example, be a protein or a carbohydrate (which can be attached to a protein or another kind of molecule) expressed on the surface of a cancer cell, a cell infected with a pathogen, or a cell that mediates an inflammatory or autoimmune condition. In some embodiments, the target cell molecule can be highly expressed on the target cell, although this is not required.

Where the target cell is a cancer cell, a V-C-Fc-V-C antibody as described herein can bind to a cancer cell antigen as described above. A cancer cell antigen can be a molecule displayed on a cancer cell such as a human protein or a protein from another species. For example, a V-C-Fc-V-C antibody may bind to a target cell protein from a mouse, rat, rabbit, new world monkey, and/or old world monkey species, among many others. Such species include, without limitation, the following species: *Mus musculus; Rattus rattus; Rattus norvegicus;* cynomolgus monkey, Macaca *fascicularis;* the hamadryas baboon, *Papio hamadryas;* the Guinea baboon, *Papio papio;* the olive baboon, *Papio anubis; the yellow baboon, Papio cynocephalus;* the Chacma baboon, *Papio ursinus, Callithrix jacchus, Saguinus oedipus,* and *Saimiri sciureus.*

In some examples, the target cell protein or molecule can be selectively expressed on an infected cell, such as, for example, a cell infected by a virus, a bacterium, or a eukaryotic pathogen. For example, in the case of an hepatitis B virus (HBV) or hepatitis C virus (HCV) infection, the target cell protein can be an envelope protein of HBV or HCV that is expressed on the surface of an infected cell. In other embodiments, the target cell protein can be gp120 encoded by human immunodeficiency virus (HIV) on HIV-infected cells. Alternatively, in some embodiments a V-C-Fc-V-C can bind directly to a pathogen.

In other aspects, a target cell can be a cell that mediates an autoimmune or inflammatory disease. For example, human eosinophils in asthma can be target cells, in which case, EGF-like module containing mucin-like hormone receptor (EMR1), for example, can be a target cell protein. Alternatively, excess human B cells in a systemic lupus erythematosus patient can be target cells, in which case CD19 or CD20, for example, can be a target cell protein. In autoimmune conditions, excess human Th2 T cells can be target cells, in which case CCR4 can, for example, be a target cell protein. Similarly, a target cell can be a fibrotic cell that mediates a fibrotic disease such as, for example, atherosclerosis, chronic obstructive pulmonary disease (COPD), cirrhosis, scleroderma, kidney transplant fibrosis, kidney allograft nephropathy, or a pulmonary fibrosis, including idiopathic pulmonary fibrosis and/or idiotypic pulmonary hypertension. For such fibrotic conditions, fibroblast activation protein alpha (FAP alpha) can, for example, be a target cell protein. Senescent cells can also be target cells, for example in conditions such as cancer, fibrotic disorders such as idiopathic pulmonary fibrosis, and age-related disorders, *e.g.*, Alzheimer's disease, lordokyphosis, sarcopenia, cataracts, fat loss, and dermal thinning.

***Target Cell Cytolysis Assays***

In Example 2 below, an assay for determining whether a V-C-Fc-V-C antibody as described herein can induce cytolysis of a target cell by an immune effector cell *in vitro* is described. In this assay, the immune effector cell is a T cell. The following very similar assay can be used where the immune effector cells are NK cells.

A target cell line expressing a target cell protein of interest can be labeled with 2 μM CFSE for 15 minutes at 37 °C and then washed. An appropriate number of labeled target cells can then be incubated in one or more 96 well culture plates for 40 minutes at 4 °C, with or without a bispecific protein, a control protein, or no added protein at varying concentrations. NK cells isolated from healthy human donors can be isolated using the Miltenyi NK Cell Isolation Kit II (Miltenyi Biotec, Auburn, CA) and then added to the target cells at an Effector:Target ratio of 10:1, a ratio that can be adjusted in some cases. The NK cells, which are the immune effector cells in this assay, can be used immediately post-isolation or after overnight culture at 37°C. Plates containing tumor target cells, bispecific proteins, and immune effector cells can be cultured for 18-24 hours at 37°C with 5% $CO_2$. Appropriate control wells can also be set up. After the 18-24 hour assay period, all cells can be removed from the wells. A volume of a 7-amino-actinomycin D (7-AAD) solution equal to the volume of the content of the wells can be added to each sample. Samples can then assayed to determine the percentage of live versus dead target cells via flow cytometry as described in the Examples below.

***Therapeutic Methods and Compositions***

The V-C-Fc-V-C antibodies described herein can be used to treat a wide variety of conditions including, for example, various forms of cancer, infections, fibrotic diseases, and/or autoimmune or inflammatory conditions.

Provided herein are pharmaceutical compositions comprising the V-C-Fc-V-C antibodies described herein. Such pharmaceutical compositions comprise a therapeutically effective amount of a V-C-Fc-V-C antibody, as described herein, plus one or more additional components such as a physiologically acceptable carrier, excipient, or diluent. Such additional components can include buffers, carbohydrates, polyols, amino acids, chelating agents, stabilizers, and/or preservatives, among many possibilities.

In some embodiments, the V-C-Fc-V-C antibodies described herein can be used to treat cell proliferative diseases, including cancer, which involve the unregulated and/or inappropriate proliferation of cells, sometimes accompanied by destruction of adjacent tissue and growth of new blood vessels, which can allow invasion of cancer cells into new areas, i.e. metastasis. These conditions include hematologic malignancies and solid tumor malignancies. Included within conditions treatable with the V-C-Fc-V-C antibodies described herein are non-malignant conditions that involve inappropriate cell growth, including colorectal polyps, cerebral ischemia, gross cystic disease, polycystic kidney disease, benign prostatic hyperplasia, and endometriosis. Other cell proliferative diseases that can be treated using the V-C-Fc-V-C antibodies of the present invention are, for example, cancers including mesotheliomas, squamous cell carcinomas, basel cell carcinomas, myelomas, osteosarcomas, glioblastomas, gliomas, carcinomas, adenocarcinomas, melanomas, sarcomas, acute and chronic leukemias, lymphomas, and meningiomas, Hodgkin's disease, Sezary syndrome, multiple myeloma, and lung, non-small cell lung, small cell lung, laryngeal, breast, head and neck, bladder, ovarian, skin, prostate, cervical, vaginal, gastric, renal cell, kidney, pancreatic, colorectal, endometrial, and esophageal, hepatobiliary, bone, skin, and hematologic cancers, as well as cancers of the nasal cavity and paranasal sinuses, the nasopharynx, the oral cavity, the oropharynx, the larynx, the hypolarynx, the salivary glands, the mediastinum, the stomach, the small intestine, the colon, the rectum and anal region, the ureter, the urethra, the penis, the testis, the vulva, the endocrine system, the central nervous system, and plasma cells.

Among the texts providing guidance for cancer therapy is Cancer, Principles and Practice of Oncology, 4th Edition, DeVita et al., Eds. J. B. Lippincott Co., Philadelphia, PA (1993). An appropriate therapeutic approach is chosen according to the particular type of cancer, and other factors such as the general condition of the patient, as is recognized in the pertinent field. The V-C-Fc-V-C antibodies described herein may be added to a therapy regimen using other anti-neoplastic agents in treating a cancer patient.

In some embodiments, the V-C-Fc-V-C antibodies can be administered concurrently with, before, or after a variety of drugs and treatments widely employed in cancer treatment such as, for example, chemotherapeutic agents, non-chemotherapeutic, anti-neoplastic agents, and/or radiation. For example, chemotherapy and/or radiation can occur before, during, concurrently with, and/or after any of the treatments described herein. Examples of chemotherapeutic agents are discussed above and include, but are not limited to, cisplatin, taxol, etoposide, mitoxantrone (Novantrone®), actinomycin D, cycloheximide, camptothecin (or water soluble derivatives thereof), methotrexate, mitomycin (e.g., mitomycin C), dacarbazine (DTIC), anti-neoplastic antibiotics such as adriamycin (doxorubicin) and daunomycin, and all the chemotherapeutic agents mentioned above.

The V-C-Fc-V-C antibodies described herein can also be used to treat infectious disease, for example a chronic hepatis B virus (HBV) infection, a hepatis C virus (HPC) infection, a human immunodeficiency virus (HIV) infection, an Epstein-Barr virus (EBV) infection, or a cytomegalovirus (CMV) infection, among many others. In such methods, the V-C-Fc-V-C antibodies can be used before, after, or concurrently with other treatments for these conditions.

The V-C-Fc-V-C antibodies described herein can find further use in other kinds of conditions where it is beneficial to deplete certain cell types. For example, depletion of human eosinophils in asthma, excess human B cells in systemic lupus erythematosus, excess human Th2 T cells in autoimmune conditions, or pathogen-infected cells in infectious diseases can be beneficial. Depletion of myofibroblasts or other pathological cells in fibrotic conditions such as lung fibrosis, such as idiopathic pulmonary fibrosis (IPF), or kidney or liver fibrosis is a further use of a V-C-Fc-V-C antibody.

Therapeutically effective doses of the V-C-Fc-V-C antibodies described herein can be administered. The amount of antibody that constitutes a therapeutically dose may vary with the indication treated, the weight of the patient, the calculated skin surface area of the patient. Dosing of the V-C-Fc-V-C antibodies described herein can be adjusted to achieve the desired effects. In many cases, repeated dosing may be required. For example, a V-C-Fc-V-C antibody as described herein can be dosed twice per week, once per week, once every two, three, four, five, six, seven, eight, nine, or ten weeks, or once every two, three, four, five, or six months. The amount of the V-C-Fc-V-C antibody administered on each day can be from about 0.0036 mg to about 450 mg or about 1000 mg. Alternatively, the dose can calibrated according to the estimated skin surface of a patient, and each dose can be from about 0.002 $mg/m^2$ to about 250 $mg/m^2$ or about 500 $mg/m^2$. In another alternative, the dose can be calibrated according to a patient's weight, and each dose can be from about 0. 000051 mg/kg to about 6.4 mg/kg or about 20 mg/kg.

The V-C-Fc-V-C antibodies, or pharmaceutical compositions containing these molecules, can be administered by any feasible method. Protein therapeutics will ordinarily be administered by parenteral route, for example by injection, since oral administration, in the absence of some special formulation or circumstance, would lead to hydrolysis of the protein in the acid environment of the stomach. Subcutaneous, intramuscular, intravenous, intraarterial, intralesional, or peritoneal injection are possible routes of administration. A V-C-Fc-V-C antibody can also be administered via infusion, for example intravenous or subcutaneous infusion. Topical administration is also possible, especially for diseases involving the skin. Alternatively, a V-C-Fc-V-C antibody can be administered through contact with a mucus membrane, for example by intra-nasal, sublingual, vaginal, or rectal administration or administration as an inhalant. Alternatively, certain appropriate pharmaceutical compositions comprising a V-C-Fc-V-C antibody can be administered orally.

Having described the invention in general terms above, the following examples are offered by way of illustration and not limitation.

The present invention further relates to the following items:

1. An antibody comprising two polypeptide chains,

   the first polypeptide chain comprising the following regions, which may or may not be separated by linkers, in the following order in an N- to C-terminal direction:
   V-1---C-1---hinge---CH2-1---CH3-1---V-2---C-2; and
   the second polypeptide chain comprising the following regions, which may or may not be separated by linkers, in the following order in an N- to C-terminal direction:
   V-3---C-3---hinge---CH2-2---CH3-2---V-4---C-4;
   wherein the V-1 to V-4 are immunoglobulin variable regions;
   wherein the C-1 to C-4 are are either a heavy chain constant region 1 (CH1) or a light chain constant regions (CL), and if C-1 is a CH1, then C-3 is a CL and vice versa, and if C-2 is a CH1, then C-4 is a CL and vice versa; and wherein the CH2-1 and CH2-2 are each a heavy chain constant region 2 and the CH3-1 and CH3-2 are each a heavy chain constant region 3.

2. The antibody of item 1, wherein the V-1 is a heavy chain variable region (VH), the C-1 is a CH1, the V-2 is a VH, the C-2 is a CH1, the V-3 is a light chain variable region (VL), the C-3 is a CL, the V-4 is a VL, and the C-4 is a CL.
3. The antibody of item 1, wherein the V-1 is a VH, the C-1 is a CH1, the V-2 is a VL, the C-2 is a CL, the V-3 is a VL, the C-3 is a CL, the V-4 is a VH, and the C-4 is a CH1.
4. The antibody of item 1, wherein the V-1 is a VH, the C-1 is a CH1, the V-2 is a VH, the C-2 is a CL, the V-3 is a VL, the C-3 is a CL, the V-4 is a VL, and the C-4 is a CH1.
5. The antibody of item 1, wherein the V-1 is a VH, the C-1 is a CL, the V-2 is a VH, the C-2 is a CH1, the V-3 is a VL, the C-3 is a CH1, the V-4 is a VL, and the C-4 is a CL.
6. The antibody of item 1, wherein the V-1 is a VL, the C-1 is a CH1, the V-2 is a VH, the C-2 is a CH1, the V-3 is a VH, the C-3 is a CL, the V-4 is a VL, and the C-4 is a CL.
7. The antibody of item 1, wherein the V-1 is a VL, the C-1 is a CH1, the V-2 is a VL, the C-2 is a CH1, the V-3 is a VH, the C-3 is a CL, the V-4 is a VH, and the C-4 is a CL.
8. The antibody of item 1, wherein the V-1 is a VH, the C-1 is a CH1, the V-2 is a VL, the C-2 is a CH1, the V-3 is a VL, the C-3 is a CL, the V-4 is a VH, and the C-4 is a CL.
9. The antibody of item 1, wherein the V-1 is a VL, the C-1 is a CH1, the V-2 is a VH, the C-2 is a CL, the V-3 is a VH, the C-3 is a CL, the V-4 is a VL, and the C-4 is a CH1.
10. The antibody of any one of items 1 to 9,
the first polypeptide chain comprising a linker between the CH3-1 and the V-2 and
the second polypeptide chain comprising a linker between the CH3-2 and the V-4.
11. The antibody of item 10, wherein either the linker between CH3-1 and V-2 or the linker between CH3-2 and V-4 comprises a cleavage site for a protease.

12. The antibody of item 11, wherein the protease is a furin.

13. The antibody of item 11, wherein the protease is a matrix metalloproteinase.

14. The antibody of any one items 9 to 13, wherein the linkers between the CH3-1 and the V-2 and between the CH3-2 and the V-4 are each from 5 to 35 amino acids long.

15. The antibody of item 13, wherein the linkers between the CH3-1 and the V-2 and between the CH3-2 and the V-4 are each from 5 to 30 amino acids long.

16. The antibody of any one of items 1 to 15, wherein the CH3-1 and the CH3-2 have different amino acid sequences.

17. The antibody of item 16, wherein the CH3-1 and the CH3-2 comprise charge pair substitutions.

18. The antibody of item 17, wherein

the CH3-1 comprises the charge pair substitutions K409D or K409E and K392D or K392E and the CH3-2 comprises the charge pair substitutions D399K or D399R and D356K or D356R; or

the CH3-2 comprises the charge pair substitutions K409D or K409E and K392D or K392E, and the CH3-1 comprises the charge pair substitutions D399K or D399R and D356K or D356R.

19. The antibody of any one of items 1 to 18, wherein the V-1 and the V-3 can bind to an immune effector cell when they are part of an IgG and/or scFv antibody, and the V-2 and the V-4 can bind to a target cell when they are part of an IgG and/or scFv antibody.

20. The antibody of any one of items 1 to 18, wherein the V-2 and the V-4 can bind to an immune effector cell when they are part of an IgG and/or scFv antibody, and the V-1 and the V-3 can bind to a target cell when they are part of an IgG and/or scFv antibody.

21. The antibody of item 19 or 20, wherein the immune effector cell is a T cell.

22. The antibody of item 21, wherein the effector cell molecule is human CD3 epsilon chain (CD3$\varepsilon$).

23. The antibody of any one of items 19 to 22, wherein the target cell is a cancer cell.

24. The antibody of any one of items 1 to 23 wherein the C-1 to C4, the hinge regions, and the CH2-1, CH2-2, CH3-1, and CH3-2 are human IgG constant regions or human lambda or kappa constant regions.

25. The antibody of item 24, wherein the human IgG constant regions are human IgG1 constant regions.

26. The antibody of item 24, wherein the human IgG constant regions are human IgG2 constant regions.

27. The antibody of item 24, wherein the human IgG constant regions are human IgG4 constant regions.

28. The antibody of any one of items 1 to 27, wherein the first and second polypeptide chains comprise one or more alterations that reduce Fc gamma receptor (FcyR) binding.

29. The antibody of item 28, wherein the one or more alterations that reduce FcyR binding are selected from the group consisting of: L234A, L235A, and any substitution at N297.

30. The antibody of any one of items 1 to 27, wherein the first and second polypeptide chains comprise one or more alterations that enhance(s) ADCC.

31. The antibody of any one of items 1 to 24, wherein the first and second polypeptide chains comprise an insertion of the amino acid sequence of any one of SEQ ID NOs:13-24 between positions 384 and 385 according to the EU numbering system as shown in Table 2 in CH3-1 and CH3-2.

32. An antibody comprising a first polypeptide chain having the following formula: V-1---L-3---C-1---L-4--- hinge---CH2-1---CH3-1---L-1---V-2---L-5---C-2 and a second polypeptide chain having the following formula: V-3-L6---C-3---L7---hinge---CH2-2---CH3-2---L-2---V-4---L-8---C-4,

wherein the V-1, V-2, V-3, and V-4 are immunoglobulin variable regions having different amino acid sequences,

wherein either the V-1 or the V-3 is a VH, and if the V-1 is a VH, then the V-3 is a VL, and if the V-3 is a VH, then the V-1 is a VL,

wherein either the V-2 or the V-4 is a VH, and if the V-2 is a VH, then the V-4 is a VL, and if the V-4 is a VH, then the V-2 is a VL,

wherein the L-1, L-2, L-3, L-4, L-5, L-6, L-7, and L-8 are linkers and L-3, L-4, L-5, L-6, L-6, and L-8 can be present or absent,,

wherein either the C-1 or the C-3 is a CH1, and if the C-1 is a CH1, then the C-3 is a CL, and if the C-3 is a CH1, then the C-1 is a CL,

wherein either the C-2 or the C-4 is a CH1, and if the C-2 is a CH1, then the C-4 is a CL, and if the C-4 is a CH1, then the C-2 is a CL, and

wherein the antibody binds to a cancer cell and to an immune effector cell.

33. The antibody of item 32, wherein the first and the second polypeptide chains each comprise a charge pair substitution.

34. The antibody of item 33, wherein

the CH3-1 comprises the charge pair substitutions K409D or K409E and K392D or K392E and the CH3-2 comprises the charge pair substitutions D399K or D399R and E356K or E356R; or

the CH3-2 comprises the charge pair substitutions K409D or K409E and K392D or K392E, and the CH3-1 comprises the charge pair substitutions D399K or D399R and D356K or D356R.

35. The antibody of any one of items 32 to 34, wherein the first and second polypeptide chains comprise one or more alterations that inhibit FcγR binding.

36. The antibody of item 35, wherein the first and second polypeptide chains each comprise at least one alteration selected from the group consisting of: L234A, L235A, and any substitution at N297.

37. The antibody of any one of items 32 to 36, wherein the immune effector cell is a T cell.

38. The antibody of item 37, wherein the antibody binds to human CD3ε.

39. The antibody of any one of items 32 to 38, wherein the V-1 and the V-3 can bind to the cancer cell when they are part of an IgG and/or an scFv antibody and wherein the V-2 and the V-4 can bind to the immune effector cell when they are part of an IgG and/or scFv antibody.

40. The antibody of any one of items 32 to 38, wherein the V-2 and the V-4 can bind to the cancer cell when they are part of an IgG and/or an scFv antibody and wherein the V-1 and the V-3 can bind to the immune effector cell when they are part of an IgG and/or scFv antibody.

41. The antibody of any one of items 32 to 40, wherein the L-1 comprises a protease cleavage site and the L-2 does not, or vice versa.

42. The antibody of item 1 or 31 comprising the amino acid sequences of SEQ ID NOs: 1 and 3, SEQ ID NOs: 5 and 7, SEQ ID NOs: 9 and 11, SEQ ID NOs: 46 and 48, SEQ ID NOs:11 and 62, SEQ ID NOs: 11 and 64, or SEQ ID NOs:67 and 69.

43. A pharmaceutical composition comprising the antibody of any one of items 1 to 42.

44. One or more nucleic acid(s) encoding the antibody of any one of items 1 to 42.

45. The nucleic acid(s) of item 44 comprising the nucleotide sequences of SEQ ID NOs:2 and 4, SEQ ID NOs:6 and 8, SEQ ID NOs:10 and 12, SEQ ID NOs:47 and 49, SEQ ID NOs: 68 and 70, SEQ ID NOs: 12 and 63, or SEQ ID NOs:12 and 65.

46. One or more vector(s) containing the nucleic acid(s) of item 44 or 45.

47. A host cell containing the nucleic acid(s) of item 44 or 45 or the vector(s) of item 46.

48. A method of making an antibody comprising culturing the host cell of item 47 under conditions such that the nucleic acid(s) is(are) expressed, and recovering the antibody from the cell mass or the culture medium.

49. A method of treatment comprising administering to a cancer patient a therapeutically effect dose of the antibody of any one of items 1 to 42.

50. The method of item 49, wherein the cancer is a hematologic malignancy.

51. The method of item 49 or 50, wherein chemotherapy or radiation is administered before, after, or concurrently with the antibody.

52. The method of any one of items 49 to 51, wherein the antibody can bind to FOLR1, EGFR, EGFRvIII, MCSP, MSLN, or HER2 and CD3ε.

53. A method of treatment comprising administering to a patient having an infectious disease an antibody of any one of items 1 to 42.

54. A method of treatment comprising administering to a patient having asthma, systemic lupus erythematosus, or a fibrotic disease an antibody of any one of items 1 to 42.

55. The use of an antibody of any one of items 1 to 42 as a medicament for treating a cancer, an infectious disease, an autoimmune disease, asthma, or a fibrotic disease.

56. The use of an antibody of any one of items 1 to 42 in the manufacture of a medicament for treating a cancer, an infectious disease, an autoimmune disease, asthma, or a fibrotic disease.

## EXAMPLES

### *Example 1: Making V-C-Fc-V-C's*

Nucleic acid constructs encoding two V-C-Fc-V-C antibodies specific for CD3ε and folate receptor 1 (FOLR1) (called aFOLR1-Fc-aCD3 or aCD3-Fc-aFOLR1, depending on the order of the domains) were made using nucleic acids encoding the heavy and light chain variable regions from an anti-CD3ε antibody designated F12Q and the heavy and light chain variable regions from an anti-FOLR1 antibody designated 5G1. See Figure 2, panels A and B. In DNA constructs encoding aCD3-Fc-aFOLR1, nucleic acids encoding an anti-CD3ε half Fab fragment (heavy or light chain fragment) were upstream and nucleic acids encoding an anti-FOLR1 half Fab fragment were downstream relative to nucleic acids encoding an Fc polypeptide chain. See Figure 2, panel A. In constructs encoding aFOLR1-Fc-aCD3, nucleic acids encoding an anti-CD3ε half Fab fragment (either heavy or light chain fragment) were downstream and nucleic acids encoding an anti-FOLR1 half Fab fragment were upstream relative to nucleic acids encoding an Fc polypeptide chain. *See* Figure 2, panel B. The encoded Fc polypeptide chains were human IgG Fc polypeptide chains with some minor alterations as described below. Downstream from nucleic acids encoding the Fc polypeptide chain in each construct are nucleic acids encoding a linker having the amino acid sequence $(G_4S)_2$ (SEQ ID NO:36). After the nucleic acids encoding the linker are nucleic acids encoding a variable region and then either a CL or a CH1. The CH1 region is, in some cases, followed by a short portion of what is generally considered to be a hinge region, including a cysteine. Nucleic acids encoding the half Fab fragments and Fc polypeptides were generated by polymerase chain reaction (PCR) from existing constructs and joined together by PCR SOE. *See, e.g.,* Warrens et al. (1997), Gene 186: 29-35, the portions of which describe this method are incorporated herein by reference. The nucleic acid sequences encoding these two V-C-Fc-V-C's are provided in SEQ ID NOs: 6 and 8 and SEQ ID NOs: 2 and 4. The amino acid sequences encoded by these pairs of nucleic acid sequences are SEQ ID NOs: 5 and 7 (aCD3-Fc-aFOLR1) and SEQ ID NOs: 1 and 3 (aFOLR1-Fc-aCD3).

In addition, constructs encoding V-C-Fc-V-C antibodies specific for CD3ε and Human Epidermal Growth Factor Receptor (HER2) (aHER2-Fc-aCD3 and aCD3-Fc-aHER2, which are diagrammed in Figure 2, panels C and D, respectively) were made using methods like those described above from nucleic acids encoding an anti-CD3ε antibody designated F12Q and the heavy and light chain variable regions from an anti-HER2 antibody designated 7052-D3-2. Amino acid sequences of the two polypeptide chains of aHER2-Fc-aCD3 and aCD3-Fc-aHER2 are provided in SEQ ID NOs: 9 and 11 and SEQ ID NOs: 46 and 48, respectively. The nucleic acid sequences encoding these are provided in SEQ ID NOs: 10 and 12 and SEQ ID NOs: 47 and 49, respectively.

The Fc region of each of the V-C-Fc-V-C antibodies described above contained charge pair substitutions in each Fc polypeptide chain to encourage heterodimer formation. For example, SEQ ID NO:1 contains the alterations D399K and E356K (at positions 419 and 376 of SEQ ID NO:1, respectively), and SEQ ID NO:3 contains the alterations K392D and K409E (at positions 407 and 424 of SEQ ID NO:3, respectively). Polypeptide chains containing these altered Fc polypeptides will tend to form heterodimers rather than homodimers. *See, e.g.,* US Patent Application Publication 2010/0286374 and Gunasekaran et al. (2010), J. Biol. Chem. 285: 19637-19646, the portions of which describe heterodimerizing alterations and their functional effects are incorporated herein by reference. In addition, all of the Fc polypeptide chains specifically exemplified herein also had the alterations L234A and L235A (corresponding to positions 254 and 255 in SEQ ID NO:1), substitutions known to reduce FcγR binding.

### *Example 2: Testing V-C-Fc-V-C's for binding and biological activity*

Binding of aFOLR1-Fc-aCD3 and a single chain bispecific molecule containing the same variable regions (called sc-aFOLR1-sc-aCD3) to T47D tumor cells (which express FOLR1) and purified human Pan-T cells (which express CD3ε) was analyzed by fluorescence activated cell sorting (FACS). The amino acid sequence of sc-aFOLR1-sc-aCD3ε is provided in SEQ ID NO:44. Cells were incubated for 16 hrs at 4°C in the absence or presence of 2 nM bi-specific molecule for tumor cells and 200 nM bispecific molecule for T cells. Cell binding of aFOLR1-Fc-aCD3 was detected using an allophycocyanin (APC)-labeled anti-human secondary antibody. The sc-aFOLR1-sc-aCD3 molecule, which contains a FLAG® tag, was detected using a mouse anti-FLAG® antibody followed by an APC-labeled mouse specific antibody.

FACS histograms in Figure 3 show the Mean Fluorescent Intensity (MFI) in the absence (unfilled tracing towards the left of each panel) and presence of 2 nM bispecific (for T47D cells) or 200 mM bispecific (for T cells). These data indicate that both bispecific molecules bind to T cells, which express CD3ε, and T47D tumor cells, which express FOLR1.

The following experiment was done to determine whether the aFOLR1-Fc-aCD3 could mediate T cell dependent cytolysis of target cells expressing FOLR1. Pan T effector cells isolated from healthy human donors were incubated with carboxyfluorescein succinylmidyl ester (CFSE)-labeled tumor target cells at a ratio of 10:1 in the presence and absence of various concentrations of aFOLR1-Fc-aCD3 or sc-aFOLR1-sc-aCD3. After 39-48 hours of incubation, cells were harvested, and tumor cell lysis was monitored by 7-amino-actinomycin D (7-AAD) uptake using flow cytometry. To determine the percent of total cell lysis, samples containing effector and labeled target cells without an antibody were lysed with cold 80% methanol. The percent specific lysis was calculated according to the following formula:

% specific lysis = [% tumor lysis with bi-specific - % tumor lysis without bi-specific / % of total lysis - % tumor lysis without bi-specific] x 100

Results are shown in Figure 4. Specific lysis of T47D cells (expressing about 101,000 FOLR1 sites/cell) and Cal-51 cells (expressing about 148,000 FOLR1 sites/cell), was observed with both aFOLR1-Fc-aCD3 and sc-aFOLR1-sc-aCD3. The concentration for half maximal lysis ($EC_{50}$) for sc-aFOLR1-sc-aCD3 was 0.127 pM for T47D cells and 0.130 pM for Cal-51 cells. The $EC_{50}$'s for aFOLR1-Fc-aCD3 were 105.5 pM for T47D cells and 113.9 pM for Cal-51 cells. No lysis of BT474 cells (which express undetectable, if any, levels of FOLR1) was observed with either antibody. Figure 4C. In addition, tumor target cells in the presence of aFOLR1-Fc-aCD3 or sc-aFOLR1-sc-aCD3, but in the absence of T cells, did not take up 7-AAD, indicating that T cells, which are the effector cells in this case, are required for cell death of the target cells. Data not shown. These observations suggest that the aFOLR1-Fc-aCD3 V-C-Fc-V-C is a highly specific reagent capable of inducing tumor cell lysis by T cells.

A further T cell dependent cytolysis experiment was done to determine whether the order of the segments in a V-C-Fc-V-C affected activity. The experiment was performed using Cal-51 cells as target cells, and percent specific cell lysis was determined as described above. The percent specific lysis is shown in Figure 5, and the $EC_{50}$'s of the bispecific antibodies are shown in Table 3 below.

**Table 3: $EC_{50}$'s of bispecific antibodies**

| Bispecific | $EC_{50}$ (pM) |
|---|---|
| aFOLR1-Fc-aCD3 | 60.88 |
| aCD3-Fc-aFOLR1 | 306.0 |
| sc-aFOLR1-sc-aCD3 | 0.086 |

Thus, both V-C-Fc-V-Cs have $EC_{50}$'s in the picomolar range, while sc-aFOLR1-sc-aCD3 has a lower $EC_{50}$. These observations suggest that both versions of the V-C-Fc-V-C are specific reagents capable of inducing tumor cell lysis by T cells, although one molecule, i.e., aFOLR1-Fc-aCD3, was slightly more potent than the other.

***Example 3: Testing of further bispecific molecules for binding and biological activity***

Binding of aHER2-Fc-aCD3, which is described in Example 1, and a single chain bispecific molecule having the same variable regions (SEQ ID NO:42; called sc-aHER2-sc-aCD3) to SKOV-3 tumor cells, which express HER2, and to purified human Pan-T cells, which express CD3, was analyzed by FACS. Cells were incubated for 16 hrs at 4 °C in the absence and presence of a 2 nM (for the SKOV-3 cells) or a 200 nM (for the T cells) concentration of either of the two bispecific molecules. Binding of the aHER2-Fc-aCD3 was detected using an APC-labeled anti-human secondary antibody. Binding of the sc-aHER2-sc-aCD3, which has a FLAG tag, was detected using a mouse anti-FLAG antibody followed by an APC-labeled mouse IgG specific antibody.

Results are shown in Figure 6. FACS histograms show the MFI in the absence (unfilled tracing) and presence (filled tracing) of a bispecific molecule to SKOV-3 cells (top) or T cells (bottom). Results from aHER2-Fc-aCD3 and sc-aHER2-sc-aCD3 binding are shown at left and right, respectively, as indicated. These results demonstrate that both molecules bind to T cells and tumor target cells.

To assess the ability of aHER2-Fc-aCD3 and sc-aHER2-sc-aCD3 to induce T cell dependent tumor cell killing, assays were performed and percent specific cell lysis was determined as described above. Results are shown in Figure 7. Specific lysis of SKOV-3 cells (Figure 7, top panel), which express about 530,000 HER2 sites/cell, and BT474 cells (Figure 7, middle panel), which express about 300,000 HER2 sites/cell, was observed with both aHER2-Fc-aCD3 and sc-aHER2-sc-aCD3. The concentration for half maximal lysis ($EC_{50}$) was in the pM range as shown in Table 4 below.

**Table 4**

| | $EC_{50}$ (pM) | |
|---|---|---|
| **Bispecific molecule** | **SKOV-3 cells** | **BT474 cells** |
| aHER2-Fc-aCD3 | 165.2 | 638.0 |
| sc-aHER2-sc-aCD3 | 0.0075 | 0.086 |

There was essentially no lysis of SHP77 cells, which do not express detectable levels of HER2 (Figure 7, bottom panel). In addition, tumor target cells in the presence of either of the bispecific molecules, but in the absence of T cells, did not result in 7-AAD uptake. Data not shown. These observations indicate that both aHER2-Fc-aCD3 and sc-aHER2-sc-aCD3 are specific reagents capable of inducing tumor cell lysis in the presence of T cells.

To determine whether the order of the segments within the V-C-Fc-V-C molecule affected activity, T cell dependent cell cytolysis assays using aHER2-Fc-aCD3, aCD3-Fc-aHER2, and sc-aHER2-sc-aCD3 was performed as described above except that the target cells are JIMT-1 cells, which express about 181,000 molecules of HER2 per cell. As shown in Figure 8, aHER2-Fc-aCD3 and aCD3-Fc-aHER2 gave very similar results, having $EC_{50}$'s of 56.55 pM and 51.82 pM, respectively, while sc-aHER2-sc-aCD3 had an $EC_{50}$ of 0.203 pM. Thus, in this case, the order of the Fab fragments had little if any effect on the potency of the V-C-Fc-V-C's.

To determine the ability of the aHER2-Fc-aCD3 bispecific to activate T cells, pan T effector cells isolated from human healthy donors were labeled with CFSE and incubated with SKOV tumor cells at a ratio of 10:1 in the presence or absence of either the aHER2-Fc-aCD3 or sc-aHER2-sc-aCD3 at the concentrations indicated in Figure 9. After 72 hours, cells were harvested, and the percent of T cells that were dividing was determined by flow cytometry by monitoring of the dilution of incorporated dye with each cell cycle. Expression of CD25, a marker for activation, was detected by flow cytometry using an anti-CD25 APC-labeled antibody.

As shown in Figure 9, proliferation of T cells was observed with both aHER2-Fc-aCD3 and sc-aHER2-sc-aCD3 when incubated with HER2-expressing tumor cells, i.e., SKOV-3 cells. The $EC_{50}$'s were in the pM range as shown in Table 5 below. There was no proliferation of T cells when incubated with SHP77 cells, which do not express detectable levels of HER2. Data not shown. In addition, T cells in the presence of the aHER2-Fc-aCD3 or sc-aHER2-sc-aCD3, expressed the activation marker CD25 following incubation with HER2-expressing SKOV-3 tumor cells (Figure 7, lower panel), but not with the HER2-negative tumor line, SHP77 (data not shown). These observations suggest that the V-C-Fc-V-C is a specific reagent capable of inducing T cell activation and proliferation.

**Table 5**

| **Bispecific molecule** | **$EC_{50}$ (pM)** | |
|---|---|---|
| | **% of T cells that are dividing** | **% $CD25^+$ T cells** |
| aHER2-Fc-aCD3 | 76.72 | 79.32 |
| sc-aHER2-sc-aCD3 | 0.330 | 0.136 |

***Example 4: Pharmacokinetic properties* of a *V-C-Fc-V-C***

The following experiment was done to evaluate the single dose pharmacokinetics of a V-C-Fc-V-C molecule (aHER-Fc-aCD3) vs. single chain bispecific molecule (sc-aHER2-sc-aCD3) containing two scFv's linked in tandem. The single chain molecule contained and an anti-HER2 scFv followed by an anti-CD3 scFv, and its amino acid sequence is provided in SEQ ID NO:42. Each polypeptide chain of the V-C-Fc-V-C contained an anti HER2 variable region (either VH or VL) at its N-terminus and an anti-CD3 variable region (either VH or VL) following the CH3 domain. The sequences of the two polypeptide chains are provided in SEQ ID NOs:9 and 11. The same anti-HER2 and anti-CD3 variable regions were included in both sc-aHER2-sc-aCD3 and aHER2-Fc-aCD3. The pharmacokinetic properties of these antibodies were compared following intravenous and subcutaneous bolus administration in male NOD.SCID mice (Harlan, Livermore, CA).

The antibodies were dosed intravenously (see Figure 10) via the lateral tail vein or subcutaneously (see Figure 11) under the skin over the shoulders at 1 mg/kg. Serial bleeds of approximately 0.100 mL of whole blood were collected via retro-orbital sinus puncture for each time point. Upon clotting of whole blood the samples were processed to obtain serum (yielding about 0.40 mL per sample). Serum samples were analyzed by immunoassay to determine the serum concentrations of the antibodies. Serum samples were collected at 0, 0.5, 2, 8, 24, 72, 120, 168, 240, 312, 384, and 480 hours. Serum samples were maintained at -70°C (±10°C) prior to analysis. Pharmacokinetic parameters were estimated from serum concentrations using NCA (Phoenix 6.3, Sunnyvale, CA). Results are shown in Figures 10 and 11.

As shown in Figures 10 and 11, a HER2-Fc-aCD3 was relatively stable *in vivo,* whereas the scFc was rapidly eliminated. Thus, the V-C-Fc-V-C has improved pharmacokinetic properties relative to the single chain antibody.

***Example 5: Making V-C-Fc-V-Cs containing protease cleavage sites and control molecules***

As a control, a heterodimeric bispecific antibody that binds to both CD3 and HER2 (P136797.3; called "aCD3/HER2 heterodimer") was constructed. One polypeptide comprises a VH region from an antibody that binds to CD3, followed by a linker, followed by a VL region from an antibody that binds to HER2, followed by a CH1 region, followed by a linker and an Fc polypeptide chain from a human IgG1 antibody. The sequence of this polypeptide is provided in SEQ ID NO:60. The other polypeptide chain of this heterodimer comprises a VH region from an antibody that binds to HER2, followed by a linker, followed by a VL region from an antibody that binds to CD3, followed by a CL region, followed by a linker and an Fc polypeptide chain from a human IgG1 antibody. The sequence of this polypeptide is provided in SEQ ID NO:61. DNAs encoding these polypeptides were introduced into HEK-293 cells, and the protein was recovered from the culture medium.

V-C-Fc-V-Cs containing furin or matrix metalloproteinase 2 (MMP2) cleavage sites were made by the same methods, i.e., PCR-SOE of existing nucleic acids, including insertion of protease cleavage sites and linkers via the PCR primers. See Example 1. A furin cleavage site (RRRRRR (SEQ ID NO:52)) was inserted downstream from the Fc polypeptide chain in one polypeptide chain of one V-C-Fc-V-C (aHer2-Fc-Fur-aCD3, comprising SEQ ID NOs:11 and 62), and an MMP2 cleavage site (GPLGIAGQ (SEQ ID NO:66)) was inserted downstream from the Fc polypeptide chain in one polypeptide chain of another V-C-Fc-V-C (aHer2-Fc-MMP2-aCD3, comprising SEQ ID NOs:11 and 64). These proteins were produced by transfecting DNA encoding them into HEK-293 cells, culturing the transfected cells, and recovering the protein from the cell culture supernatant. As is known in the art, HEK-293 cells express furin protease intracellularly, which has been observed to cleave recombinant proteins produced in HEK-293 cells. *See, e.g.,* Wu et al. (2003), J. Biol. Chem. 278: 25847-25852. Hence, it was expected that the V-C-Fc-V-C comprising a furin cleavage site would be cleaved. As expected, Western blots of polyacrylamide gels run under reducing conditions revealed that the aHer2-Fc-Fur-aCD3 protein recovered from the HEK-293 cells was cleaved, whereas the aHer2-Fc-MMP2-aCD3 protein was not. Data not shown.

***Example 6: Testing V-C-Fc-V-Cs containing protease cleavage site for binding and cytolytic activity***

The aHER2-Fc-Fur-aCD3 and aHER2-Fc-MMP-aCD3 V-C-Fc-V-Cs were tested for binding to pan T cells (which express CD3) or T47D cells (which express HER2) as described in Example 2. The results are shown in Figures 12A, 12B, and 13. The aHER2-Fc-Fur-aCD3 V-C-Fc-V-C, which was cleaved at the furin site (see above), binds to pan T cells almost as well as the positive control aCD3/HER2 heterodimer and much better than the aHER2-Fc-aCD3 V-C-Fc-V-Cs that do not contain protease cleavage sites. Figure 12 A. The aHER2-Fc-MMP-aCD3 V-C-Fc-V-C, which was not cleaved, is similar to the aHER2-Fc-aCD3 V-C-Fc-V-Cs in its binding to pan T cells. Figure 12A,. These results indicate that cleavage of one polypeptide chain of a V-C-Fc-V-C in the linker between the CH3 region and the following variable region, increases binding of the pair of variable regions following the CH3 regions to their target, in this case, CD3. As shown in Figure 12B, all molecules tested had similar binding properties with regard to HER2. Together these results indicate that cleavage at a protease site downstream from one of the CH3 regions of a V-C-Fc-V-C facilitates binding of the two variable regions downstream from the CH3 regions to their target, but has no effect on the binding properties of the amino-terminal variable regions.

The same antibodies were tested for their ability to cause lysis of HER2-expressing cells using the methods described in Example 2, using T47D cells as target cells and pan T cells as effector cells and using an effector:target ratio of 10:1. Only the positive control, aCD3/HER2 heterodimer, showed substantial amounts of target cell lysis, although the aHER2-Fc-Fur-aCD3 antibody showed some indication of lysis at the highest concentration tested. Figure 13. Thus, while cleavage of one polypeptide chain of a V-C-Fc-V-C downstream from the Fc polypeptide chain increased binding of the more C-terminal variable regions to their antigen such that it was comparable to that of a control molecule, cleavage did not increase cytolytic activity of a cleaved V-C-Fc-V-C to be comparable to that of a control molecule. However, other linkers and/or protease cleavage sites could possibly cause substantial increases in both binding and cytolytic activity.

## Claims

1. An antibody comprising two polypeptide chains, the first polypeptide chain comprising the following regions, which may or may not be separated by linkers, in the following order in an N- to C-terminal direction:
V-1---C-1---hinge---CH2-1---CH3-1---V-2---C-2; and
the second polypeptide chain comprising the following regions, which may or may not be separated by linkers, in the following order in an N- to C-terminal direction:
V-3---C-3---hinge---CH2-2---CH3-2---V-4---C-4;
wherein the V-1 to V-4 are immunoglobulin variable regions;
wherein the C-1 to C-4 are are either a heavy chain constant region 1 (CH1) or a light chain constant regions (CL), and if C-1 is a CH1, then C-3 is a CL and vice versa, and if C-2 is a CH1, then C-4 is a CL and vice versa; and
wherein the CH2-1 and CH2-2 are each a heavy chain constant region 2 and the CH3-1 and CH3-2 are each a heavy chain constant region 3.

2. The antibody of claim 1, the first polypeptide chain comprising a linker between the CH3-1 and the V-2 and the second polypeptide chain comprising a linker between the CH3-2 and the V-4, wherein either the linker between CH3-1 and V-2 or the linker between CH3-2 and V-4 comprises a cleavage site for a protease, and wherein the protease is a furin or a matrix metalloproteinase.

3. The antibody of claim 1 or 2, wherein the V-1 and the V-3 can bind to an immune effector cell when they are part of an IgG and/or scFv antibody, and the V-2 and the V-4 can bind to a target cell when they are part of an IgG and/or scFv antibody; or wherein the V-2 and the V-4 can bind to an immune effector cell when they are part of an IgG and/or scFv antibody, and the V-1 and the V-3 can bind to a target cell when they are part of an IgG and/or scFv antibody.

4. The antibody of claim 3, wherein the target cell is a cancer cell.

5. An antibody comprising a first polypeptide chain having the following formula: V-1-L-3---C-1---L-4---hinge---CH2-1---CH3-1---L-1---V-2---L-5---C-2 and a second polypeptide chain having the following formula: V-3-L6---C-3---L7---hinge---CH2-2---CH3-2---L-2---V-4---L-8---C-4,
wherein the V-1, V-2, V-3, and V-4 are immunoglobulin variable regions having different amino acid sequences,
wherein either the V-1 or the V-3 is a VH, and if the V-1 is a VH, then the V-3 is a VL, and if the V-3 is a VH, then the V-1 is a VL,
wherein either the V-2 or the V-4 is a VH, and if the V-2 is a VH, then the V-4 is a VL, and if the V-4 is a VH, then the V-2 is a VL,
wherein the L-1, L-2, L-3, L-4, L-5, L-6, L-7, and L-8 are linkers and L-3, L-4, L-5, L-6, L-6, and L-8 can be present or absent,,
wherein either the C-1 or the C-3 is a CH1, and if the C-1 is a CH1, then the C-3 is a CL, and if the C-3 is a CH1, then the C-1 is a CL,
wherein either the C-2 or the C-4 is a CH1, and if the C-2 is a CH1, then the C-4 is a CL, and if the C-4 is a CH1, then the C-2 is a CL, and
wherein the antibody binds to a cancer cell and to an immune effector cell.

**6.** The antibody of claim 5, wherein the V-1 and the V-3 can bind to the cancer cell when they are part of an IgG and/or an scFv antibody and wherein the V-2 and the V-4 can bind to the immune effector cell when they are part of an IgG and/or scFv antibody; or wherein the V-2 and the V-4 can bind to the cancer cell when they are part of an IgG and/or an scFv antibody and wherein the V-1 and the V-3 can bind to the immune effector cell when they are part of an IgG and/or scFv antibody.

**7.** The antibody of claim 5 or 6, wherein the L-1 comprises a protease cleavage site and the L-2 does not, or vice versa.

**8.** The antibody of claim 3 or 5, wherein the immune effector cell is a T cell.

**9.** The antibody of claim 8, wherein the antibody binds to human CD3ε.

**10.** The antibody of claim 1 or 5 comprising the amino acid sequences of SEQ ID NOs: 1 and 3, SEQ ID NOs: 5 and 7, SEQ ID NOs: 9 and 11, SEQ ID NOs: 46 and 48, SEQ ID NOs:11 and 62, SEQ ID NOs: 11 and 64, or SEQ ID NOs:67 and 69.

**11.** One or more nucleic acid(s) encoding the antibody of any one of claims 1 to 10.

**12.** The nucleic acid(s) of claim 11 comprising the nucleotide sequences of SEQ ID NOs:2 and 4, SEQ ID NOs:6 and 8, SEQ ID NOs:10 and 12, SEQ ID NOs:47 and 49, SEQ ID NOs: 68 and 70, SEQ ID NOs: 12 and 63, or SEQ ID NOs:12 and 65.

**13.** The antibody of any one of claims 1 to 12 for use in therapy.

**14.** The antibody of any one of claims 1 to 12 for use in the treatment of cancer, preferably wherein the cancer is a hematologic malignancy.

**15.** The antibody for the use of claims 14, wherein the antibody can bind to FOLR1, EGFR, EGFRvIII, MCSP, MSLN, or HER2 and CD3ε.

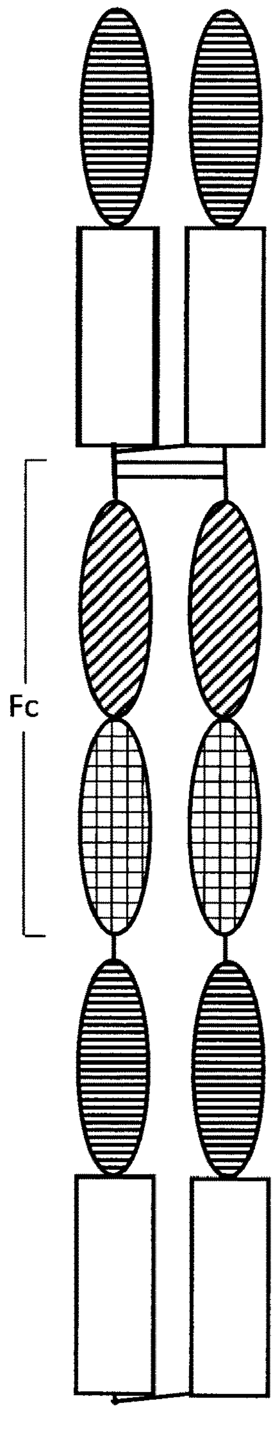


**Figure 1**

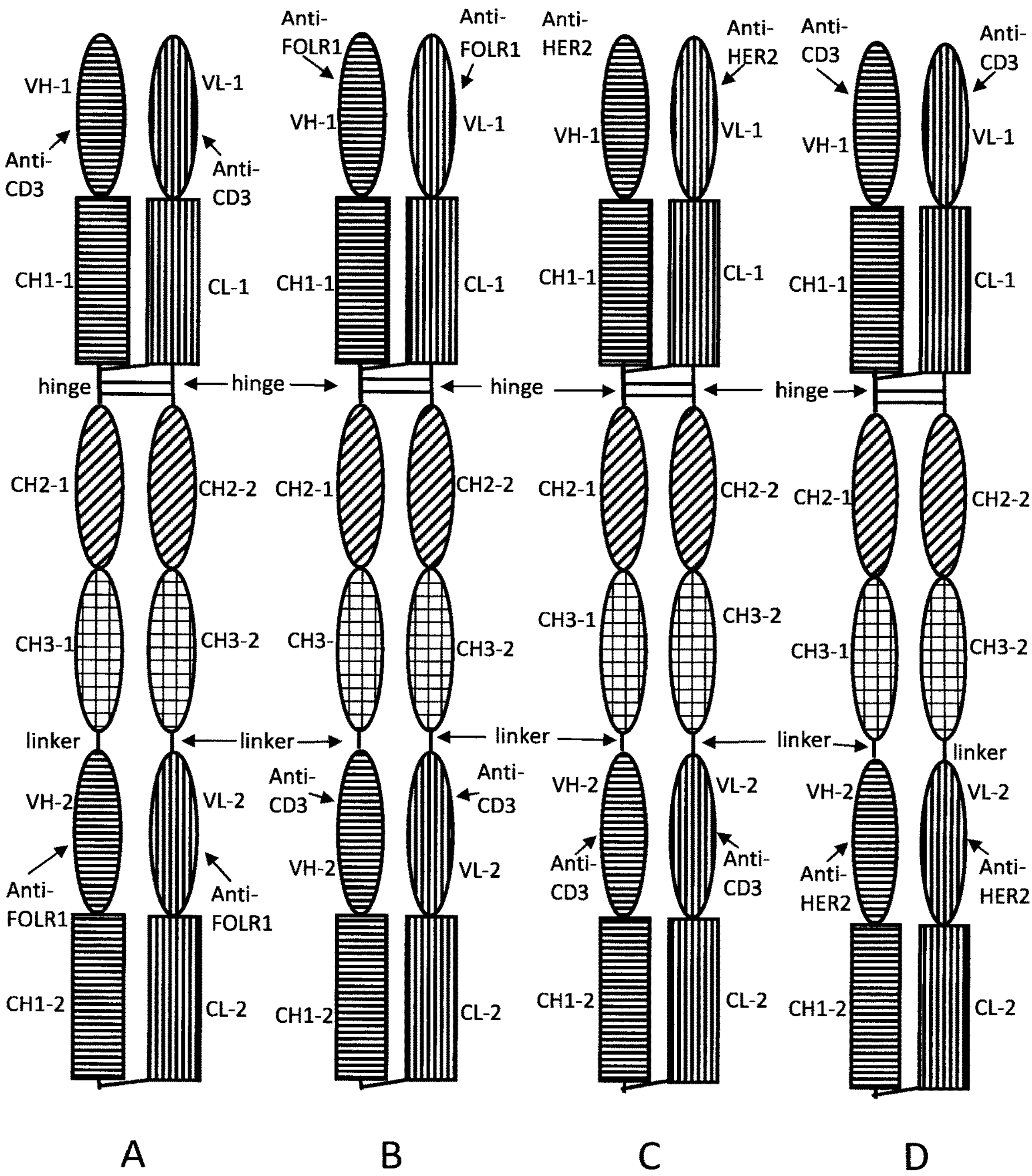
Anti-CD3
VH-1
VL-1
CH1-1
CL-1
hinge
CH2-1
CH2-2
CH3-1
CH3-2
linker
VH-2
VL-2
Anti-FOLR1
CH1-2
CL-2
A
Anti-FOLR1
Anti-CD3
B
Anti-HER2
C
D

**Figure 2**

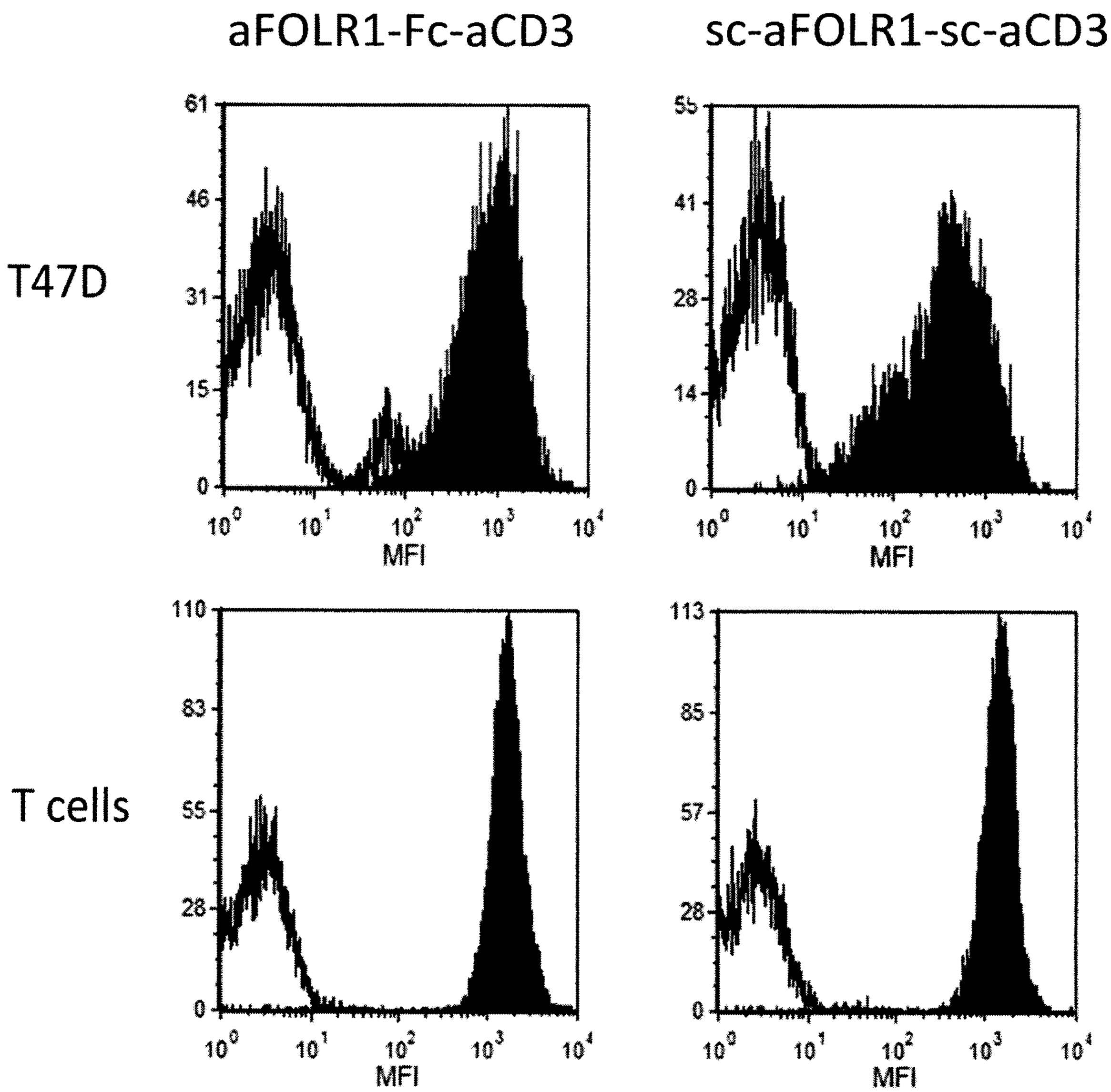
aFOLR1-Fc-aCD3
sc-aFOLR1-sc-aCD3
T47D
T cells
61
46
31
15
0
55
41
28
14
0
110
83
55
28
0
113
85
57
28
0
$10^0$ $10^1$ $10^2$ $10^3$ $10^4$
MFI

Figure 3

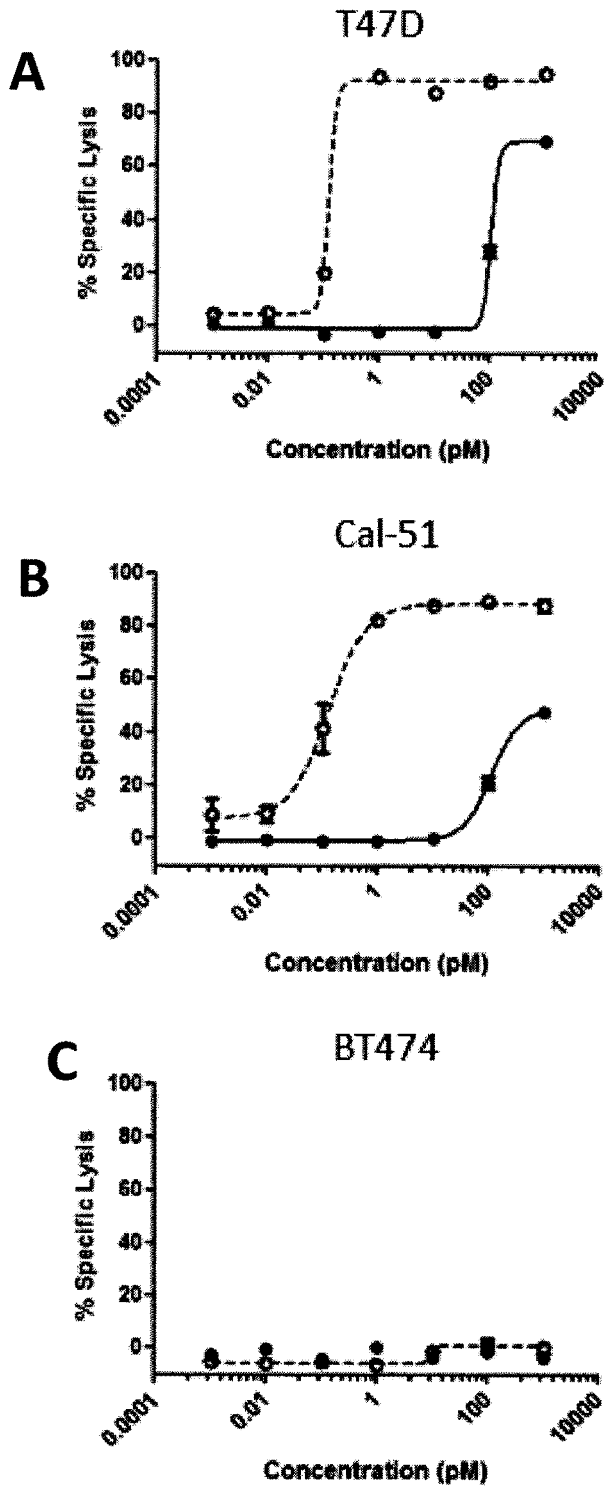

T47D
A
% Specific Lysis
Concentration (pM)
Cal-51
B
% Specific Lysis
Concentration (pM)
BT474
C
% Specific Lysis
Concentration (pM)


**Figure 4**

Cal-51

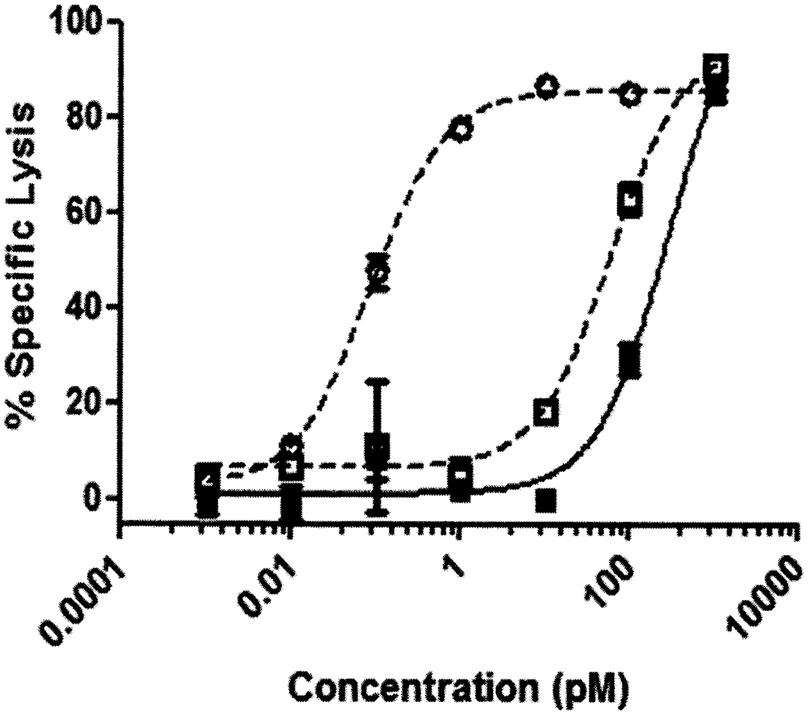


**Figure 5**

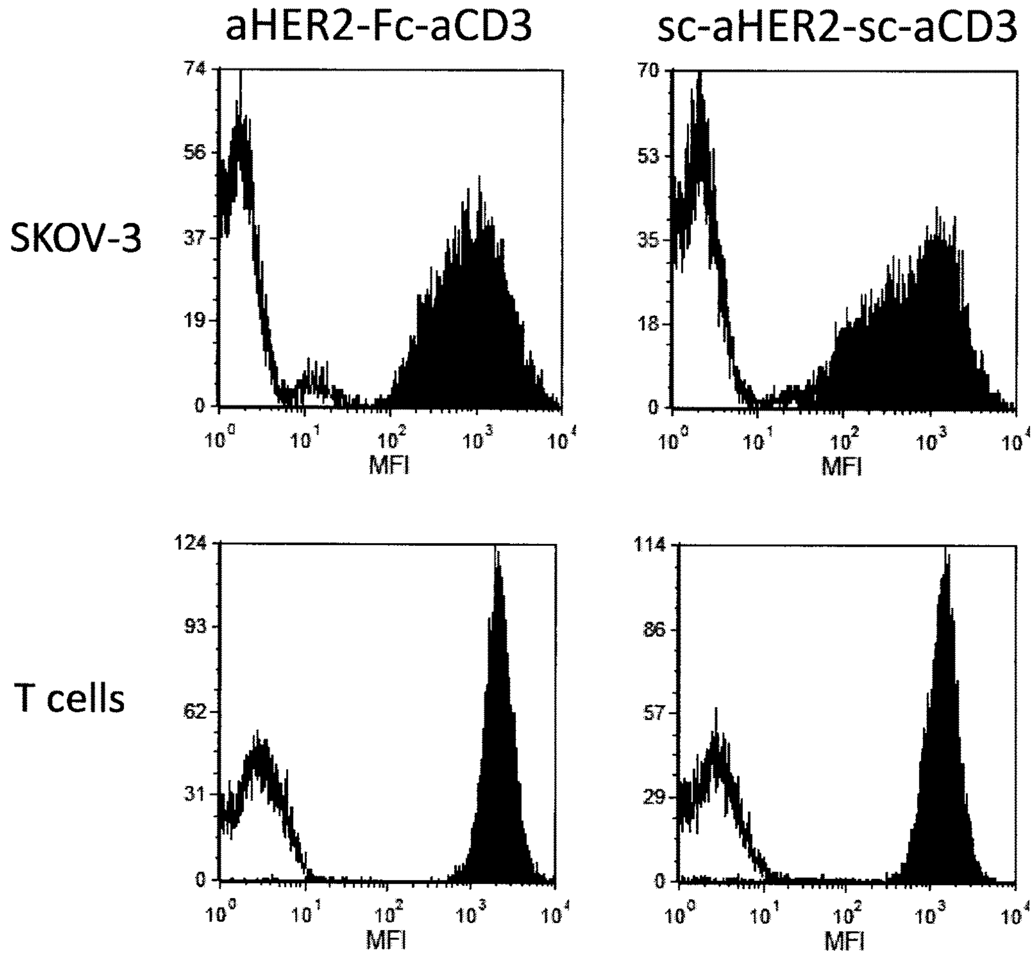
aHER2-Fc-aCD3
sc-aHER2-sc-aCD3
SKOV-3
T cells
74
56
37
19
0
70
53
35
18
124
93
62
31
114
86
57
29
MFI

**Figure 6**

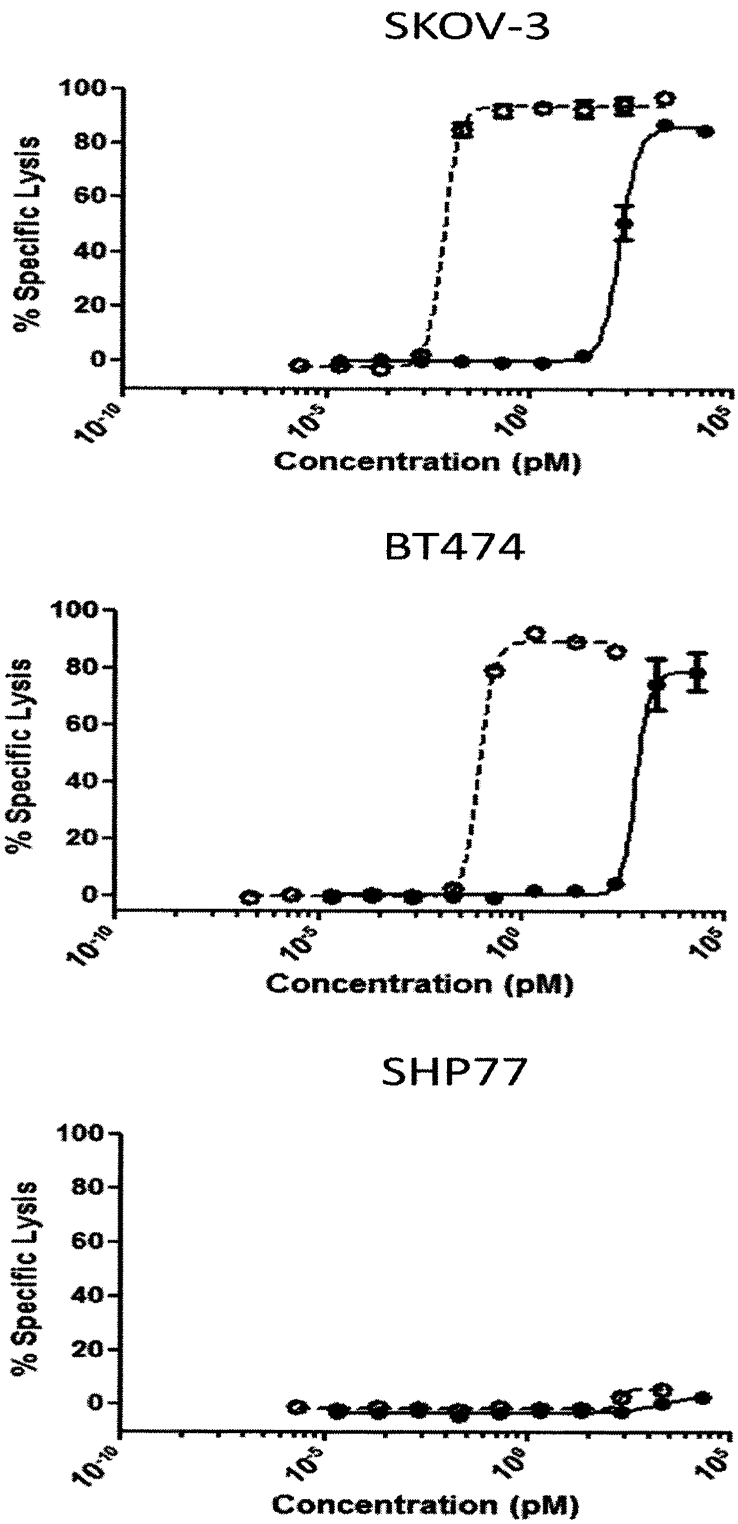
SKOV-3
% Specific Lysis
100
80
60
40
20
0
10⁻¹⁰
10⁻⁵
10⁰
10⁵
Concentration (pM)
BT474
% Specific Lysis
Concentration (pM)
SHP77
% Specific Lysis
Concentration (pM)

Figure 7

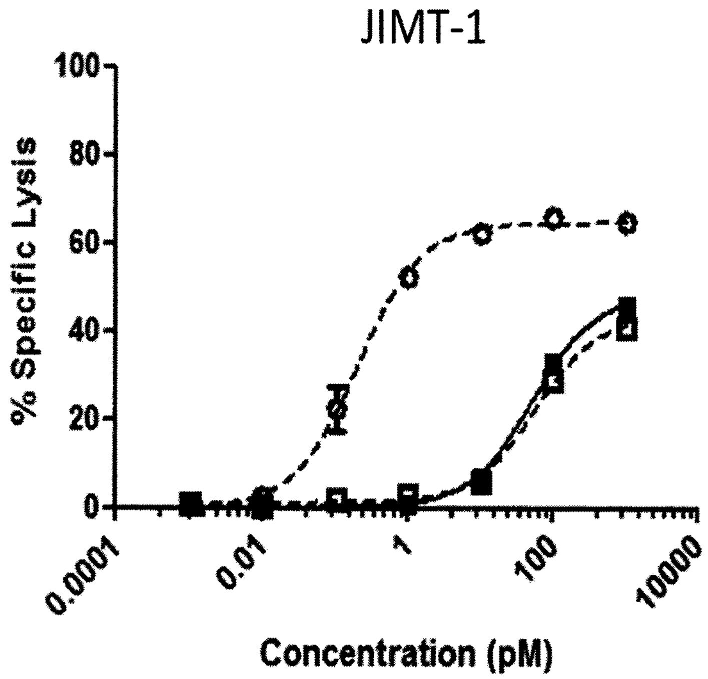
JIMT-1
100
80
60
40
20
0
% Specific Lysis
0.0001
0.01
1
100
10000
Concentration (pM)

Figure 8

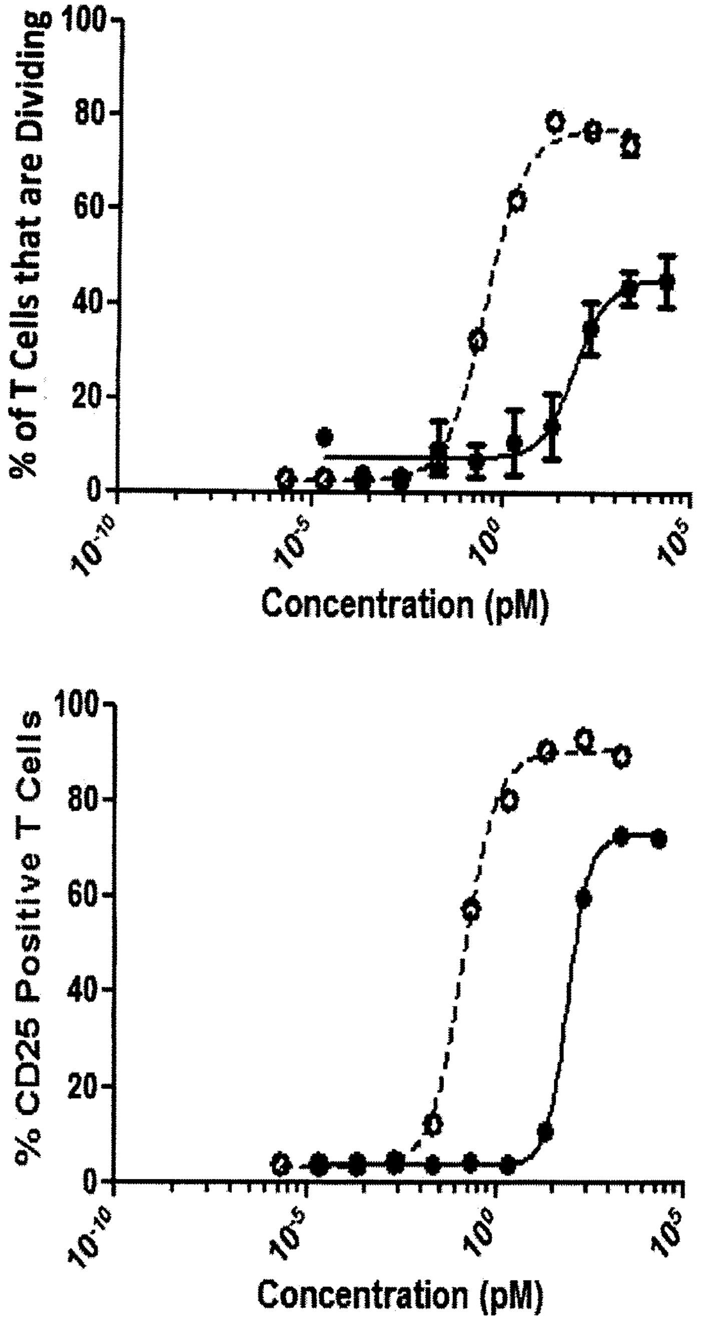
100
80
60
40
20
0
% of T Cells that are Dividing
10-10
10-5
100
105
Concentration (pM)
100
80
60
40
20
0
% CD25 Positive T Cells
10-10
10-5
100
105
Concentration (pM)

Figure 9

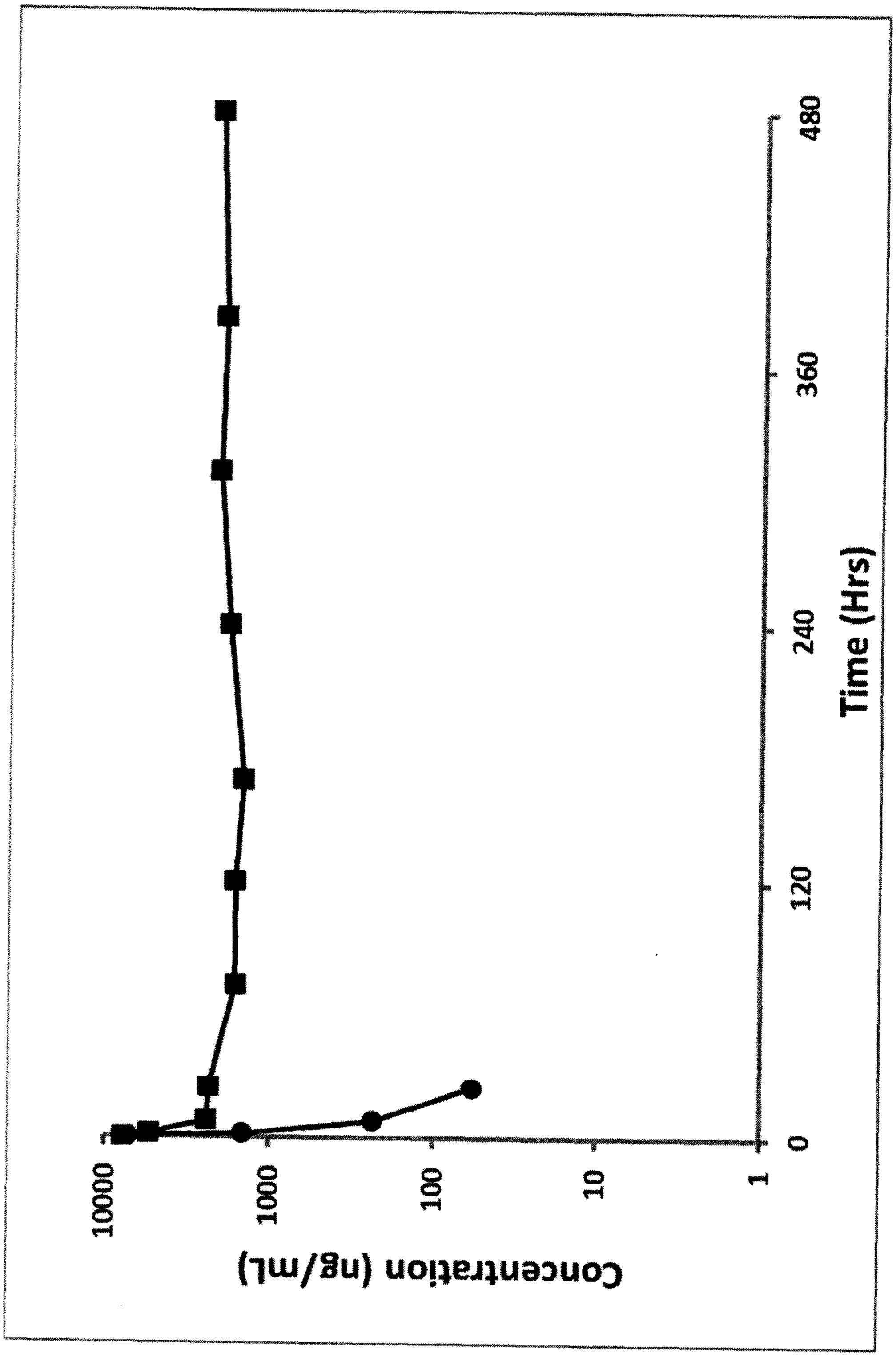
Concentration (ng/mL)
10000
1000
100
10
1
0
120
240
360
480
Time (Hrs)

Figure 10

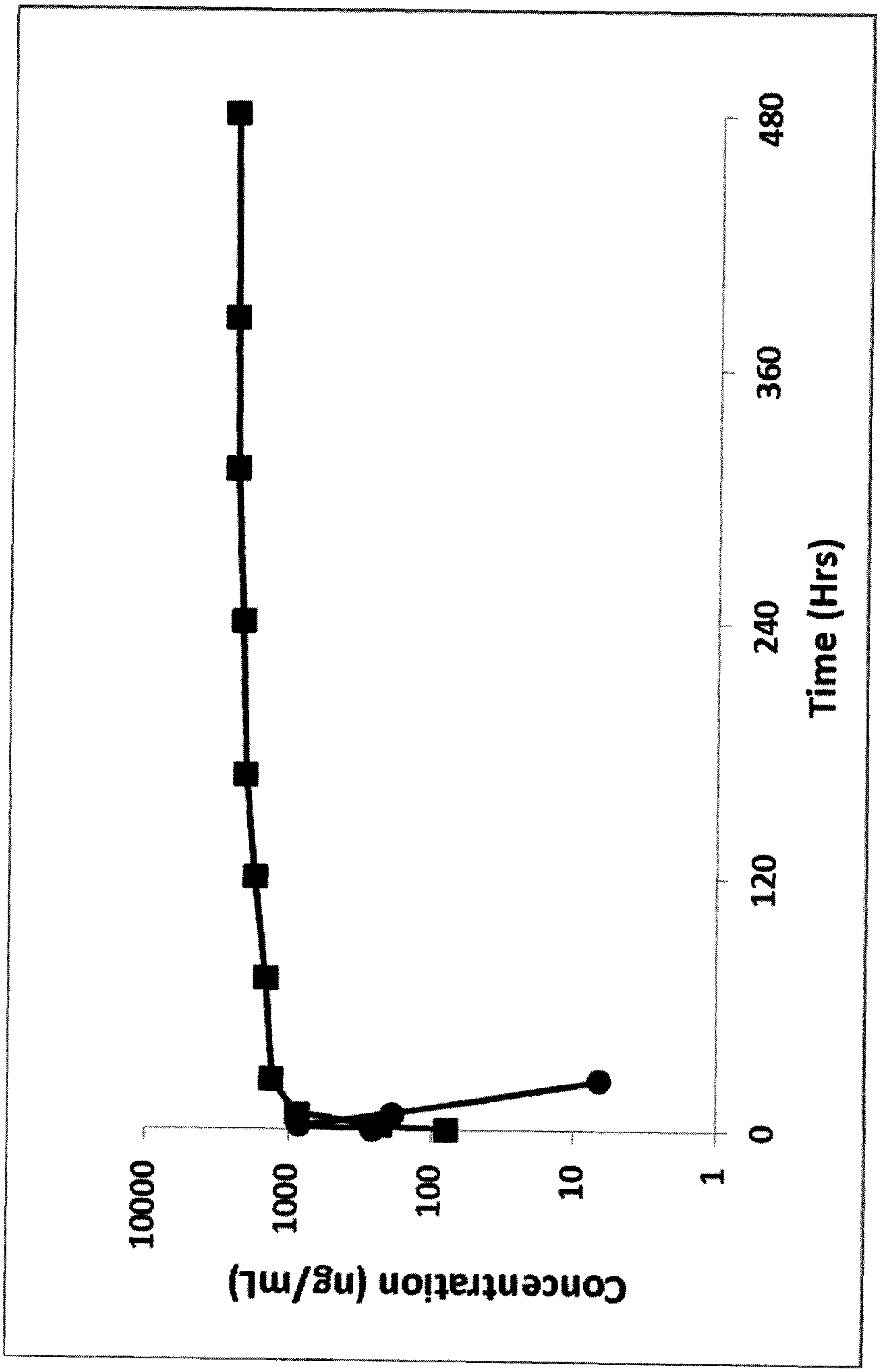
Concentration (ng/mL)
10000
1000
100
10
1
0
120
240
360
480
Time (Hrs)

Figure 11

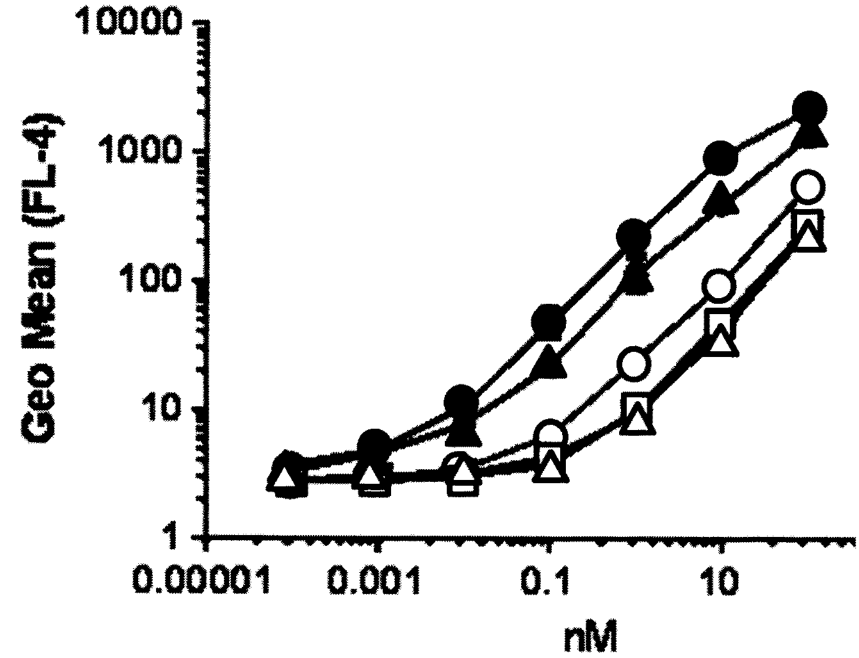


Figure 12A

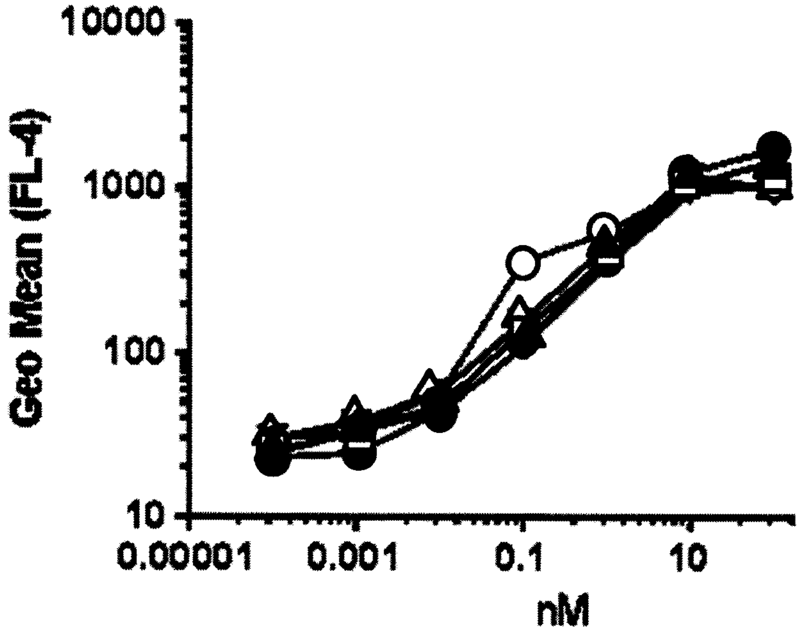


Figure 12B

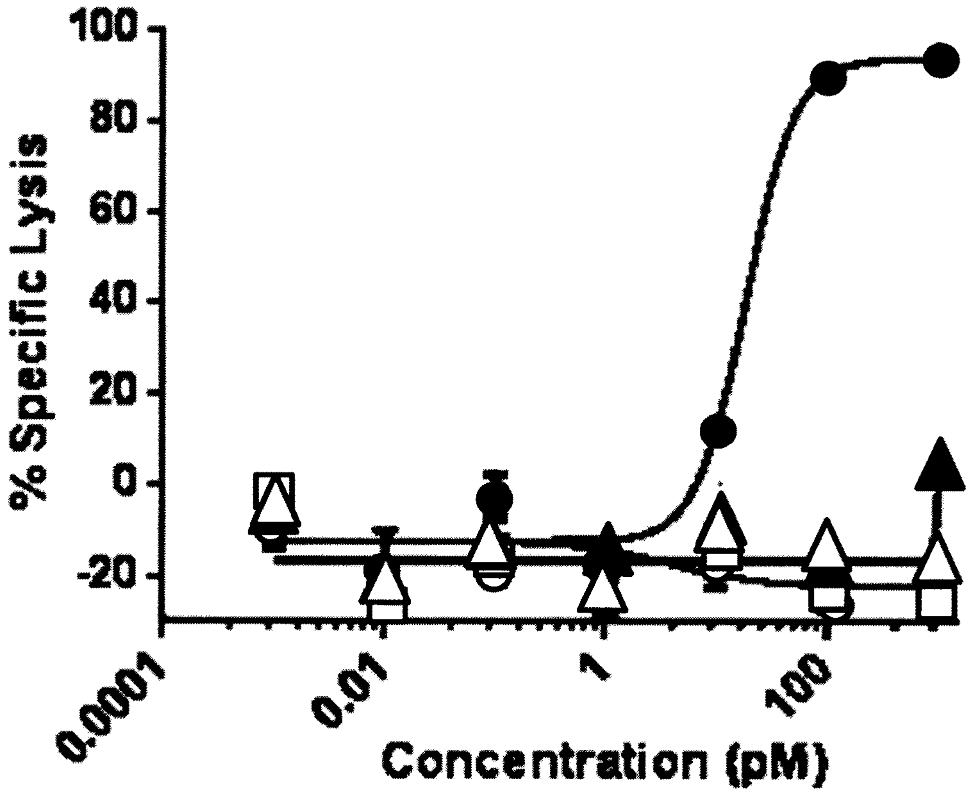
100
80
60
40
20
0
-20
% Specific Lysis
0.0001
0.01
1
100
Concentration (pM)

Figure 13

European Patent Office / Europäisches Patentamt / Office européen des brevets

# EUROPEAN SEARCH REPORT

Application Number
EP 20 16 5091

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2007/048037 A2 (AMGEN INC [US]; WEI YAN [US]; WITTEKIND MICHAEL [US]; FORTE CARLA [US]) 26 April 2007 (2007-04-26)<br>* See examples, claims, figure 1 * | 1,3-15<br>2 | INV.<br>C07K16/28<br>C07K16/32 |
| X<br>Y | WO 2012/025525 A1 (ROCHE GLYCART AG [CH]; BRINKMANN ULRICH [DE] ET AL.)<br>1 March 2012 (2012-03-01)<br>* See example, claims, figures * | 1,3-15<br>2 | |
| Y | B. J. WRANIK ET AL: "LUZ-Y, a Novel Platform for the Mammalian Cell Production of Full-length IgG-bispecific Antibodies", JOURNAL OF BIOLOGICAL CHEMISTRY, vol. 287, no. 52, November 2012 (2012-11), pages 43331-43339, XP055204058, ISSN: 0021-9258, DOI: 10.1074/jbc.M112.397869<br>* See the whole document, especially figure 1 * | 2 | |
| | | | TECHNICAL FIELDS SEARCHED (IPC) |
| | | | C07K |

The present search report has been drawn up for all claims

1

| Place of search | Date of completion of the search | Examiner |
|---|---|---|
| The Hague | 17 September 2020 | Nauche, Stéphane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 20 16 5091

This annex lists the patent family members relating to the patent documents cited in the above-mentioned European search report.
The members are as contained in the European Patent Office EDP file on
The European Patent Office is in no way liable for these particulars which are merely given for the purpose of information.

17-09-2020

| Patent document cited in search report | Publication date | Patent family member(s) | Publication date |
|---|---|---|---|
| WO 2007048037 A2 | 26-04-2007 | AR 056142 A1 | 19-09-2007 |
| | | AU 2006304846 A1 | 26-04-2007 |
| | | CA 2626013 A1 | 26-04-2007 |
| | | EP 1957533 A2 | 20-08-2008 |
| | | EP 2465871 A1 | 20-06-2012 |
| | | JP 5199876 B2 | 15-05-2013 |
| | | JP 2009512453 A | 26-03-2009 |
| | | TW 200732350 A | 01-09-2007 |
| | | US 2007105199 A1 | 10-05-2007 |
| | | WO 2007048037 A2 | 26-04-2007 |
| WO 2012025525 A1 | 01-03-2012 | BR 112013002167 A2 | 31-05-2016 |
| | | CA 2807269 A1 | 01-03-2012 |
| | | CN 103068847 A | 24-04-2013 |
| | | EP 2609112 A1 | 03-07-2013 |
| | | JP 5753903 B2 | 22-07-2015 |
| | | JP 2013538204 A | 10-10-2013 |
| | | KR 20130041963 A | 25-04-2013 |
| | | MX 340556 B | 14-07-2016 |
| | | RU 2013110876 A | 27-09-2014 |
| | | US 2013266568 A1 | 10-10-2013 |
| | | WO 2012025525 A1 | 01-03-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61882428
- WO 2009089004 A
- US 20070105199 A
- US 20090311253 A
- US 7083784 B
- US 20100234575
- US 7670600 B
- WO 2013096221 A
- US 7695936 B
- US 20030078385
- US 20100286374
- WO 2012125850 A
- US 4965195 A
- EP 0367566 A
- US 4968607 A
- EP 0460846 A
- US 7037784 B
- US 7371827 B
- US 2012070146 W
- US 20100183615
- US 20100150918


### Non-patent literature cited in the description


- **BARGOU et al.** *Science,* 2008, vol. 321 (5891), 974-977
- **HAAS et al.** *Immunobiology,* 2009, vol. 214 (6), 441-453
- Immunoglobulins: Structure and Function. **CARAYANNOPOULOS ; CAPRA.** Fundamental Immunology. Raven Press, 1993, 283-314
- **CARAYANNOPOULOS ; CAPRA.** FUNDAMENTAL IMMUNOLOGY. Raven Press, 1993, 284-286
- **MULDERMANS et al.** *J. Biotechnol.,* 2001, vol. 74, 277-302
- **DESMYTER et al.** *J. Biol. Chem.,* 2001, vol. 276, 26285-90
- **STRELTSOV et al.** *Protein Science,* 2005, vol. 14, 2901-2909
- BISPECIFIC ANTIBODIES. Springer, 2011
- Cancer: Principles and Practice of Oncology. J.B. Lippincott Co, 1993
- Hematology and Oncology, 144. Principles of Cancer Therapy. Merck Manual of Diagnosis and Therapy. 1999
- Immunoglobulins: Structure and Function. **CARAYANNOPOULOS ; CAPRA.** FUNDAMENTAL IMMUNOLOGY. Raven Press, 1993, 283-314, 284-285
- **EDELMAN et al.** *Proc. Natl. Acad. Sci.,* 1969, vol. 63, 78-85
- **KABAT et al.** SEQUENCES OF IMMUNOLOGICAL INTEREST. Public Health Service N.I.H, 1991
- **HONEGGER ; PLUCKTHUN.** *J. Mol. Biol.,* 2001, vol. 309, 657-670
- **KABAT et al.** SEQUENCES OF PROTEINS OF IMMUNOLOGICAL INTEREST. Public Health Service N.I.H, 1991
- **NIELSEN et al.** *Protein Eng.,* 1997, vol. 10 (1), 1-6
- **VON HEIJNE.** *Eur. J. Biochem.,* 1983, vol. 133, 17-21
- **VON HEIJNE.** *J. Mol. Biol.,* 1985, vol. 184, 99-105
- **COSMAN et al.** *Nature,* 1984, vol. 312, 768
- **XU et al.** *Cell. Immunol.,* 2000, vol. 200 (1), 16-26
- **SCHELLER et al.** *Nature Biotechnol.,* 2001, vol. 19, 573-577
- **ZHU et al.** *Nature Biotechnol.,* 2005, vol. 23, 1159-1169
- **LAIBLE et al.** *Reprod. Fertil. Dev.,* 2012, vol. 25 (1), 315
- Cancer, Principles and Practice of Oncology. J. B. Lippincott Co, 1993
- **WARRENS et al.** *Gene,* 1997, vol. 186, 29-35
- **GUNASEKARAN et al.** *J. Biol. Chem.,* 2010, vol. 285, 19637-19646
- **WU et al.** *J. Biol. Chem.,* 2003, vol. 278, 25847-25852